# EUROPEAN PATENT APPLICATION

(11) **EP 4 403 633 A1**
(43) Date of publication of application: **24.07.2024**
(21) Application number: 22869445.1
(22) Date of filing: 19.09.2022
(51) Int. Cl.: C12N 15/113, A61K 31/713, A61P 9/00, A61K 48/00, C12N 15/11

(54) **LPA INHIBITOR AND USE THEREOF**

(30) Priority: 18.09.2021 CN 202111110773; 20.01.2022 CN 202210068123
(71) Applicant: Genoval Therapeutics Co., Ltd., Shanghai 200120 (CN)
(72) Inventor: LI, Chong, Shanghai 200120 (CN); GU, Wei, Shanghai 200120 (CN); LI, Qian, Shanghai 200120 (CN); YIN, Ke, Shanghai 200120 (CN); MA, Haiping, Shanghai 200120 (CN)
(74) Representative: Fuchs Patentanwälte Partnerschaft mbB
(86) International application number: PCT/CN2022/119582
(87) International publication number: WO 2023/041079

(57) **Abstract**

An LPA RNA interference (RNAi) agent, comprising nucleotides targeting a nucleic acid molecule encoding LPA(apo(a)), the oligonucleotide comprising at least 12 consecutive nucleotides in any one of the nucleotide sequences SEQ ID NO:2 to SEQ ID NO:92. A pharmaceutical composition comprising one or more RNA interference agents, in vivo delivery of the RNA interference agent to liver cells providing inhibition of LPA gene expression, and the use of the nucleic acidbased therapeutic agent to lower lipoprotein(a) (Lp(a)) and treat or prevent cardiovascular-related diseases.

## Description

### CROSS-REFERENCE

The present application claims priority to Chinese Patent Application No. 202111110773.1, entitled LPA INHIBITOR AND USE THEREOF, filed on September 18, 2021, and Chinese Patent Application No. 202210068123.3, entitled siRNA CONJUGATE AND USE THEREOF, filed on January 20, 2022, the entire contents of both of which are incorporated herein by reference.

### TECHNICAL FIELD

The present application relates to the technical field of biological medicine, in particular to an LPA inhibitor and use thereof.

### BACKGROUND

RNA interference (RNAi) refers to the efficient and specific degradation of homologous mRNAs induced by double-stranded RNA (dsRNA), which is highly conserved during evolution. Since the expression of specific genes can be specifically knocked out or turned off using RNAi technology (in which dsRNAs longer than thirty nucleotides may cause interferon toxicity), the technology has been widely used in the fields of exploring gene functions and the treatment of infectious diseases and malignant tumors. Short dsRNAs direct gene-specific post-transcriptional silencing in many organisms, including vertebrates, and this has become a tool that can be used to study gene function. RNAi is mediated by RNA-induced silencing complexes (RISC), a complex formed by siRNA in complex with Argonaute protein and Dicer enzyme. It is first cleaved by the enzyme Dicer to obtain small dsRNA intermediate molecules, i.e., small RNA duplexes, which are then unstranded to form "functional" single strands, thus acting as "guides" for the RISC complex. For each small dsRNA molecule, only one strand, i.e., the guide strand, binds to a specific Argonaute protein to form the activated RISC complex. RNA interference agents such as siRNAs and microRNAs inhibit protein translation by gene silencing, i.e., by degrading the mRNA molecule to prevent formation of the oligonucleotide of protein.

Lipoprotein(a) [Lp(a)] is a heterogeneous type of low-density lipoprotein (LDL)-like particle that contains a lipid core and apolipoprotein B (apoB-100), and a unique component, apolipoprotein(a) (apo(a)), which is attached to apoB-100 by disulfide bonds.

The LPA (Apo(a)) gene is predominantly expressed in the liver and the expression is restricted to humans and non-human primates. Lp(a) levels in humans are genetically limited and do not change significantly with diet, exercise, or other lifestyle changes. The length of LPA varies based on the number of existing Kringle KIV2 structural domains, and its expression is negatively correlated with the number of existing structural domains.

The analysis of Lp(a) levels in several studies has suggested that high Lp(a) levels are an independent risk factor for cardiovascular disease, stroke and other related conditions, including atherosclerotic stenosis. In addition, genome-wide association analyses have indicated that LPA is a genetic risk factor for diseases such as atherosclerotic stenosis. Therefore, it is needed to develop a therapeutic agent that can be associated with LPA-related diseases, especially a pharmaceutical agent that reduces LPA expression levels.

### SUMMARY

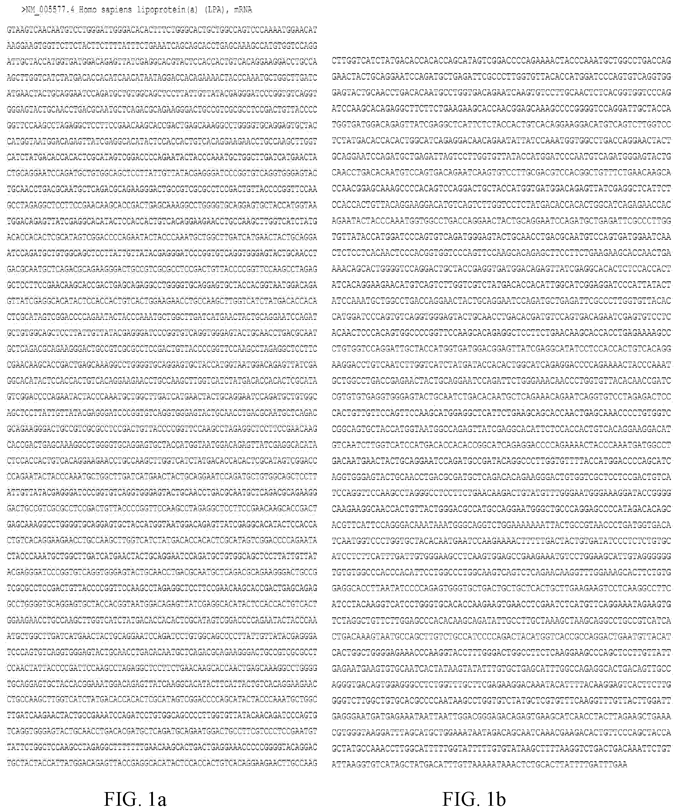

One purpose of the present application is to provide an LPA RNA interference (RNAi) agent capable of modulating (e.g. reducing) LPA (apo(a)) levels in cells and subjects such as mammals (e.g. humans) and method thereof. LPA is the name of the gene encoding apolipoprotein (a) (apo(a)), the key component of the lipoprotein(a) particle (Lp(a)), and the human DNA sequence (SEQ ID NO.1) is shown in FIGs. 1a and 1b. The LPA (also known as apo(a)) RNA interference (RNAi) agent (also known as RNAi trigger or trigger) and the composition containing LPA RNAi agent are used to selectively and effectively inhibit the expression of LPA genes.

Another purpose of the present application is to provide a pharmaceutical composition including the said LPA RNAi agent.

Another purpose of the present application is to provide the use of the said LPA RNAi agent in the prevention or treatment of a disease or condition or in the preparation of a medicine for the prevention or treatment of the disease or condition.

The first aspect of the present application provides an LPA RNA interference (RNAi) agent including a first nucleotide sequence, which includes at least 12 consecutive nucleotides of any one selected from the group consisting of the nucleotide sequences of SEQ ID NO: 2 to SEQ ID NO: 183 or a nucleotide sequence that is complementary thereto, or a sequence that differs not more than 3 nucleotides therefrom, wherein each of the nucleotides is independently in a modified or unmodified state.

Preferably, the first nucleotide sequence consists of 12 to 30 nucleotides, preferably 19 to 23 nucleotides.

Preferably, the first nucleotide sequence is a single-stranded oligonucleotide or a double-stranded nucleotide.

Preferably, one or more nucleotides of the first nucleotide sequence are modified to form modified nucleotides.

Preferably, the first nucleotide sequence includes any one of the modified sense strand nucleotide sequences listed in Table 2 or a sequence that differs not more than 3 nucleotides therefrom.

Preferably, the first nucleotide sequence is a sense strand or an antisense strand.

Preferably, the first nucleotide sequence includes a double-stranded structure of an antisense strand and a sense strand.

Preferably, the sense strand and/or antisense strand independently contains one or more modified nucleotides.

Preferably, the modified nucleotide is at least one selected from the group consisting of 2'-fluoro-modified nucleotide, 2'-O-methyl modified nucleotide, 2'-O-methoxyethyl modified nucleotide, 2'-O-alkyl modified nucleotide, 2'-O-allyl modified nucleotide, bicyclic nucleic acid, deoxyribonucleotide, EVP, UNA, and GNA.

Preferably, the antisense strand has at least one position , selected from the group consisting of positions 2, 5, 6, 8, 10, 14, and 16 from the 5' end, has a 2' -fluoro-modified nucleotide, and preferably, 5-7 of these positions have 2' -fluoro-modified nucleotides.

Preferably, the sense strand and/or antisense strand independently contains one or more thiophosphate ester bonds; more preferably, the sense strand contains two consecutive thiophosphate ester bonds between the end nucleotides at the 3' and 5' ends, or the antisense strand contains two consecutive thiophosphate ester bonds between the end nucleotides at the 3' and 5' ends.

In some embodiments, the antisense strand includes any one of the modified antisense strand nucleotide sequences in Table 2 or a nucleotide sequence differs not more than 3 nucleotides therefrom.

More preferably, the sense strand and the antisense strand have the following characteristics: the sense strand contains or consists of the modified sense strand sequence listed in Table 2, and the antisense strand contains or consists of the modified antisense strand sequence listed in Table 2.

Preferably, the RNA interfering agent also contains a ligand.

Preferably, the ligand includes a targeting group.

Preferably, the targeting group includes asialoglycoprotein receptor ligand.

Preferably, the asialoglycoprotein receptor ligand includes a galactose cluster.

Preferably, the ligand is configured to conjugate to the sense strand and/or the antisense strand, and preferably, to conjugate to the sense strand.

In one embodiment, the LPA RNA interference agent includes the following structure:

Z-X (I),

Where X is the nucleotide sequence, Z is the first connector part of the nucleotide sequence, wherein Z and X can be connected directly or through a chemical group, and the general formula of Z is shown as (Z-1), wherein one end of the O group is connected with the nucleotide sequence:

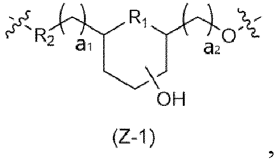

Where R₁ is O, S, NR₃ or CR₃R₄, wherein R₃ and R₄ are independently hydrogen, halogen, substituted or unsubstituted aliphatic group, substituted or unsubstituted aryl group, substituted or unsubstituted heteraryl group, substituted or unsubstituted heterocyclic ring or substituted or unsubstituted cycloalkyl group;

Wherein R₂ is -O-, -S-, -NH-, -CH₂-, -C(O)-, -OC(O)-, -C(O)O-, - NHC(O)-, -C(O)NH-, -CH₂NH-, -CH₂O-, -NH-C(O)-CH₂- and -C(O)-CH₂- NH- or -NH(CO)NH-, where the -CH₂- may optionally be substituted by one of the substituents selected from halogens, alkyl groups, alkoxy groups, and alkane amino groups; The a₁ and a₂ are the same or different, and are selected from integers of 0 to 20, respectively, preferably integers of 1 to 10.

Preferably, the nucleotide sequence represented by X is the first nucleotide sequence described above.

Preferably, the first connector part Z is connected to the second connector part L1, and the second connector part L1 is connected to the branch point group E.

Preferably, the branch point group E is connected with targeting assemblies that the quantity is a, and the a is an integer selected from 0 to 10, preferably an integer from 1 to 5; The targeting assembly includes a tethered part L₂ and a targeting part T in a 1:1 ratio.

Specifically, the structural formula of LPA RNA interference agent can be expressed as:

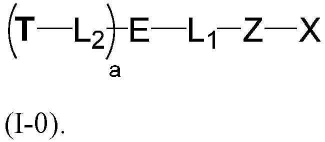

In one embodiment, the LPA RNA interference agent has the following structure:

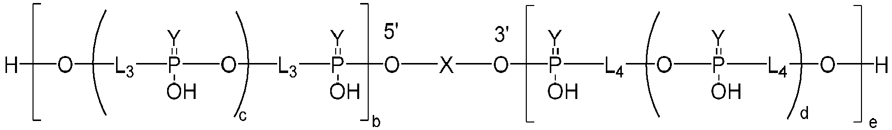

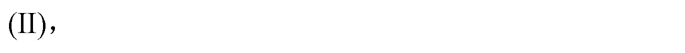

In the general formula (II):

L₃ includes a third connector part, L₄ is a targeting part, specifically, L₃ and L₄ can be the same or different, preferably, L₃ and L₄ are respectively the following structure: 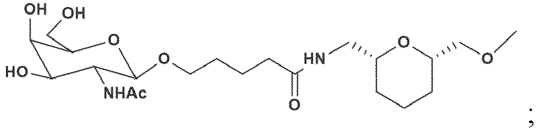
wherein X is a nucleotide sequence, which can be a sense strand or an antisense strand,
Y is O or S,
where b, c, d, and e are integers from 0 to 10, and b and e are 0 when they are different. In one embodiment, b is 0 and e is an integer from 3 to 6.

On the other hand, the present application also provides the use of the LPA RNA interference agent described above in the preparation of drugs, the drugs are used to reduce LPA mRNA or protein expression in mammals, or to prevent and/or treat relevant diseases or conditions, or to reduce the risk of diseases or conditions.

In some embodiments, the relevant diseases or conditions are defined that an expression level of the LPA protein is 100 nmol/L or more.

In some embodiments, the diseases or conditions include diseases of liver origin.

In some embodiments, the diseases or conditions include inflammatory, cardiovascular and cerebrovascular or metabolic diseases.

In some embodiments, the cardiovascular and cerebrovascular diseases include hyperlipidemia, stroke, atherosclerosis, thrombosis, coronary heart disease or aortic stenosis.

In some embodiments, the drugs include a pharmaceutically acceptable excipient and, preferably, the pharmaceutically acceptable excipient is a PBS buffer or saline solution.

In some embodiments, the drugs are composition that also includes at least one of LDL-C, cholesterol, and triglyceride lowering agents.

In some embodiments, the drugs are composition that also includes a PCSK9 RNAi inhibitor, a PCSK9 antibody inhibitor, and a PCSK9 small molecule inhibitor.

On the other hand, the present application provides a method for reducing LPA mRNA or protein expression in cells or tissues, including exposing cells or tissues to an effective amount of the said LPA RNAi agent or the said drug composition.

In some embodiments, the cells are liver cells.

In some embodiments, the tissues are liver tissues.

In some embodiments, the cells and tissues are in vitro.

In some embodiments, the cells and tissues are located in the body of the subject.

On the other hand, the present application provides a method for reducing LPA mRNA or protein expression in subjects, including the administration of an effective amount of the said LPA RNAi agent or the said drug composition to subjects who need it.

On the other hand, the present application provides a method for the prevention or treatment of diseases or conditions, the method includes the administration of an effective amount of the said LPA RNAi agent or the said drug composition to target subjects.

In some embodiments, the compound or the drug composition is administered to the subject in a manner that is subcutaneous, intravenous, oral, transrectal, or intraperitoneal application.

Beneficial effect: Experiments have proved that the LPA RNAi agent applied in the present application can significantly inhibit the expression of Apo(a) protein and corresponding mRNA in both in vitro and in vivo (mice) experiments. Among them, in mouse experiments, for the expression of Apo(a) protein, up to 99% knockdown inhibition can be achieved at high doses.

### BRIEF DESCRIPTION OF THE DRAWINGS

FIGs. 1a-1b show the sequence of SEQ ID NO. 1.
FIG. 2 shows the experimental results of example 7 of the present application.
FIG. 3 shows the experimental results of example 8 of the present application.
FIG. 4 shows the experimental results of example 9 of the present application.
FIG. 5 shows the experimental results of example 10 of the present application.
FIG. 6 shows the experimental results of example 11 of the present application.

### DETAILED DESCRIPTION OF THE EMBODIMENTS

In order to make the technical problem to be solved, technical scheme and beneficial effect by the present application more clearly understood, the present application is further explained in detail in combination with the attached drawings, examples and chemical reaction formulas. It should be appreciated that the specific Examples described herein are intended only to explain the present application rather than limit the protection scope.

### Definition of terms

In this application, the terms "LPA," "Apo(a)," or "apo(a)" are used interchangeably and generally refer to any nucleic acid or protein sequence encoding an apo(a). For example, in some embodiments, apo(a) includes a DNA sequence encoding apo(a), an RNA sequence transcribed from DNA encoding apo(a) (including genomic DNA containing introns and exons), an mRNA sequence encoding apo(a), or a peptide sequence of apo(a).

In this application, the term "apo(a)-specific inhibitor" refers to any reagent capable of specifically inhibiting the expression of apo(a) nucleic acids and/or apo(a) proteins. For example, apo(a)-specific inhibitors include nucleic acids (including antisense oligonucleotides), peptides, antibodies, small molecules, and other reagents capable of inhibiting the expression of apo(a) nucleic acids and/or apo(a) proteins. In certain embodiments, by specifically modulating apo(a) nucleic acid expression and/or apo(a) protein expression, the apo(a)-specific inhibitor can affect other components of the lipid transport system, including downstream components. Similarly, in some embodiments, the apo(a)-specific inhibitor can affect other molecular processes in the animal.

In this application, the terms "nucleic acid" and "polynucleotide" are used interchangeably and refer to any polymerized form of nucleotide (deoxyribonucleotide or ribonucleotide or analogs thereof) of any length, which can be both double-stranded and single-stranded molecules. The following are non-limiting examples of polynucleotides: genes or gene fragments (e.g., probes, primers, EST or SAGE tags), exons, introns, messenger RNAs (mRNAs), transfer RNAs, ribosomal RNAs, ribozymes, cDNAs, recombinant polynucleotides, branched polynucleotides, plasmids, vectors, isolated DNA of any sequence, isolated RNA of any sequence, nucleic acid probes, siRNA, miRNA, shRNA, RNAi reagents, and primers. The polynucleotide may be modified or substituted at one or more bases, sugars and/or phosphates with any of a variety of modifications or substitutions known in the art. The polynucleotide may comprise modified nucleotides, such as methylated nucleotides and nucleotide analogs. In addition, the nucleotide structure may be modified before or after polymer assembly. The polynucleotide can be modified after polymerization, for example by coupling to a labeled component. Unless otherwise stated or required, any embodiment of the present application as a polynucleotide includes each of the double-stranded form and each of the two complementary single-stranded forms known or predicted to constitute the double-stranded form.

In this application, the terms "target nucleic acid" or "target sequence" generally mean a nucleic acid molecule with which an antisense compound is intended to hybridize to produce a desired effect (e.g., antisense activity). Exemplary antisense compounds include antisense oligonucleotides that are sufficiently complementary to their target nucleic acids to allow hybridization under physiological conditions. In some embodiments, a "target nucleic acid" includes, but is not limited to, DNA or RNA encoding mammalian LPA, such as human LPA mRNA.

In this application, the term "oligonucleotide" generally refers to a polymer comprising a plurality of nucleotide residues (deoxyribonucleotides or ribonucleotides, or related structural variants or synthetic analogs thereof) linked by phosphodiester bonds (or related structural variants or synthetic analogs thereof). Thus, while the term "oligonucleotide" generally refers to a nucleotide polymer in which the nucleotide residues and the linkages between them are naturally occurring, it should be understood that the scope of the term also encompasses a wide variety of analogs including, but not limited to: peptide nucleic acids (PNAs), aminophosphoric acid esters, phosphorothioate esters, methyl phosphonates, 2-O-methyl ribonucleic acids, and the like. The exact size of the molecule can depend on the specific application. Oligonucleotides are generally short in length, usually having about 10-30 nucleotide residues, but the term can also refer to molecules of any length, although the terms "polynucleotide" or "nucleic acid" are generally used for larger oligonucleotides.

In some embodiments, an oligonucleotide comprises one or more unmodified ribonucleosides (RNA) and/or unmodified deoxyribonucleosides (DNA) and/or one or more modified nucleosides. The term "modified oligonucleotide" is generally intended to refer to an oligonucleotide comprising at least one modified nucleoside and/or at least one modified inter-nucleoside linkage.

For the purposes of this application, the term "modified nucleoside" is generally intended to mean a nucleoside that contains at least one chemical modification compared to a naturally occurring RNA or DNA nucleoside. A modified nucleoside comprises a modified sugar portion and/or a modified nucleobase.

In this application, the term "nucleobase" generally means a heterocyclic pyrimidine or purine compound, which is a component of all nucleic acids and includes adenine (A), guanine (G), cytosine (C), thymine (T) and uracil (U). Nucleotides may include modified nucleotides or nucleotide mimics, abasic sites (Ab or X), or surrogate replacement moiety. As used herein, "nucleobase sequence" generally means a sequence of consecutive nucleobases that does not depend on any sugar, bonding or nucleobase modification. The terms "unmodified nucleobases" or "naturally occurring nucleobases" generally mean the naturally occurring heterocyclic nucleobases of RNA or DNA: the purine bases adenine (A) and guanine (G); and the pyrimidine bases thymine (T), cytosine (C) (including the 5-phosphorothioate), and uracil (U). "Modified nucleobase" generally means any nucleobase that is not a naturally occurring nucleobase.

For the purposes of this application, the term "sugar portion" generally means the naturally occurring sugar portion or a modified sugar portion of a nucleoside. The term "naturally occurring sugar portion" generally means a furanosyl group such as found in naturally occurring RNA or a deoxyfuranosyl group such as found in naturally occurring DNA. "Modified sugar fraction" means a substituted sugar fraction or sugar substitute.

In this application, the term "inter-nucleoside linkages" generally means covalent linkage between neighboring nucleosides in an oligonucleotide. "Naturally occurring inter-nucleoside linkage" means a 3' to 5' phosphodiester linkage. "Modified inter-nucleoside linkage" means any inter-nucleoside linkage other than a naturally occurring inter-nucleoside linkage.

In this application, the term "antisense oligonucleotide" refers to a single-stranded oligonucleotide molecule having a sequence of nucleobases that is complementary to a corresponding fragment of a target nucleic acid (e.g., a target genomic sequence, an mRNA precursor, or an mRNA molecule). In certain embodiments, the antisense oligonucleotide is from 12 to 30 nucleobases in length. In certain embodiments, the antisense oligonucleotide is an unmodified or modified nucleic acid having a nucleotide sequence that is complementary to the sequence of a target nucleic acid (e.g., an LPA polynucleotide).

As used herein, the term "antisense strand" generally refers to a strand of an RNAi agent (e.g., dsRNA) that includes a region that is substantially complementary to a target sequence. As used herein, the term "complementary region" generally refers to a region of the antisense strand that is substantially complementary to a sequence defined herein (e.g., a target sequence). When the complementary region is not fully complementary to the target sequence, the mismatch can be in the internal or terminal region of the molecule. Typically, the most tolerated mismatches are in the terminal region, e.g., within 5, 4, 3, or 2 nucleotides at the 5' end and/or the 3' end.

For the purposes of this application, the term "sense strand" (S) generally refers to such a strand of an RNAi agent that comprises a region that is substantially complementary to a region that is the antisense strand of the term as defined herein. "Sense" strands are sometimes referred to as "coding" strands. By virtue of their sequences, antisense strands target the desired mRNA, while sense strands target a different target. Thus, if the antisense strand is incorporated into the RISC, the correct target is targeted. Doping of the sense strand can lead to off-target effects. These off target effects can be limited by using modifications on the sense strand or by using a 5' end cap.

In this application, the term "complementary" when used to describe a first nucleotide sequence (e.g., an RNAi agent antisense strand) with respect to a second nucleotide sequence (e.g., an RNAi agent sense strand or LPA mRNA) refers to the ability of an oligonucleotide or polynucleotide comprising a first nucleotide sequence to hybridize (form base-to-base hydrogen bonds) and form a double-stranded body or double-helix structure with an oligonucleotide or polynucleotide comprising a second nucleotide sequence under certain conditions. The ability of an oligonucleotide or polynucleotide to hybridize (form base-pair hydrogen bonds) with an oligonucleotide or polynucleotide containing a second nucleotide sequence under certain conditions and form a double-stranded body or double-helix structure. Complementary sequences include Watson-Crick base pairs or non-Watson-Crick base pairs, and include natural or modified nucleotides or nucleotide mimics as long as the above requirements with respect to their ability to hybridize are met. "Complementary" does not require base complementarity on every nucleoside. On the contrary, some mismatches can be tolerated.

In this application, the term "fully complementary" generally means that all (100%) of the bases in the continuous sequence of the first polynucleotide will hybridize to the same number of bases in the continuous sequence of the second polynucleotide. The continuous sequence may comprise all or a portion of the first or second nucleotide sequence. As used herein, "partially complementary" generally means that at least about 70% of the bases in the consecutive sequence of the first polynucleotide will hybridize to the same number of bases in the consecutive sequence of the second polynucleotide in the hybridized base sequence pair. As used herein, the term "substantially complementary" generally means that in the hybridized base sequence pair, at least about 90% of the bases in the contiguous sequence of the first polynucleotide will hybridize to the same number of bases in the contiguous sequence of the second polynucleotide. As used herein, the terms "complementary," "fully complementary," and "substantially complementary" may be used in connection with the hybridization of bases between the positive and negative strands of an RNAi agent or between the negative strand of an RNAi agent and the negative strand of an LPA mRNA. strand of the RNAi agent or between the antisense strand of the RNAi agent and the sequence of the LPA mRNA. Sequence identity or complementarity is not dependent on modification. For the purpose of determining identity or complementarity, for example, A and Af are complementary to U (or T) and identical to A.

In this application, the term "ligand" generally refers to any compound or molecule that is capable of covalently or otherwise chemically binding to a biologically active substance, such as an oligonucleotide. In some embodiments, the ligand is capable of interacting directly or indirectly with another compound, such as a receptor, the receptor with which the ligand interacts may be present on the surface of a cell, or alternatively may be an intracellular and/or intercellular receptor, and the interaction of the ligand with the receptor may result in a biochemical reaction or may be merely a physical interaction or binding.

In this application, the terms "induced", "inhibited", "enhanced", "elevated", "increased", "reduced", "decreased", etc. generally denote a quantitative difference between two states. For example, "effective inhibition of apo(a) activity or expression" means that the level of apo(a) activity or expression in a treated sample will be lower than the level of apo(a) activity or expression in an untreated sample. Said terminology applies, for example, to the expression level and the activity level. The terms "reduced" and "decreased" are used interchangeably and generally indicate any change that is less than the original. The terms "reduced" and "decreased" are relative terms and need to be compared between pre- and post-measurement. "Reduction" and "decrease" include total depletion.

In some embodiments, the term "reduced" can mean an overall reduction in the level/amount of expression of a gene, gene product, e.g., protein, or biomarker in a first sample compared to the level/amount of expression of the corresponding gene, gene product, e.g., protein, or biomarker in a second sample, as detected by a standardized method known in the art (such as those described in the present application) of about 5% to 95%, or 100% overall reduction. In some embodiments, the term "reduced" refers to a reduction in the expression level/amount of the gene or biomarker in the subject sample, wherein the reduction is at least about 0.9-fold to 0.01-fold of the expression level/amount of the corresponding gene or biomarker.

For purposes of this application, the term "expression" generally refers to the process by which a gene ultimately produces a protein. Expression includes, but is not limited to, transcription, post-transcriptional modifications (e.g., splicing, polyadenylation, addition of a 5'-cap), and translation.

In this application, the term "pharmaceutically acceptable" generally refers to one or more non-toxic substances that do not interfere with the effectiveness of the biological activity of the active ingredient. Such formulations may typically contain salts, excipients, buffers, preservatives, compatible carriers and optionally other therapeutic agents. For use in medicine, the salt should be a pharmaceutically acceptable salt, but non-pharmaceutically acceptable salts may be conveniently used to prepare pharmaceutically acceptable salts and they cannot be excluded from the scope of the present application. Such pharmacologically and pharmaceutically acceptable salts include salts prepared from the following acids: hydrochloric acid, hydrobromic acid, sulfuric acid, nitric acid, phosphoric acid, maleic acid, acetic acid, salicylic acid, citric acid, boric acid, formic acid, malonic acid, succinic acid and the like. Pharmaceutically acceptable salts may also be prepared as alkali metal salts or alkaline earth metal salts, such as sodium, potassium or calcium salts.

For the purposes of this application, the term "preventing and/or treating" includes not only preventing and/or treating a disease, but also generally includes preventing the onset of a disease, slowing or reversing the progression of a disease, preventing or slowing the onset of one or more symptoms associated with a disease, reducing and/or mitigating one or more symptoms associated with a disease, reducing the severity and/or duration of a disease and/or any symptoms associated with it, and/or preventing a further increase in the severity of a disease and/or any symptoms associated with it, preventing, reducing or reversing any physiological impairment caused by a disease, and generally, for the purposes of this application, preventing, mitigating or reversing any physical impairment caused by a disease. severity and/or duration of any symptom associated therewith and/or preventing a further increase in the severity of the disease and/or any symptom associated therewith, preventing, reducing, or reversing any physiological impairment caused by the disease, and any pharmacological effect that would ordinarily be of benefit to the patient being treated. The RNAi agents or pharmaceutical compositions of the present application form viable therapeutic agents need not achieve a complete cure or eradication of any symptom or manifestation of the disease. As recognized in the relevant field, a drug used as a therapeutic agent may reduce the severity of a given disease state, but does not need to eliminate every manifestation of the disease to be considered a useful therapeutic agent. Similarly, a prophylactically administered treatment constituting a viable prophylactic agent need not be completely effective in preventing the onset of the disease. Simply reducing the effects of the disease in a subject (e.g., by reducing the number or severity of its symptoms, or by increasing the effectiveness of another treatment, or by producing another beneficial effect) or reducing the likelihood of the disease occurring or worsening is sufficient.

For the purposes of this application, the terms "disease" or "illness" are used interchangeably and generally refer to any deviation from the normal state of the subject, such as any change in the state of the body or of certain organs, which interferes with or disrupts the fulfillment of functions and/or causes symptoms such as discomfort, dysfunction, pain or even death, either in the person who is ill or in contact with the subject. contact, causing symptoms such as discomfort, dysfunction, suffering or even death. Diseases or conditions may also be referred to as disorders (distemper), discomfort (ailing), minor ailments (ailment), illness (malady), disorders (disorder), sickness (sickness), disease (illness), bodily discomfort (compliant), inderdisposion or affectation.

For the purposes of this application, the term "administration" generally refers to the introduction of a pharmaceutical formulation of the present application into the body of a subject by any route of introduction or delivery. Any method known to those skilled in the art for contacting cells, organs or tissues with said drug may be employed. Said administration may include, but is not limited to, intravenously, intra-arterially, intranasally, intraperitoneally, intramuscularly, subcutaneously, or orally. The daily dose may be divided into one, two or more doses in a suitable form to be administered at one, two or more times during a certain time period.

For the purposes of this application, the term "contact" generally refers to two or more substances of different types coming into contact in any order, in any manner, and for any duration. The contact may occur in vivo, indirectly in vivo, or in vitro. In some embodiments, it may mean that the RNAi agent or composition of the present application is brought into direct contact with a cell or tissue. In other embodiments, the term refers to causing an RNAi agent or composition of the present application to indirectly contact a cell or tissue. For example, methods of the present application include methods in which a subject is exposed to an RNAi agent or composition of the present application, and then the RNAi agent or composition contacts a cell or tissue by diffusion or any other active transport or passive transport process known in the art through which the compound circulates in the body.

For the purposes of this application, the term "effective amount" or "effective dose" generally refers to an amount sufficient to achieve, or at least partially achieve, the desired effect. A "therapeutically effective amount" or "therapeutically effective dose" of a drug or therapeutic agent is generally one that, when used alone or in combination with another therapeutic agent, promotes the regression of a disease (which is accomplished by a reduction in the severity of symptoms of the disease, an increase in the frequency and duration of symptom-free periods of the disease, or an increase in damage or impairment due to the development of the disease). (as evidenced by a reduction in the severity of disease symptoms, an increase in the frequency and duration of symptom-free periods of disease, or the prevention of damage or disability due to the development of a disease). A "prophylactically effective amount" or "prophylactically effective dose" of a drug generally refers to an amount of the drug that inhibits the progression or recurrence of a disease when administered alone or in combination with another therapeutic agent to a subject who is at risk for disease progression or disease recurrence. In some embodiments, an "effective amount" refers to an amount of an RNAi agent that produces a desired pharmacologic, therapeutic, or prophylactic result.

For the purposes of this application, the term "subject" generally refers to a human or non-human animal (including mammals), such as humans, non-human primates (apes, gibbons, gorillas, chimpanzees, orangutans, rhesus macaques), domestic animals (dogs and cats), farm animals (horses, cattle, goats, sheep, pigs), and laboratory animals (mice, rats, rabbits, guinea pigs), in which the disease needs to be diagnosed, prognosticated, ameliorated, prevented, and/or treated, sheep, pigs) and laboratory animals (mice, rats, rabbits, guinea pigs). Human subjects included fetal, neonatal, infant, adolescent, and adult subjects. Subjects included animal disease models.

In this application, the terms "includes", "contains", "has", "may", "comprises" and variations thereof are generally intended to be open-ended transitional phrases, terms or words that do not exclude the possibility of additional behavior or structure. The term "consistis of" generally indicates that no other component (or, similarly, feature, integer, step, etc.) can exist.

The term "a," "an," and "the" includes plural reference unless the context clearly dictates otherwise..

When the term "approximately" is used to refer to a range of values, a cut-off or specific value is used to indicate that the value stated may vary by up to 10% from the enumerated value. Thus, the term "about" may be used to cover a variation of ± 10% or less, ± 5% or less, ± 1% or less, ± 0.5% or less, or ± 0.1% or less from a specified value.

### LPA RNAi agent

The RNAi agents described herein for inhibiting the expression of the LPA gene (referred to in this application as LPA RNAi agents) inhibit or knock down the expression of LPA in vitro and/or in vivo by the biological process of RNA interference (RNAi) after delivery to cells expressing the LPA gene. As used in this application, unless otherwise specifically indicated, LPA may refer to the LPA gene, LPA mRNA, or LP(a) protein, where appropriate. RNAi agents include, but are not limited to: short interfering RNA (siRNA), double-stranded RNA (dsRNA), microRNA (miRNA), and short hairpin RNA (shRNA).

On the one hand, the present application provides an LPA RNAi agent that can comprise an oligonucleotide targeting a nucleic acid molecule encoding LPA (apo(a)), wherein said oligonucleotide comprises at least 12, at least 13, at least 14, at least 15, at least 16, at least 17, at least 18 of a nucleotide sequence of any one of SEQ ID NO: 2 to SEQ ID NO: 183, at least 19, at least 20, at least 21, at least 22 or at least 23 consecutive nucleotides. Said nucleotide sequence may be in a modified state (and modified with ligand) and in an unmodified original state.

In certain embodiments, said oligonucleotide comprises from 12 to 30 nucleotides. For example, said oligonucleotide may be 12, 13, 14, 15, 16, 17, 18, 19, 20, 21, 22, 22, 23, 24, 25, 26, 27, 28, 29, or 30 nucleotides long. By way of further example, said length of said oligonucleotide may be 17, 18, 19, 20, 21, 22, 23, 24, 25 or 26 nucleotides long.

In certain embodiments, wherein said oligonucleotide comprises a nucleotide sequence selected from: any one of SEQ ID NO: 2 to SEQ ID NO: 183 or a sequence that differs from it by no more than three nucleotides. For example, when up to one, two, or three nucleotide substitutions (e.g., adenosine is replaced by uracil), deletions, or additions occur, different from the nucleotide sequence shown in any one of SEQ ID NO: 2 to SEQ ID NO: 183, a derived RNAi agent can still have reduced inhibitory activity, as compared to the nucleotide sequence shown in any one of SEQ ID NO: 2 to SEQ ID NO: 183, which serves as the source of the derivative. 183 of any of the nucleotide sequences shown in any one of SEQ ID NO: 2 to SEQ ID NO: 183, no less than 20% inhibition.

In certain embodiments, wherein said oligonucleotide is a single-stranded oligonucleotide or a double-stranded oligonucleotide.

For example, said oligonucleotide may be an antisense oligonucleotide. Another example is that said oligonucleotide is siRNA.

For example, said oligonucleotide may comprise only the antisense strand. For another example, said oligonucleotide may comprise a sense strand with an antisense strand.

In certain embodiments, said the length of LPA RNAi agent sense and antisense strands are independently 17 to 30 nucleotidesin length. In certain embodiments, the sense and antisense strands are independently 17 to 26 nucleotides in length. In certain embodiments, the sense and antisense strands are 19 to 26 nucleotides in length. In certain embodiments, said RNAi agent sense and antisense strands are independently 17, 18, 19, 20, 21, 22, 23, 24, 25, or 26 nucleotides in length. The sense and antisense strands can be either the same length or can be different lengths. In certain embodiments, the sense and antisense strands are each 26 nucleotides in length. In certain embodiments, the sense strand is 23 nucleotides in lengthand the antisense strand is 21 nucleotides in length. In certain embodiments, the sense strand is 22 nucleotides in length and the antisense strand is 21 nucleotides in length . In certain embodiments, the sense strand is 21 nucleotides in length and the antisense strand is 21 nucleotides in length. In certain embodiments, the sense strand is 19 nucleotides in length and the antisense strand is 21 nucleotides in length.

In some embodiments, the antisense strand sequence is 100% (perfect) complementary or at least 90% (substantially) complementary to a nucleotide sequence (sometimes referred to as a target sequence) present in the LPA mRNA. The sense strand sequence is 100% (perfectly) complementary or at least 90% (substantially) complementary to the sequence in the antisense strand, and thus the sense strand sequence is perfectly identical or at least 90% identical to the nucleotide sequence present in the LPA mRNA (the target sequence).

In certain embodiments, the sense and antisense strands of said LPA RNAi agent are annealed to form a double-stranded body. the sense and antisense strands of the LPA RNAi agent are partially, substantially, or fully complementary to each other. Within the region of the complementary duplex region, the sense strand core sequence is at least 90% complementary or 100% complementary to the antisense core sequence. In some embodiments, the sense strand core sequence comprises a sequence of at least 17, at least 18, at least 19, at least 20, or at least 21 nucleotides that is at least 90% or 100% complementary to a corresponding 17, 18, 19, 20, or 21 nucleotide sequence of the antisense strand core sequence (i.e., the sense strand and antisense core sequences of the LPA RNAi agent have a region of at least 17, at least 18, at least 19, at least 20, or at least 21 nucleotides that is at least 90% base-paired or 100% base-paired ).

In some embodiments, the senes strand and/or the antisense strand may optionally and independently contain an additional 1, 2, 3, 4, 5, or 6 nucleotides (extension) at the 3' end and/or the 5' end of the core sequence. If extension nucleotide is on antisense strand, it is or not is complementary to the corresponding sequence in the LPA mRNA. The sense strand may or may not be identical to the corresponding sequence in the LPA mRNA if extension nucleotide is present. If extension nucleotide is on antisense strand, it may or may not be complementary to the corresponding sense strand.

In some embodiments, said LPA RNAi agent comprises an antisense strand having a 3' extension of 1, 2, 3, 4, 5, or 6 nucleotides in length. In other embodiments, said LPA RNAi agent comprises an antisense strand having a 3' extension of 1, 2, or 3 nucleotides in length. In some embodiments, the one or more of the antisense strand extension nucleotides comprise uracil or thymidine nucleotides or nucleotides which are complementary to the corresponding LPA mRNA sequence.

In some embodiments, the LPA RNAi agent comprises an antisense strand having a 5' extention of 1, 2, 3, 4, or 5 nucleotides in length. In other embodiments, the LPA RNAi agent comprises an antisense strand having a 5' extension of 1 or 2 nucleotides in length. In some embodiments, the one or more antisense strand extension nucleotides comprise uracil or thymidine nucleotides or nucleotides which are complementary to the corresponding LPA mRNA sequence. The antisense strand may have any of the 3' extensions described above in combination with any of the 5' antisense strand extensions aboved described.

In some embodiments, said LPA RNAi agent comprises a sense strand having a 3' extension of 1, 2, 3, 4, or 5 nucleotides in length. In certain embodiments, the one or more of the sense strand extension nucleotides comprise adenosine, uracil or thymidine nucleotides, AT dinucleotides, or nucleotides corresponding to nucleotides in the LPA mRNA sequence.

In some embodiments, the LPA RNAi agent comprises a sense strand having a 5' extension of 1, 2, 3, 4, 5, or 6 nucleotides in length. In certain embodiments, the one or more of the sense strand extension nucleotides comprise uracil or adenosine nucleotides or nucleotides corresponding to nucleotides in the LPA mRNA sequence. The sense strand may have a 3' extension and/or a 5' extension.

In some embodiments, the said antisense strand comprises a nucleotide sequence of: any one of SEQ ID NO: 2 to SEQ ID NO: 92 or a sequence that differs by no more than 3 nucleotides therefrom.

In certain embodiments, the said sense strand comprises a nucleotide sequence of any one of SEQ ID NO: 93-183 or a sequence that differs by no more than 3 nucleotides therefrom.

In some embodiments, the antisense and sense strand sequences of said LPA RNAi agent are shown in Table 1.

The LPA RNAi agent described in this application can be formed by annealing the antisense strand with a sense strand. A sense strand containing a sequence listed in Table 1 may be hybridized to any antisense strand containing a sequence listed in Table 1 provided the two sequences have a region of at least 90% complementary over a contiguous 16, 17, 18, 19, 20 or 21 nucleotides sequence.

In some embodiments, the LPA RNAi agent antisense strand comprises a nucleotide sequence of any antisense strand sequence in Table 1. In other embodiments, the LPA RNAi agent antisense strand comprises a sequence of nucleotides 1-17, 2-17, 1-18, 2-18, 1-19, 2-19, 1-20, 2-20, 1-21, 2-21, 1-22, 2-22, 3-22, 1-23, 2-23, 3-23, or 4-23 of any antisense strand sequences in Table 1. In some embodiments, the LPA RNAi agent sense strand comprises the nucleotide sequence of any of the sensestrand sequences in Table 1. In some embodiments, the LPA RNAi agent sense strand comprises a sequence of nucleotides 1-17, 2-17, 1-18, 2-18, 1-19, 2-19, 1-20, 2-20, 1-21, or 2-21 of any of the sense sequences in Table 1.

In some embodiments, the sense and antisense strands of the LPA RNAi agents described in this application contain the same number of nucleotides. In certain embodiments, the sense and antisense strands of the LPA RNAi agent described in this application contain different numbers of nucleotides. In certain embodiments, the sense strand 5' end and the antisense strand 3' end of the LPA RNAi agent form a blunt end. In certain embodiments, the sense strand 3' end and the antisense strand 5' end of the LPA RNAi agent form a blunt end. In certain embodiments, both ends of the RNAi agent are blunt ends. In certain embodiments, neither end of the LPA RNAi agent is blunt-ended. As used in the present application, a blunt end is an end of a double-stranded RNAi agent in which the terminal nucleotides of the two annealed strands are complementary (form complementary base-pair). In certain embodiments, the sense strand 5' end and the the antisense strand 3' end of the LPA RNAi agent form a frayed end. In certain embodiments, the sense strand 3' end and the antisense strand 5' end of the RNAi agent form a frayed end. In certain embodiments, both ends of the LPA RNAi agent form a frayed end. In certain embodiments, neither end of the LPA RNAi agent is a frayed end. As used in the present application, a frayed end refers to an end of a double-stranded RNAi agent in which the terminal nucleotides of the two annealed strands form pair (i.e., do not form an overhang), but are not complementary (i.e., form non-complementary pair). In the present application, an overhang is a stretch of one or more unpaired nucleotides at the end of one strand of a double-stranded RNAi agent. The unpaired nucleotides may be on the sense or antisense strand, creating a 3' or 5' overhangs. In certain embodiments, the LPA RNAi agent contains: a blunt end and a frayed end, a blunt end and a 5'overhang end, a blunt end and a 3' overhang end, a frayed end and a 5' overhang end, a frayed end and a 3' overhang end, two 5 ' overhang ends, two 3' overhang ends, 5'overhang ends and 3' overhang ends, twofrayed ends, or two blunt ends.

In some embodiments, the LPA RNAi agent is prepared as a salt, mixed salt, or free acid.

### Modified Nucleotide

The LPA RNAi agent of the present application include unmodified nucleotides and modified nucleotides to enhance efficacy, and polymers of nucleoside substitutes. Unmodified nucleotides are those in which the sugar, base, and phosphate structural components are the same or substantially the same as those found naturally, preferably in the human body. The prior art treats rare or unusual but naturally occurring RNAs as modified RNAs, see Limbach et al, (1994) Nucleic Acids Res. 22: 2183-2196. Such rare or unusual modified RNAs are often referred to as modified RNAs (obviously as they are the results of post-transcriptional modifications), and are unmodified RNAs in the present application. Modified RNAs as used in the present application are those in which the components of the nucleotide, i.e., the one or more components of the sugar, base and phosphate structure, are different from the natural components, preferably different from the natural components produced from the human body. Nucleoside substitutes are molecules in which the ribose phosphate backbone is replaced by a non-ribose phosphate construct that places the bases in the correct spatial relationship. Therefore, the hybridization is substantially similar to that seen with ribose phosphate backbones, e.g., analogs of non-charged ribose phosphate backbones.

In some embodiments, the LPA RNAi agent comprises one or more modified nucleotides.

In some embodiments, at least 50%, at least 60%, at least 70%, at least 80%, at least 90%, at least 95%, or 100% of the nucleotides in the LPA RNAi agent are modified.

In certain embodiments, wherein said sense and/or antisense strand independently comprises one or more modified nucleotides.

The two main purposes of modifying RNAi agents are to obtain their stability against degradation in the biological environment and to improve pharmacological properties, such as pharmacodynamic properties. RNAi agents can contain non-naturally occurring bases or non-naturally occurring sugars, such as non-sugar analogs of cyclic carrier molecules, which are typical of the non-naturally occurring sugars used in RNAi reagents. RNAi reagents may include inter-nucleoside linkages (e.g., chiral phosphorothioates linkages) between nucleotides to increase nuclease resistance. RNAi reagents may additionally include, or optionally include, riboseanalogues to increase nuclease resistance.

Modified nucleotides include, but are not limited to, deoxyribonucleotides, nucleotide mimics, abasic nucleotides ( represented herein as X, Ab), 2'-modified nucleotides, 3' to 3-bonded (reverted) nucleotides (represented herein as invdN, invN, invn, invX, invAb, non-natural base-comprising nucleotides , bridged nucleotides, peptide nucleic acids (PNAs), 2',3'-seco nucleotide mimics (unlocked nucleobase analogues, represented herein as N_{UNA} or NUNA), locked nucleotides (represented herein as N_{LNA} or NLNA), 3 '-O-Methoxy (2' inter-nucleoside-linked) nucleotide (represented herein as 3'-OMen), 2'-F-Arabinose nucleotide (represented herein as NfANA or Nf_{ANA}), 5 '-Me,2'-fluorinated nucleotides (represented herein as 5Me-Nf), morpholino nucleotides, vinyl phosphonate deoxyribonucleotides (represented herein as vpdN), vinyl phosphonate containing nucleotides, and cyclopropyl phosphonate containing nucleotides (cPrpN). 2' -modified nucleotides (i.e., a nucleotide with a group other than a hydroxyl group at the 2' position of the five-membered sugar ring) include, but are not limited to, 2'-O-methyl nucleotides ( represented herein as the lowercase letter n in a nucleotide sequence), 2'-deoxy-2'-fluoro nucleotides (represented herein as Nf, and also d represented herein as 2'-fluoro nucleotide), 2'-deoxy nucleotides (represented herein as dN), 2'-methoxyethyl (2'-O-2-methoxyethyl) nucleotides (represented herein as NM or 2 '-MOE), 2'-amino nucleotides, and 2'-alkyl nucleotides. Specifically, more than one modification can be incorporated in a single LPA RNAi agent or even in a single nucleotide thereof. LPA RNAi agent sense and antisense strands may be synthesized and/or modified by methods known in the art. The modification at one nucleotide is independent of the modification at the other nucleotide.

Modified nucleotides also include nucleotides having modified nucleobases. Modified nucleobases include, but are not limited to, synthetic and natural nucleobases, 5-substituted pyrimidines, 6-azopyrimidines and N-2, N-6 and O-6 substituted purines including 2-aminopropyladenine, 5-propargyluracil and 5-propargylcytosine, 5-methylcytosine (5-me-C), 5-hydroxymethylcytosine, 2-propyl and other alkyl derivatives of xanthine, adenine and guanine, 2-thiothymine and 2-thiocytosine, 5-halogenated uracil and cytosine, 5-propargyl uracil and cytosine, 6-azo uracil, cytosine and thymine, 8-halo, 8-amino, 8-thiol, 8-thioalkyl, 8-hydroxy and other 8-substituted adenine and guanine, 5-halo, in particular 5-bromo, 5-trifluoromethyl and other 5-substituted uracil and cytosine, 7-methylguanine and 7-methyladenine, 8-azaguanine and 8-azaguanine.

In certain embodiments, the one or more nucleotides of the LPA RNAi agent are linked by non-standard linkages or backbone (i.e., modified inter-nucleoside linkages or modified backbone). In some embodiments, the modified inter-nucleoside linkage is a non-phosphate containing covalent inter-nucleoside linkage. The modified inter-nucleoside linkage or backbone includes a 5'-phosphorothioate moiety having a normal 3'-5' linkage (represented herein as a lowercase s before the nucleotide, as in sN, sn, sNf, or sdN), a chiral phosphorothioate, a thiophosphate (thiophosphate), phosphorodithioate, phosphotriesters, aminoalkyl-phosphotriesters , methyl and other alkyl phosphonates including 3'-alkylenephosphonate and chiral phosphonates, phosphinate (phosphinate), phosphoramidates including 3'-amino phosphoramidate and aminoalkylphosphoramidates, thionophosphoramidates, thionoalkyl-phonates, thionnoalkuulphosphotriesters, morpholino linkages, and boranophosphates ; 2'-5' linked analogs of these, and those having inverted polarity, wherein adjacent pairs of nucleoside units are linked 3'-5' to 5'-3' or 2'-5' to 5'-2'. In other embodiments, the modified nucleoside linkage or backbone is lack of phosphorus atoms. Modified inter-nucleoside linkages without phosphorus atoms include, but are not limited to, short chain alkyl or cycloalkyl inter-sugar linkages, mixed heteroatom and alkyl or cycloalkyl inter-sugar linkages, one or more short chain heteroatomic or heterocyclic inter-sugar linkages. In certain embodiments, modified inter-nucleoside backbones include, but are not limited to, siloxane backbones, sulfide, sulfoxide, and sulfone backbones; formacetyl (formacetyl) and thiocarbonyl backbones, methylene formacetyl and thioformacetyl backbones, alkene-containing backbones, sulfamate backbones, methyleneimino and methylenehydrazino backbones, sulfonate and sulfonamide backbones, amide backbones; and others having a mixed N, O, S, and CH₂ component parts.

### Ligand

In certain embodiments, wherein said LPA RNAi agent further comprises a ligand.

In certain embodiments, the ligand alters the distribution, targeting, or lifetime of the RNAi agent to which it binds. In certain embodiments, the ligand enhances the affinity to the target compared with that lacking the ligand. The selected target is, for example, a molecule, a cell or class of cells and a lumen such as a cell or organ lumen, a tissue, an organ, or a region of the body.

In certain embodiments, the ligand is capable of promoting transport, hybridization, and specificity properties, and is capable of promoting nuclease resistance of the obtained natural or modified oligoribonucleotide, or promoting nuclease resistance of a multimeric molecule comprising any one of the monomers and/or conjugates of the natural or modified ribonucleotides described in this application.

For example, the ligand may also include a targeting moiety, e.g., a cell- or tissue-targeting reagent such as a lectin, a glycoprotein, a lipid, or a protein, such as an antibody that binds to a particular cell such as a hepatocyte or a jejunal cell. The targeting moiety may be thyrotropin, melanocyte stimulating hormone, lectins, glycoproteins, surfactant protein A, mucin carbohydrates, polyvalent lactose, polyvalent galactose, N-acetylgalactosamine, N-acetylglucosamine, polyvalent mannose, polyvalent alginate, glycosylated polyamino acids, polyvalent galactose, iron-transporting proteins, diphosphonates, polyglutamates, polyaspartates, lipids, cholesterol, steroids, bile acids, folic acid, vitamin B12 or biotin.

In some embodiments, said ligand comprises a targeting moiety.

In some embodiments, the targeting moiety may be monovalent, bivalent, trivalent, tetravalent, or have higher valency. Representative targeting moieties include compounds with affinity to cell surface molecules, cell receptor ligands, hapten, antibodies, monoclonal antibodies, antibody fragments, and antibody mimics with affinity to cell surface molecules.

In certain embodiments, wherein said targeting moiety is capable of targeting hepatocytes or the liver.

In certain embodiments, wherein said targeting moiety comprises an asialogycoprotein receptor ligand.

In certain embodiments, the asialogycoprotein receptor ligand includes or consists one or more galactose derivatives or galactose clusters. In the present application, the term galactose derivative includes both galactose, and derivates of galactose having affinity for the asialogycoprotein receptor that is equal to or greater than that of galactose. Galactose derivatives include: galactose, galactosamine, N-formyl-galactosamine, N-acetyl-galactosamine, N-propionyl-galactosamine, N-n-butanoyl-galactosamine, and N-iso-butanoyl-galactosamine. Galactose derivatives have been used to target molecules to hepatocytes in vivo by binding to the asialogycoprotein receptor (ASGPr) on the surface of hepatocytes. Binding of the ASGPr ligand to the ASGPr facilitates cell-specific targeting to hepatocytes and endocytosis of the molecules into hepatocytes. The galactose cluster can be attached to the 3' or 5' end of the RNAi polynucleotide using methods known in the art.

In certain embodiments, wherein said ligand is linked to said sense and/or antisense strand. For example, said ligand may be linked to the 5' end and/or the 3' end of the sense and/or antisense strand.

### delivery vehicles

In certain embodiments, a delivery vehicle can be used to deliver an LPA RNAi agent to a cell or tissue. A delivery vehicle is a compound that improves delivery of an LPA RNAi agent to a cell or tissue. The delivery vehicle may comprise or consist of: a polymer, such as an amphiphathic polymer, a reversibly modified polymer or peptide, or a reversibly modified membrane-active polyamine.

In certain embodiments, the LPA RNAi agent can be combined with lipids, nanoparticles, polymers, liposomes, micelles, or other delivery systems available in the art. The LPA RNAi agent may also be chemically conjugated to a targeting moiety, lipids (including cholesterol and cholesteryl derivatives), nanoparticles, polymers, or other delivery systems available in the art.

The present application describes methods for delivery of LPA RNAi agent to mammalian hepatocytes in vivo. In some embodiments, a delivery vehicle is used. The delivery vehicle may be, but is not limited to, polymers (e.g. amphipathic polymers, membrane active polymers), ligands, etc. In some embodiments, the LPA RNAi agent is linked to a targeting ligand that comprises an asialogycoprotein ligand. For example, LPA RNAi agents can be linked to a targeting ligand that comprises or consists of galactose clusters.

As mentioned above, this present application provides a siRNA conjugate with the following general formula, 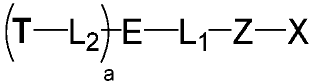 (I-0) or

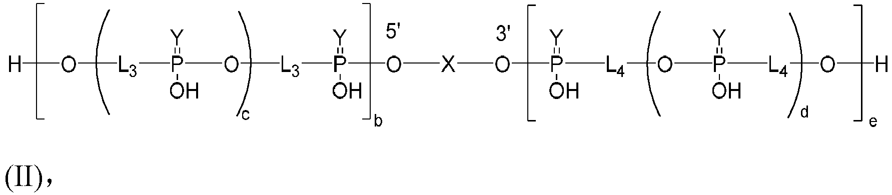

### In the general formula (I) :

X is the nucleotide sequence, which can be the sense strand or antisense strand of siRNA, and Z is the first connector part of the nucleotide sequence, where Z and X can be connected directly or through chemical groups, and the general formula of Z is shown as (Z-1), where one end of the O group is connected with the nucleotide sequence:

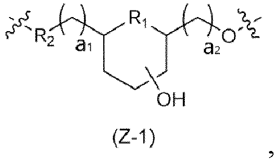

Where R₁ is O, S, NR₃ or CR₃R₄, where R₃ and R₄ are independently hydrogen, halogen, substituted or unsubstituted aliphatic group, substituted or unsubstituted aryl group, substituted or unsubstituted heteraryl group, or substituted or unsubstituted heterocyclic ring or substituted or unsubstituted cycloalkyl group;

Wherein R₂ is -O-, -S-, -NH-, -CH₂-, -C(O)-, -OC(O)-, - C(O)O-, -NHC(O)-, -C (O)NH-, -CH₂NH-, -CH₂O-, -NH-C(O)-CH₂-, -C(O)-CH₂-NH- or -NH(CO)NH-, where the -CH₂- may optionally be substituted by one of the substituents selected from halogens, alkyl groups, alkoxy groups, and alkane amino groups; The a₁ and a₂ are the same or different, and are selected from integers of 0 to 20, preferably integers of 1 to 10, and further preferably integers of 1 to 5.

Preferably, the nucleotide sequence represented by X is the first nucleotide sequence described above.

Preferably, the first connector part Z is connected to the second connector part L₁, and the second connector part L₁ is connected to the branch point group E.

Preferably, the branch point group E is connected with targeting assemblies that the quantity is a, and the a is an integer selected from 0 to 10, preferably an integer from 1 to 5; The targeting assembly includes a tethered part L₂ and a targeting part T in a 1:1 ratio.

### In general formula (II) :

L₃ includes a third connector part, L₄ is a targeting part, specifically, T in general formula (I) and L₃ and L₄ in general formula (II) can be the same or different, preferably, L₃ and L₄ are respectively the following structure:

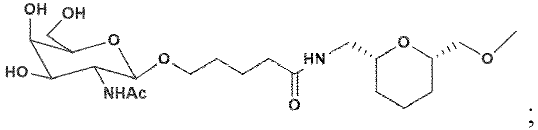

Wherein X is a nucleotide sequence, which can be a sense strand or an antisense strand,
YisOorS,

Where b, c, d, and e are integers from 0 to 10 respectively, and b and e are 0 when they are different. In one embodiment, b is 0 and e is an integer from 3 to 6.

On the other hand, the second connector part L₁ has the following structure:

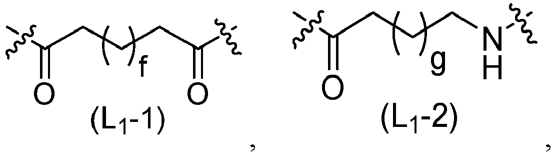

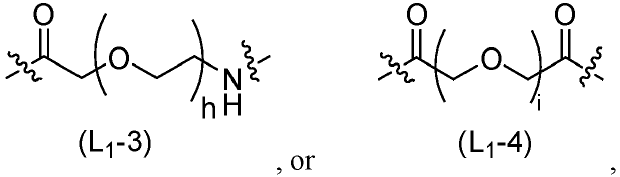

Where f, g, h, and i are integers from 1 to 20 respectively, preferably integers from 1 to 10.

In one embodiment, the position of L₁ in the ligand is shown in the following frame structure:

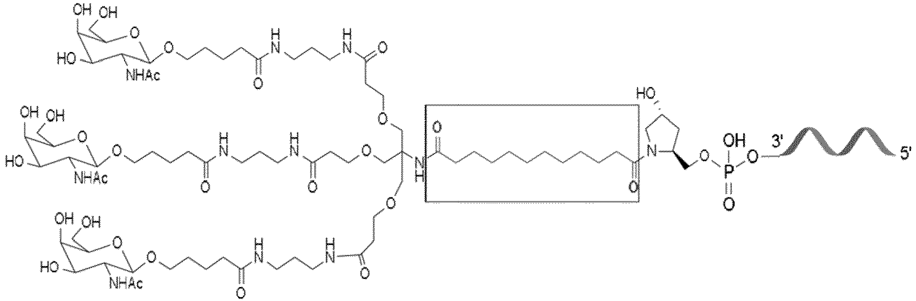

On the other hand, the tethered part L₂ has the following structural formula: 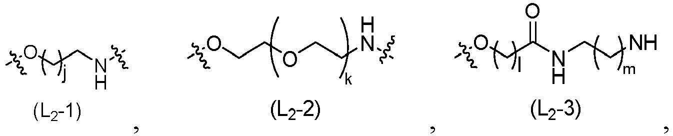 or

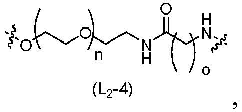

Where j, k, l, m, n, and o are integers from 1 to 20 respectively, preferably integers from 1 to 10.

Specifically, the position of L₂ in the ligand is shown in the following frame structure:

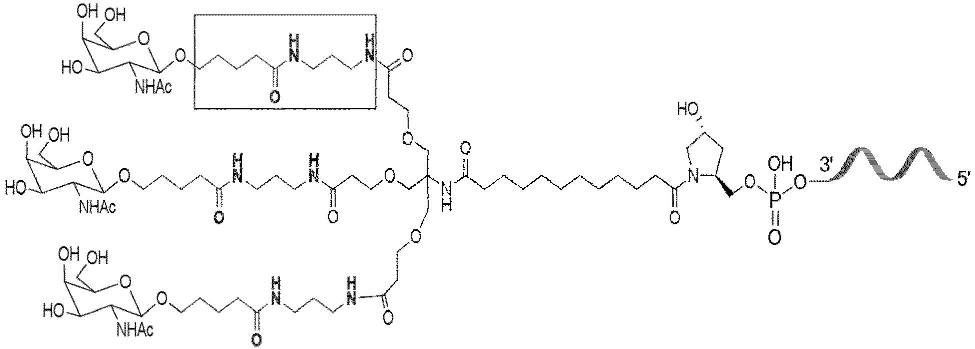

On the other hand, the invention also provides an LPA RNA interference agent, which comprises a sense strand and an antisense strand, and the siRNA conjugate.

Another purpose of the embodiment of the invention is to provide a composition including the RNA interference agent.

The siRNA conjugate provided by the embodiment of the invention can improve the efficiency of targeting part binding to cells or cell receptors and increase the effect of RNA interference.

In a preferred embodiment, the general formula for compound Z in the targeting ligand is as follows (Z-2) :

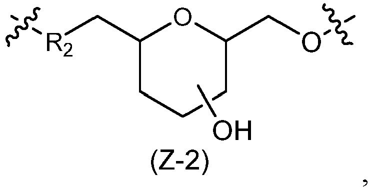

Preferably R₂ is -NH-, where compound Z is:

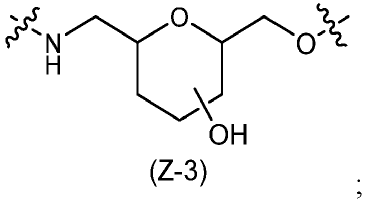

Preferably R₂ is -C (O)-, where compound Z is:

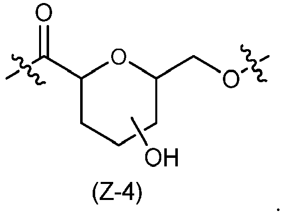

Preferably, L₁ is the following structure: 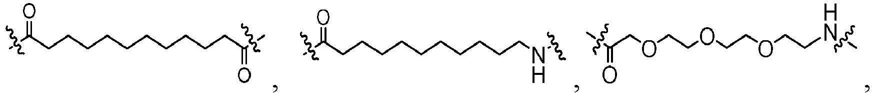 or

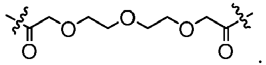

In one embodiment, in general formula (I), the compound E is selected from one of the following five structures:

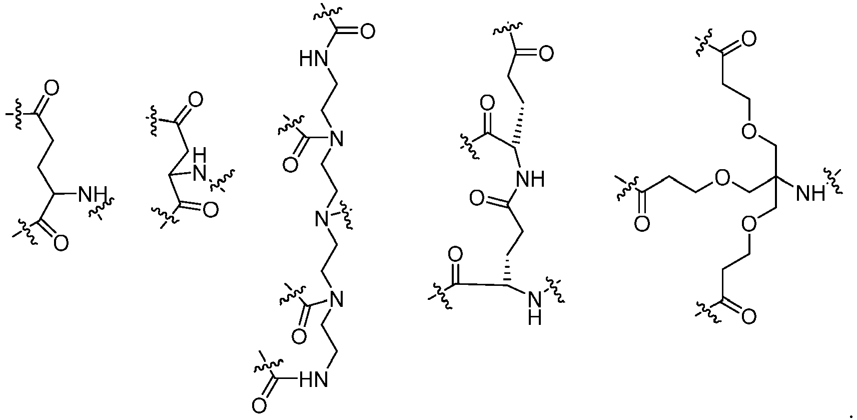

In one embodiment, the compound L₂ in the general formula (I) is 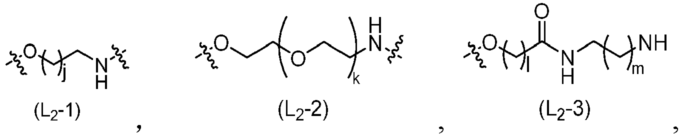 or

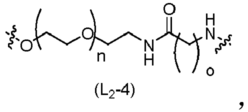

Where j, k, l, m, n, and o are integers from 1 to 20 respectively;

Preferably, L₂ has the following structural formula: 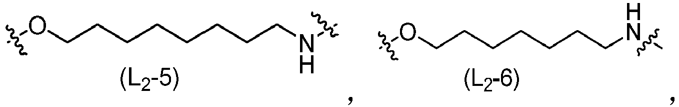 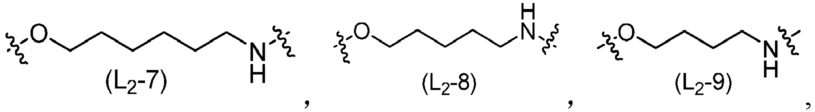 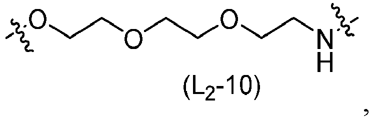 or

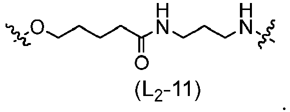

In the preferred embodiment, the targeting ligand described in general formula (I) (excluding the X part) has the following structure:

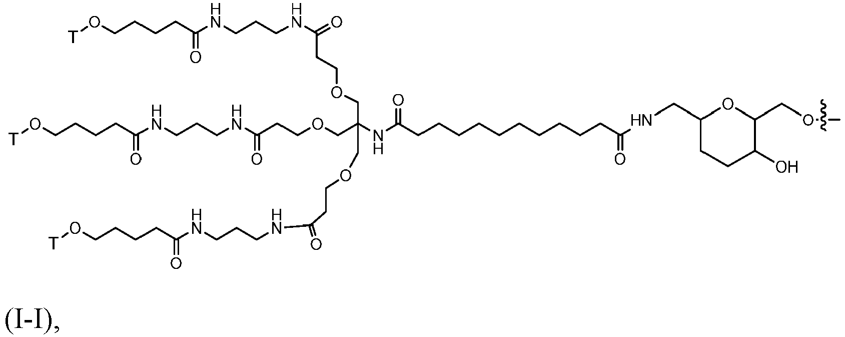

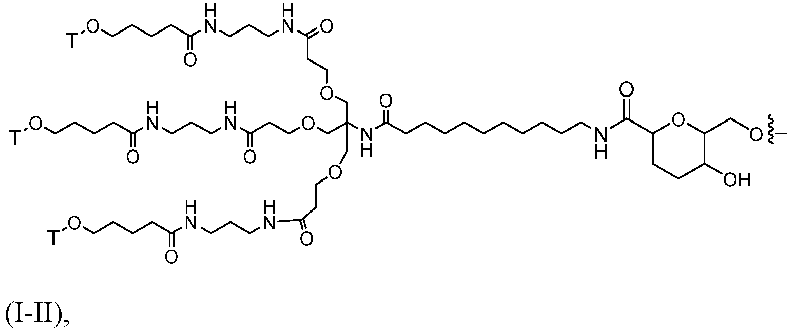

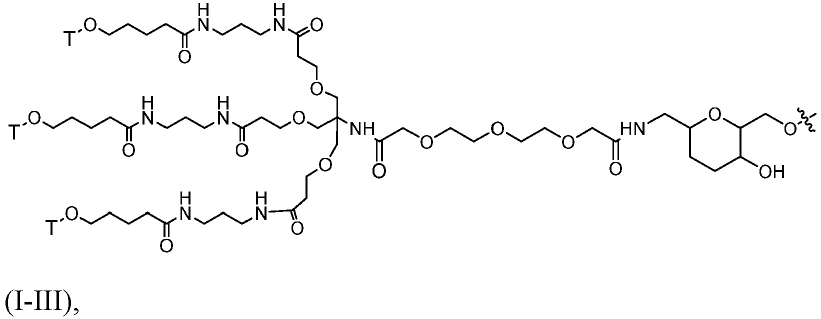

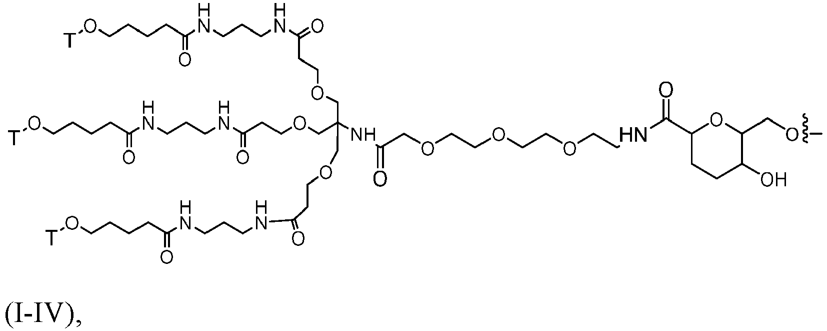

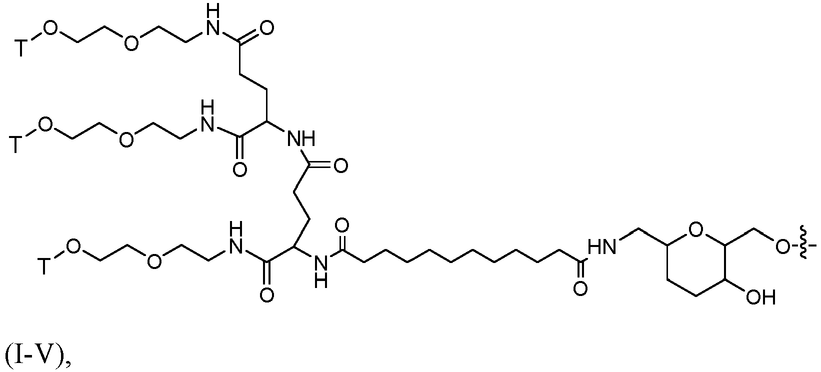

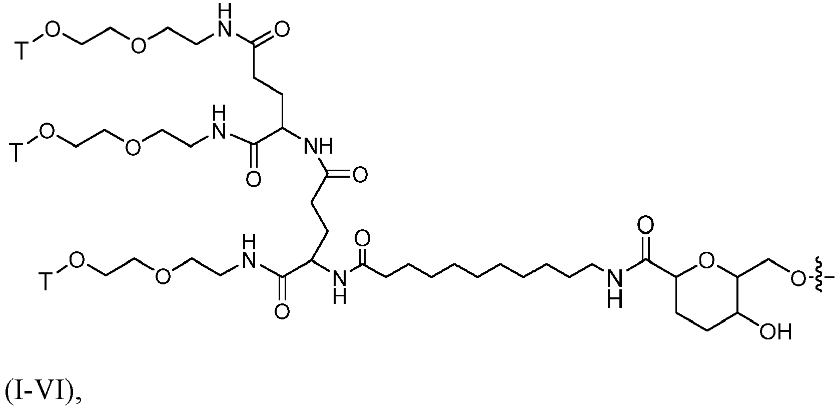

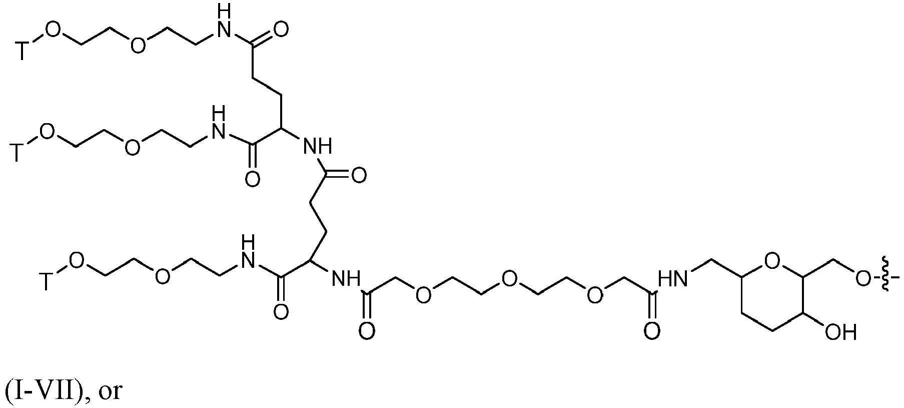

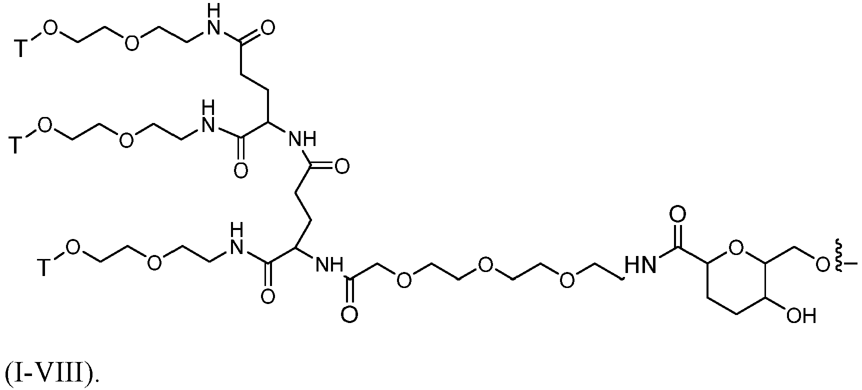

In one embodiment, the T of the targeted ligand is selected from one of N-acetyl-galactosamine, galactose, galactosamine, N-formyl-galactosamine, N-propionyl-galactosamine, N-butyl-galactosamine, and N-isobutyl-galactosamine, preferably N-acetyl-galactosamine, and the structural formula is as follows:

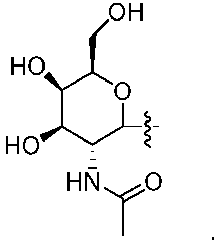

In the preferred embodiment, the targeting ligand in general formula (I) is attached to the end of the siRNA by a phosphate ester group or thiophosphate ester group or phosphonic acid group.

In the preferred embodiment, the targeted ligand has the following structure:

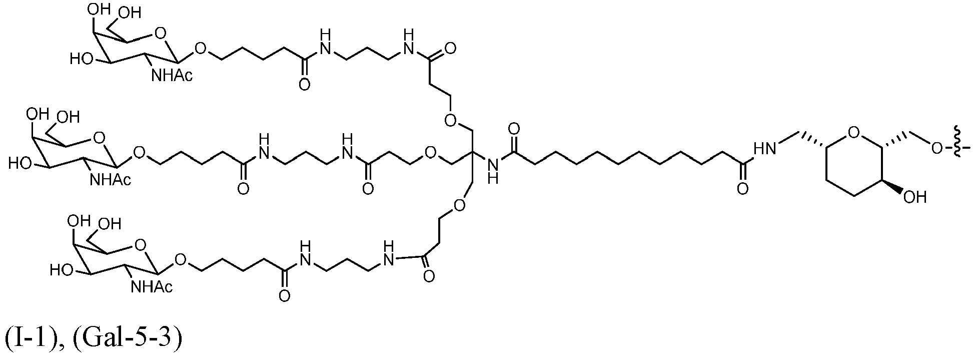

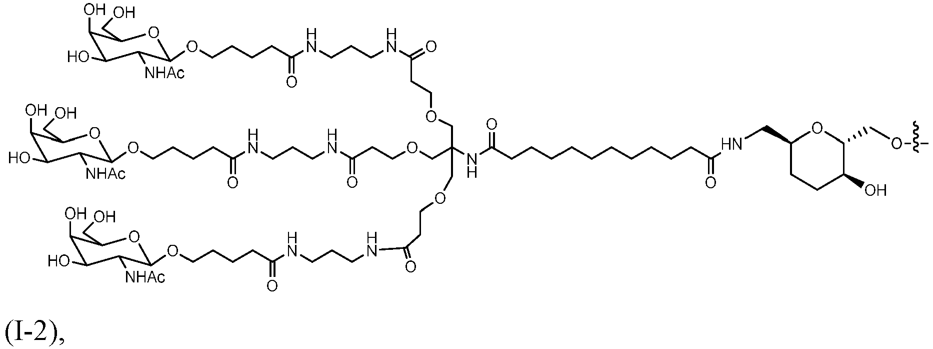

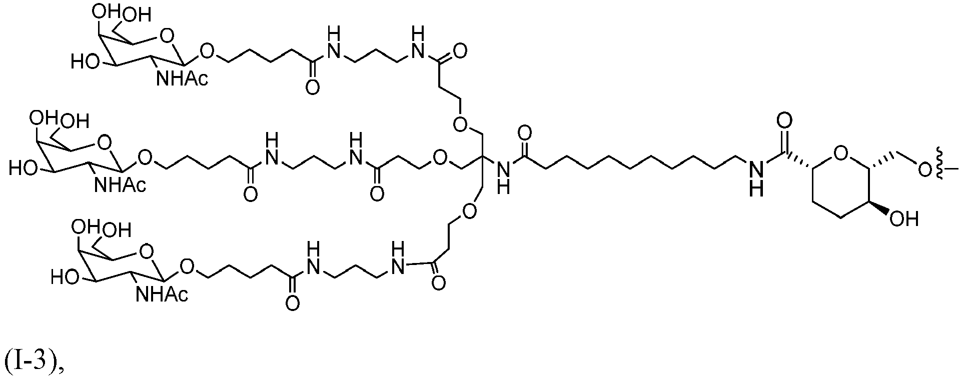

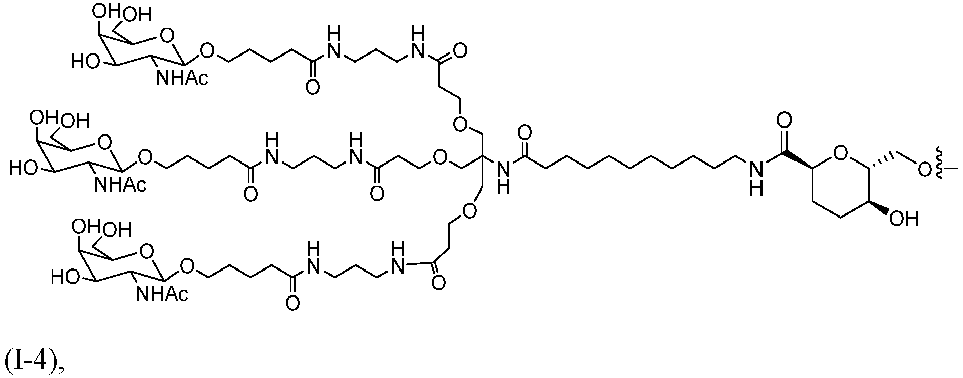

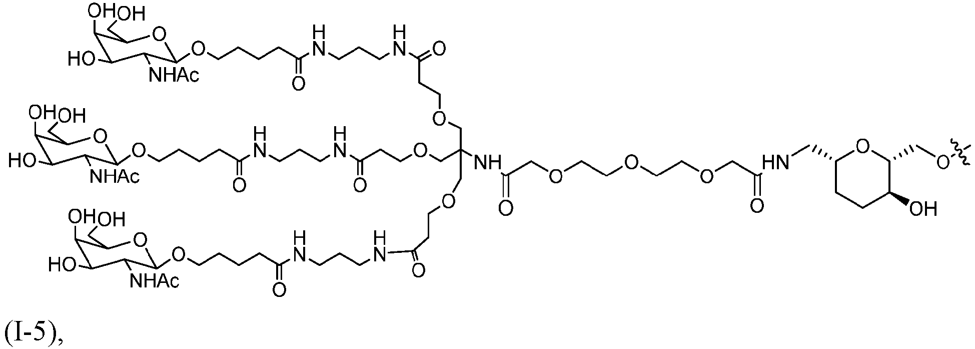

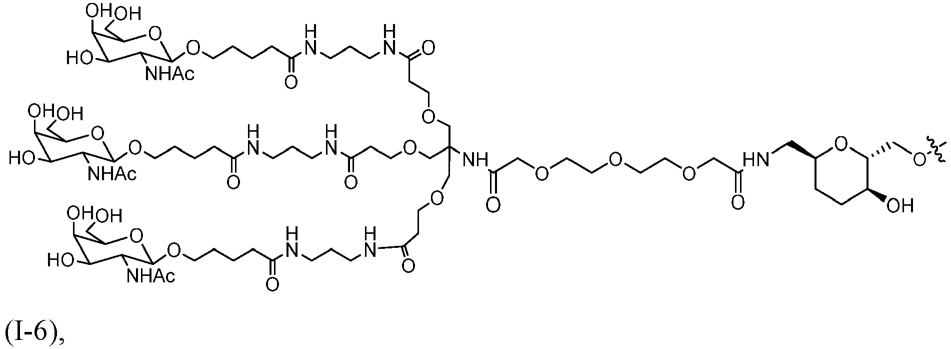

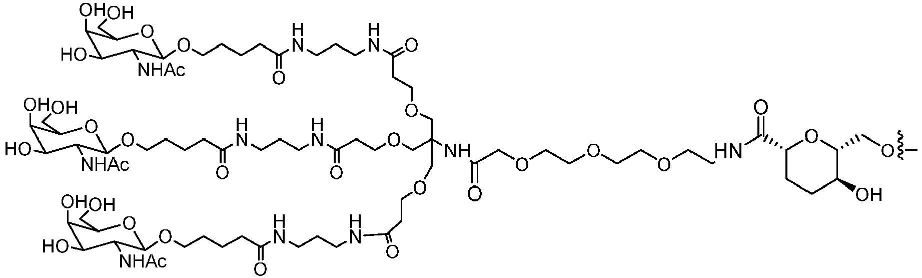

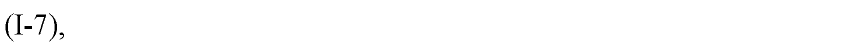

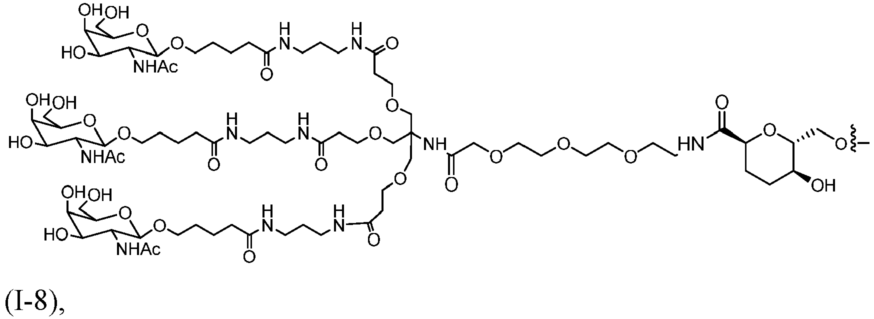

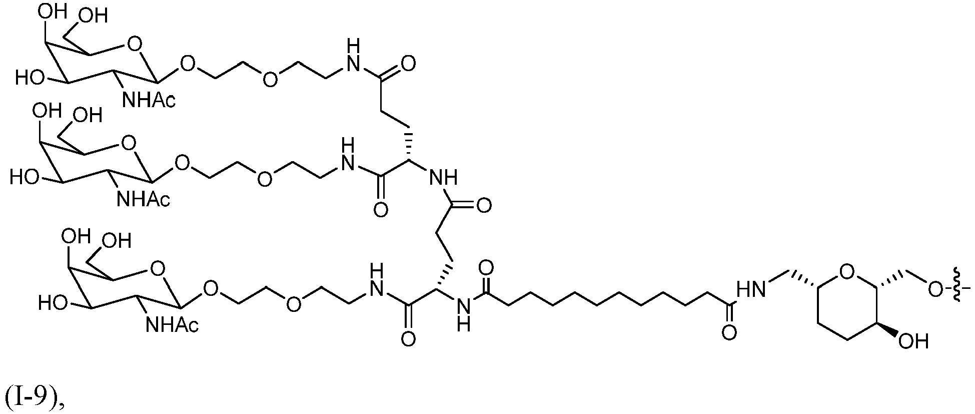

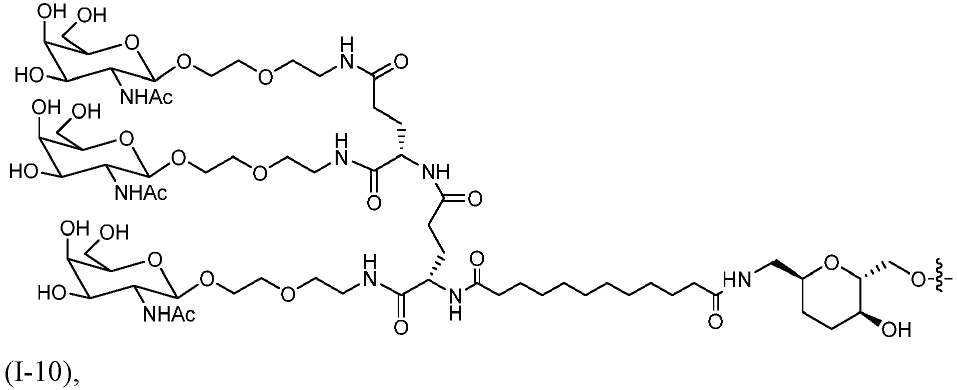

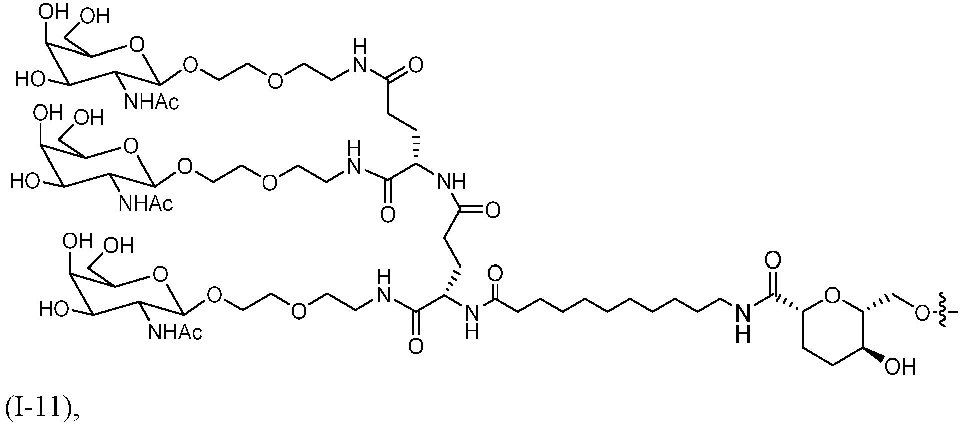

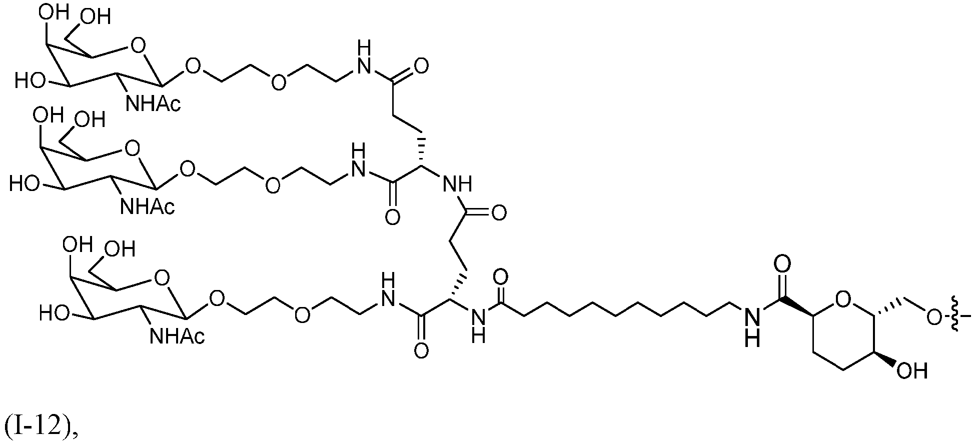

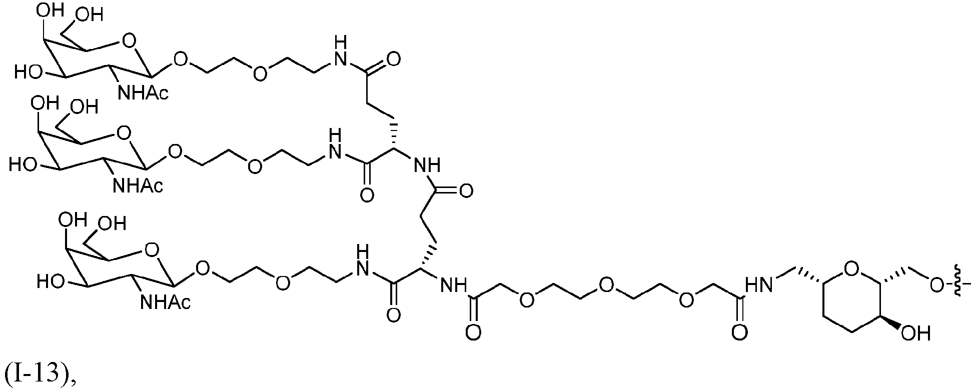

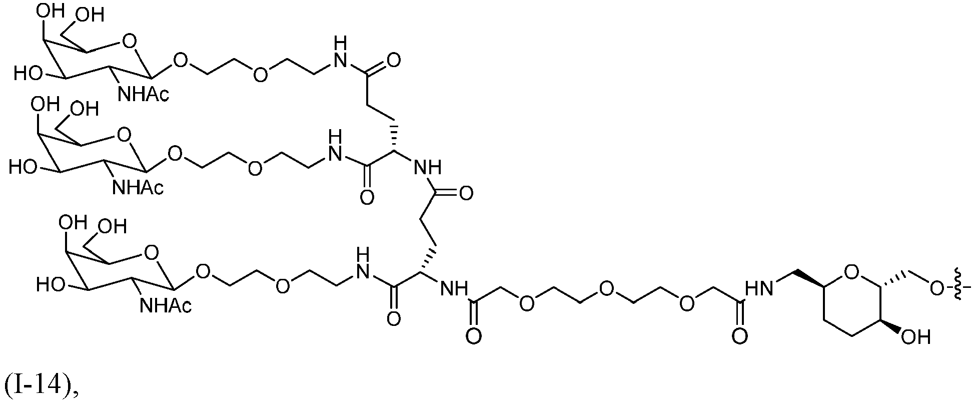

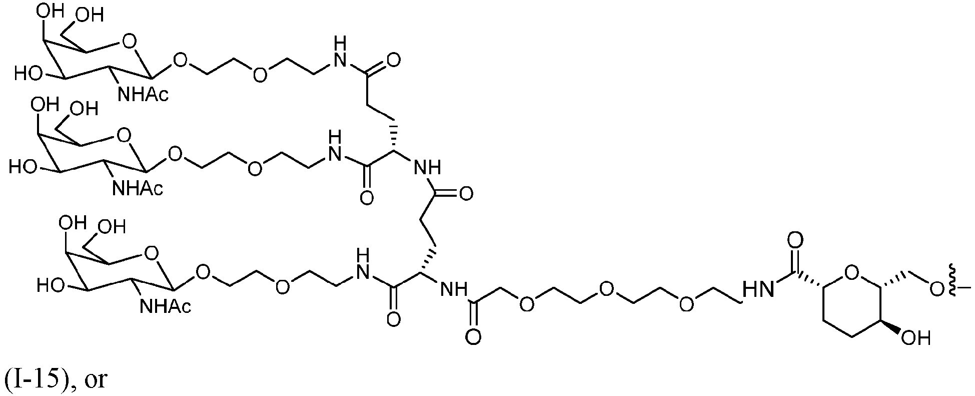

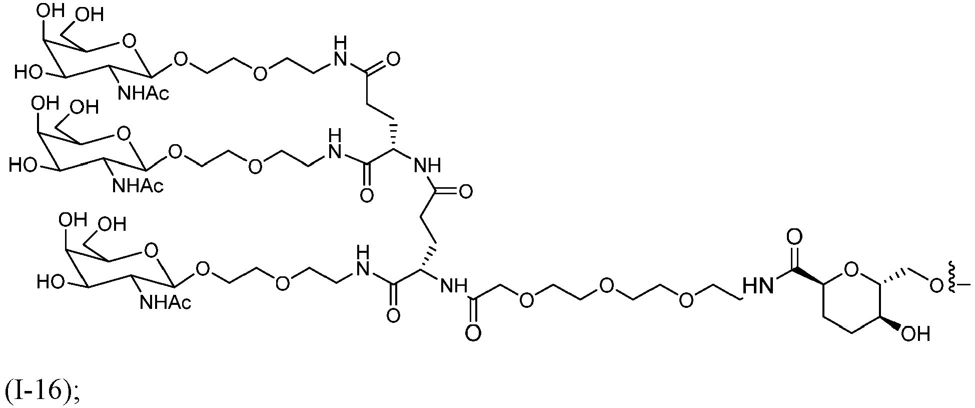

In one embodiment of general formula (II), the H-O-L₃ of the targeting ligand is:

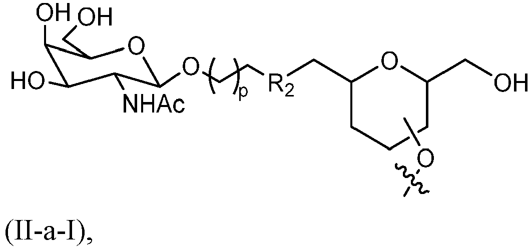

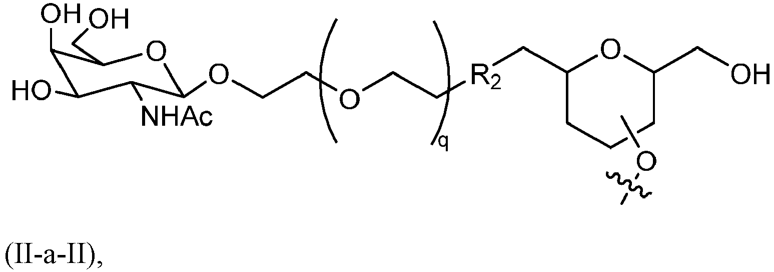

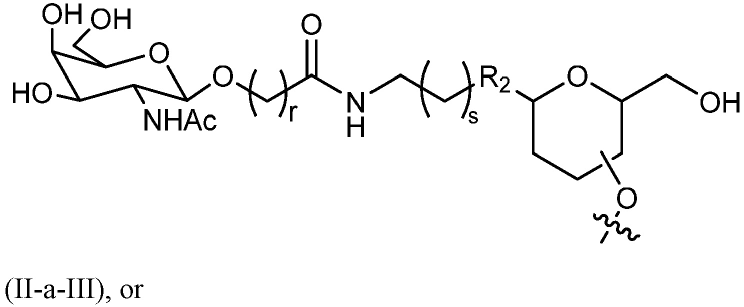

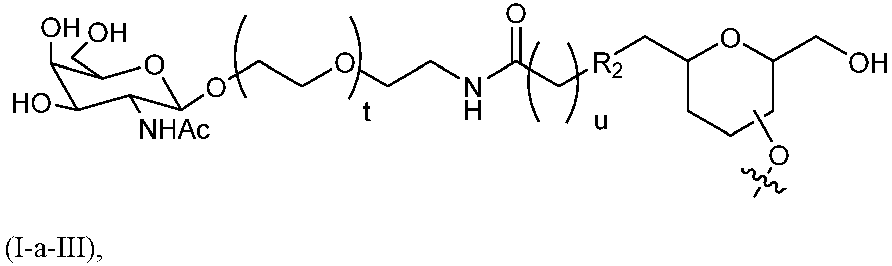

R₂ is independently selected from the group consisting of -O-, -S-, -NH-, -CH₂-, -C(O)-, -OC(O)-, -C(O)O-, -NHC(O)-, -C(O)NH-, - CH₂NH-, -CH₂O-, -NH-C(O)-CH₂-, -C(O)-CH₂-NH-, and -NH(CO)NH-, wherein the -CH₂- may optionally be substituted by a halogen or an alkyl group, where the alkyl group is further substituted by a substituent group selected from hydroxyl group, an amino group, a halogen, an alkoxy group, and an alkylamino group;

The p, q, r, s, t, and u are independently selected from integers of 0 to 20, preferably integers of 1 to 10.

In the preferred embodiment, H-O-L₃ has the following structure: 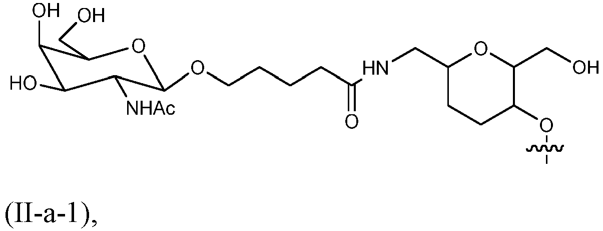 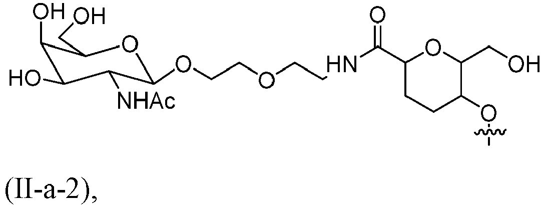 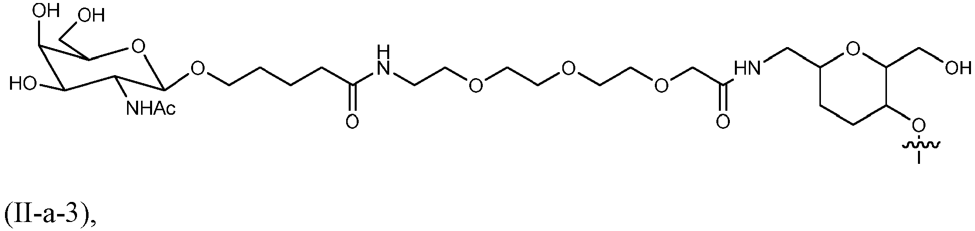 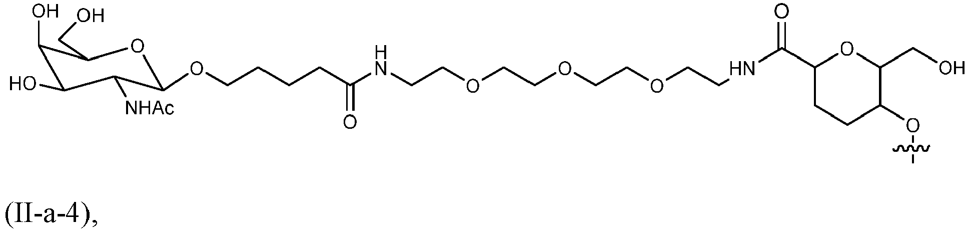 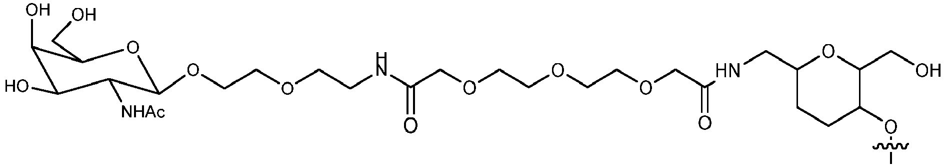 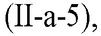 or

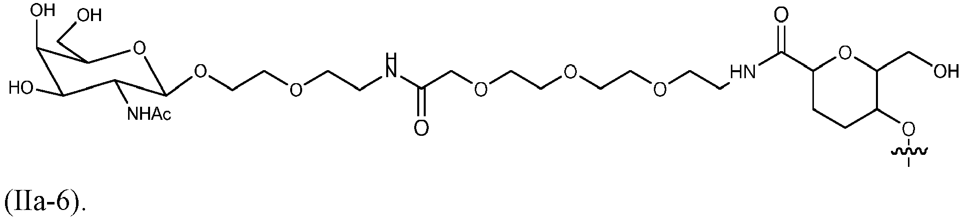

In another preferred embodiment of general formula (II), the H-O-L₄ of the targeting ligand is:

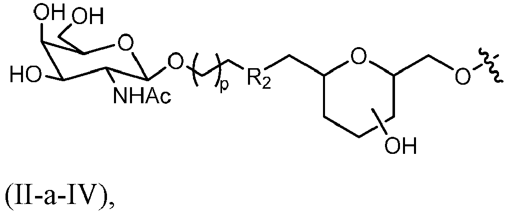

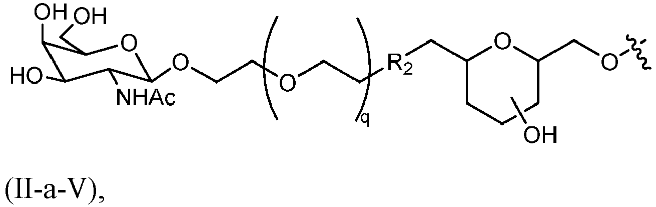

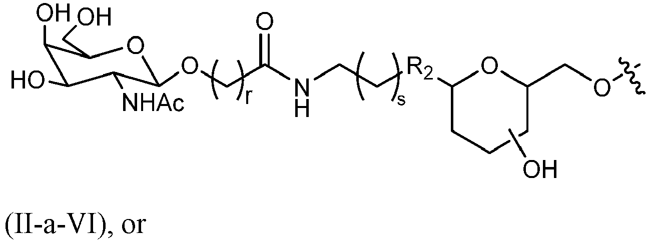

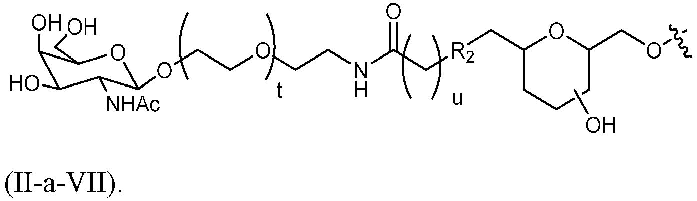

In a preferred embodiment, H-O-L₄ has the following structure:

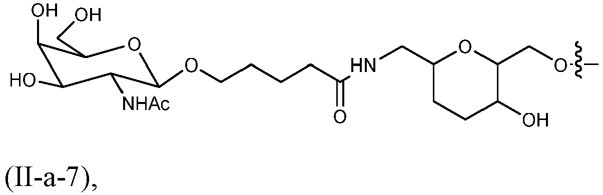

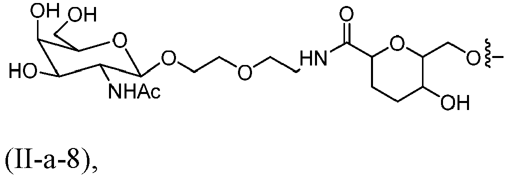

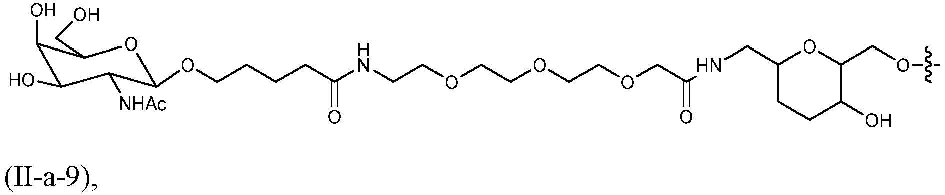

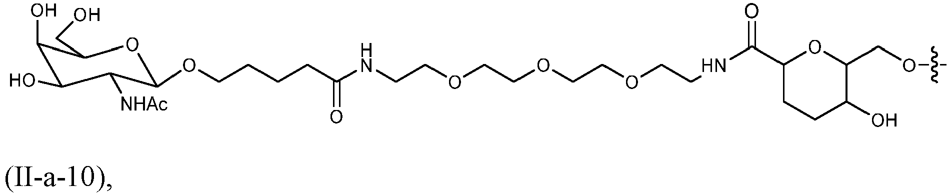

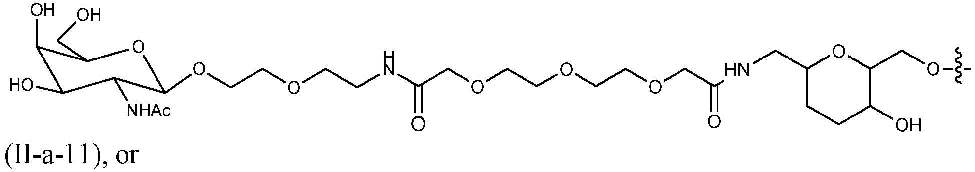

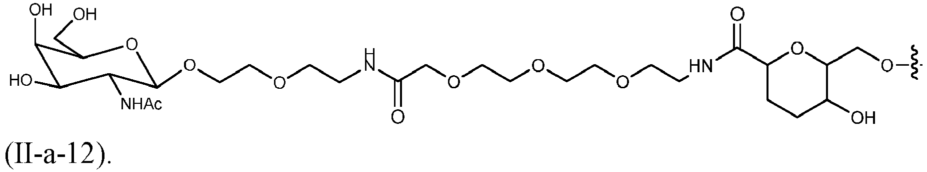

In a preferred embodiment, general formula (II) has the following structure:

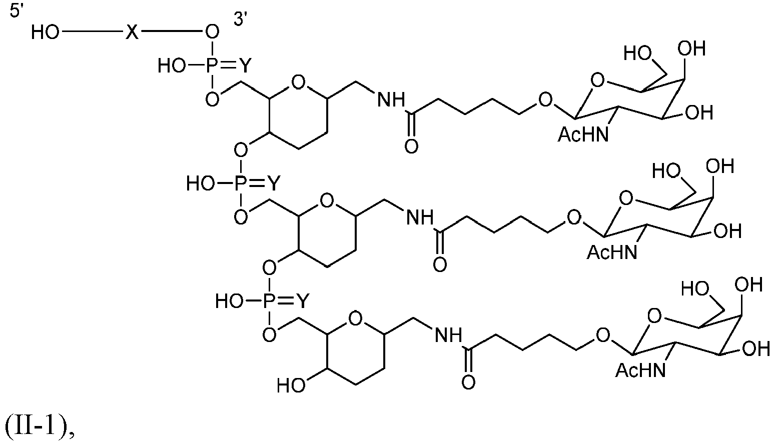

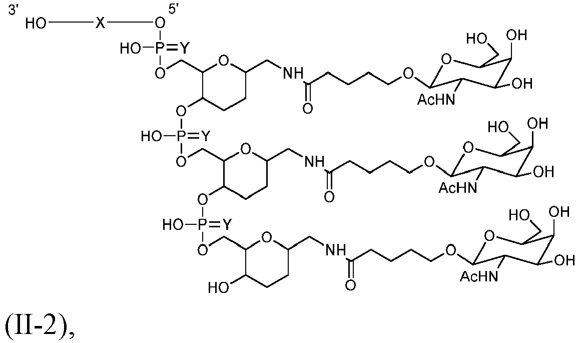

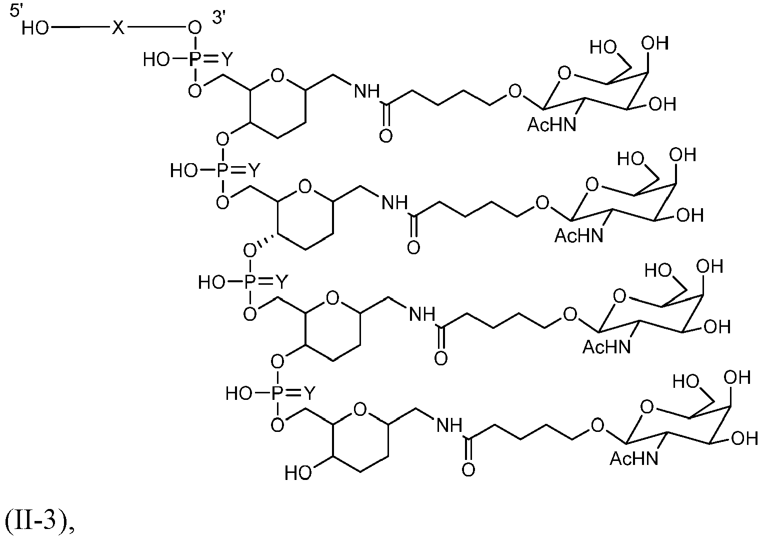

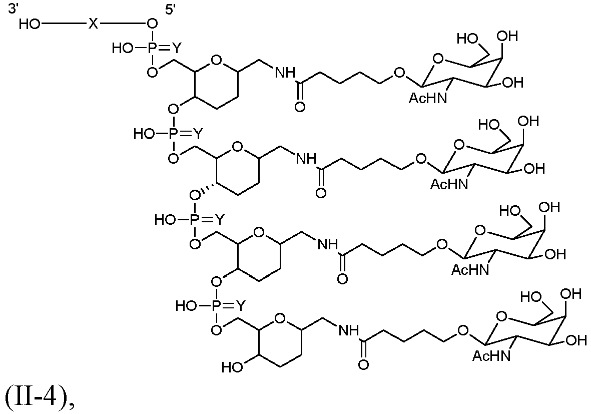

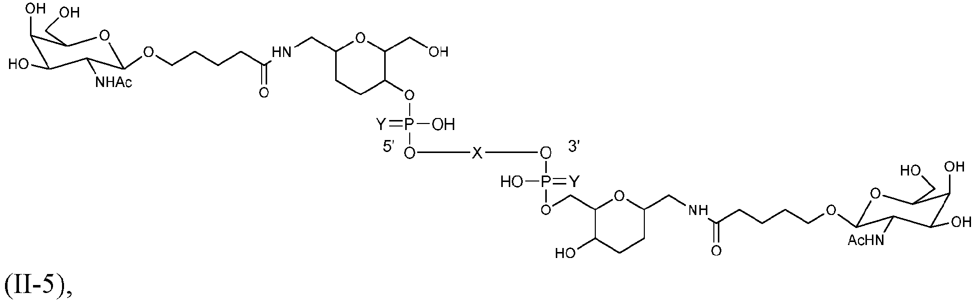

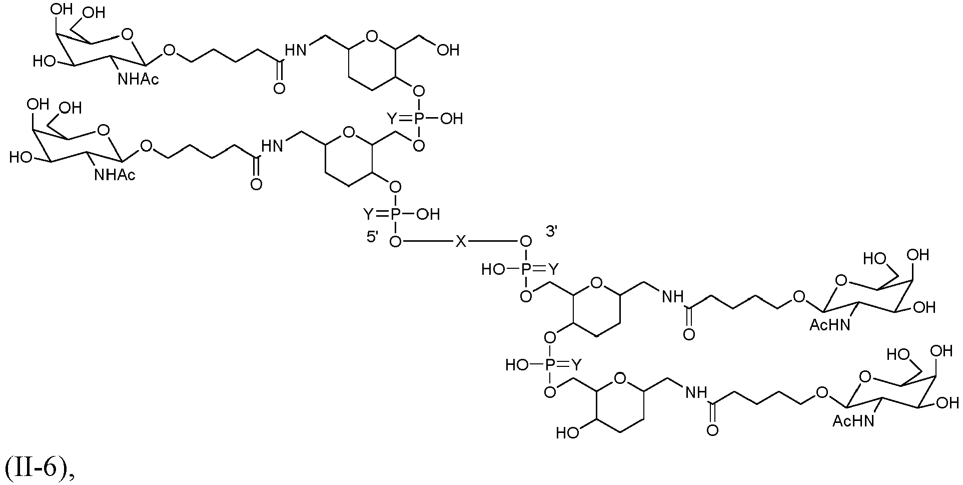

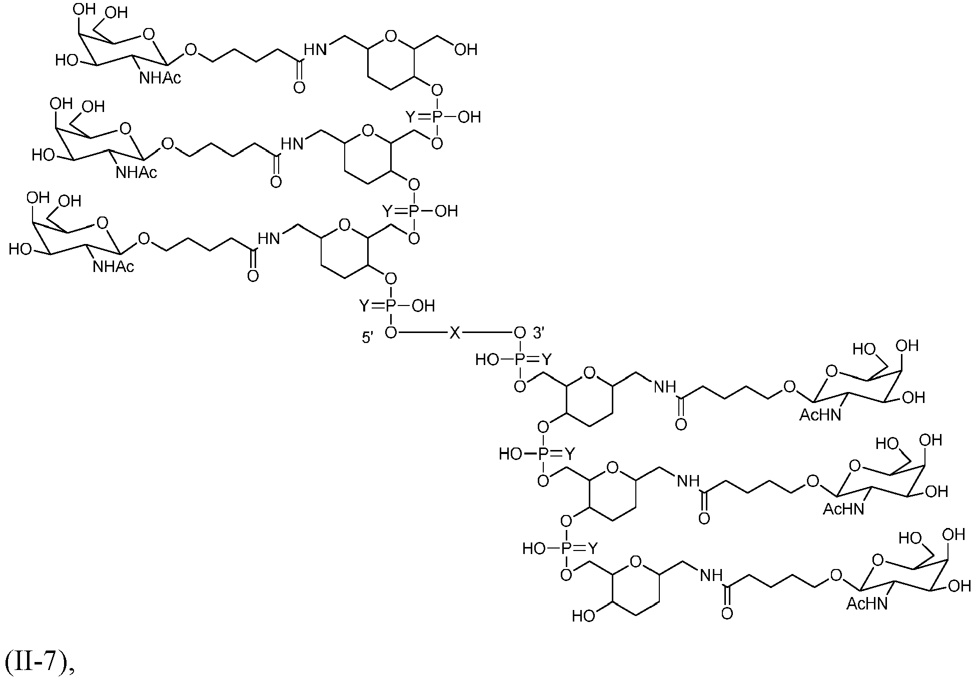

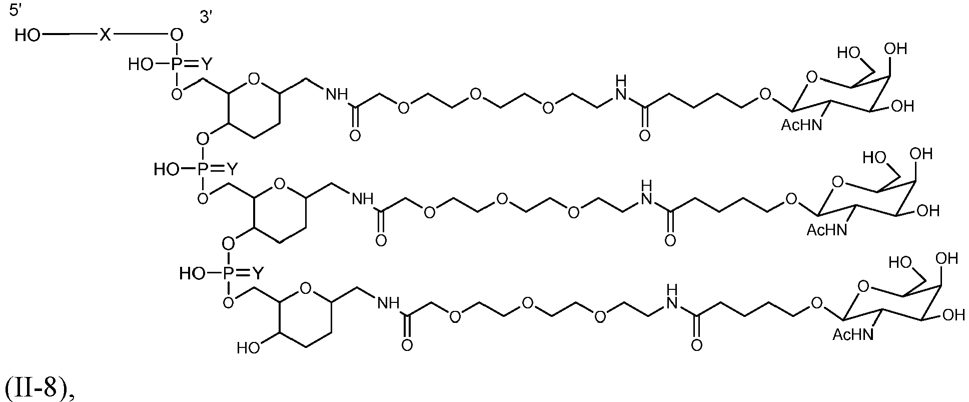

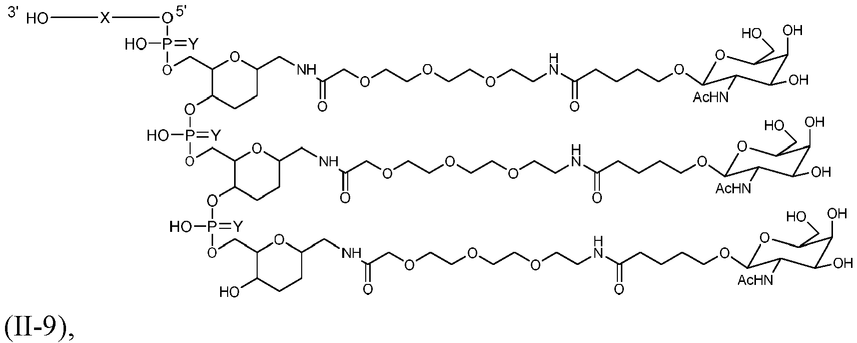

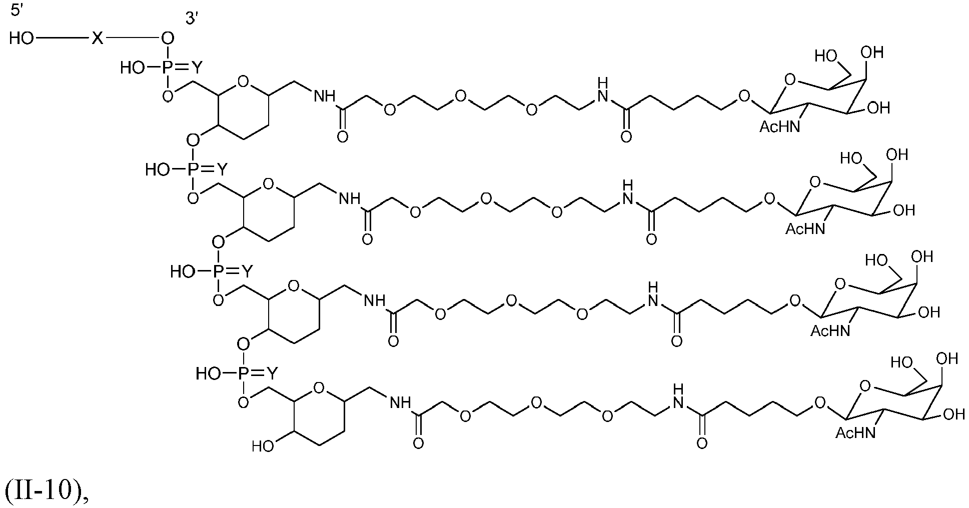

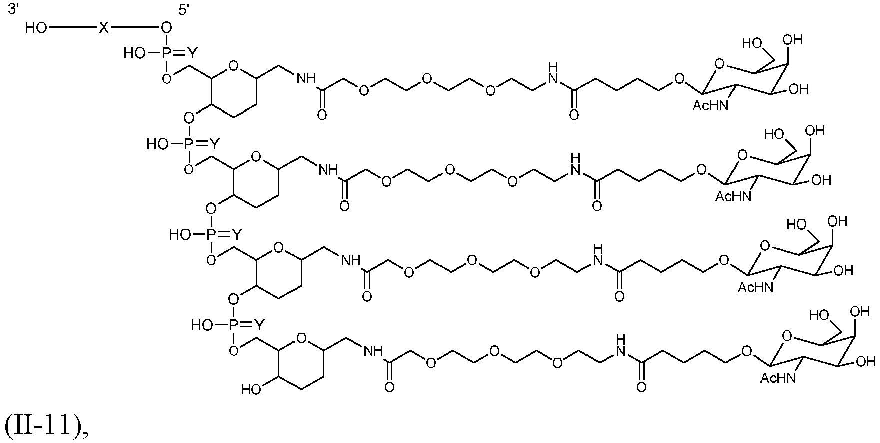

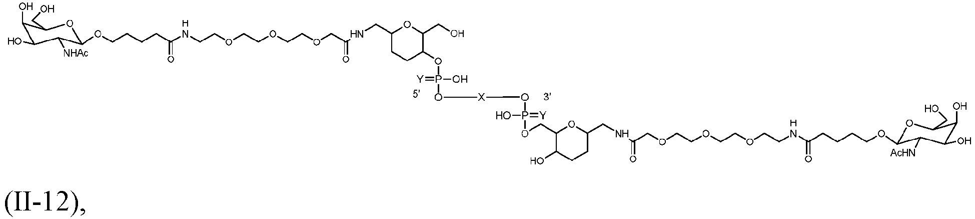

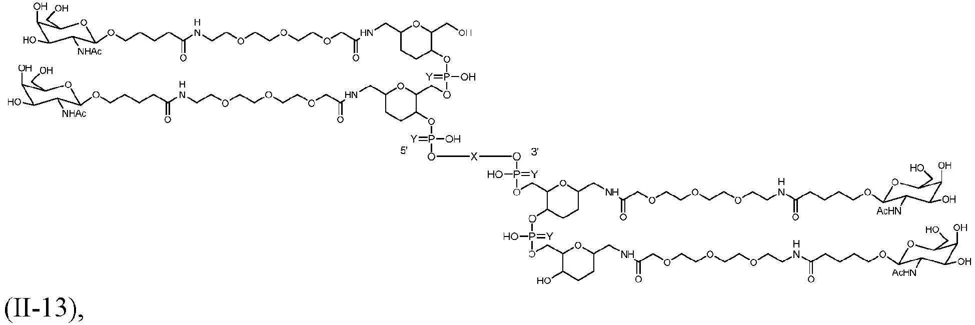

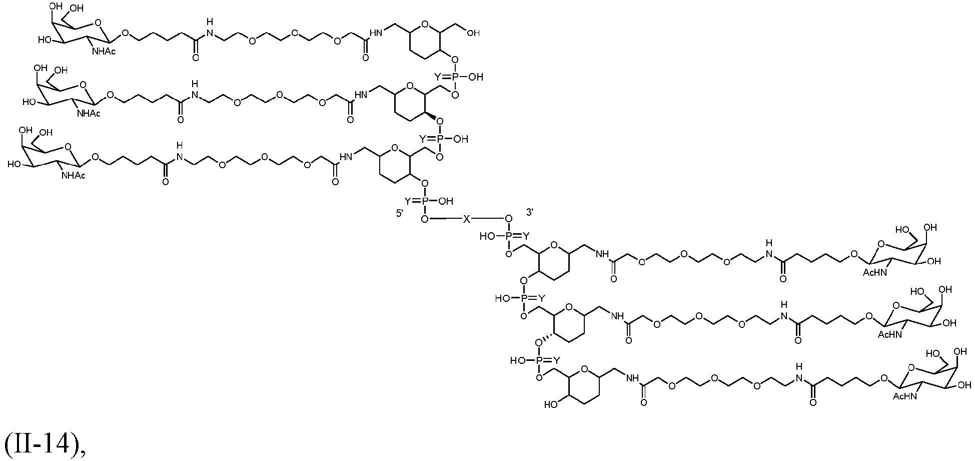

Where Y is O or S.

In another preferred embodiment, the siRNA conjugate has the following structure:

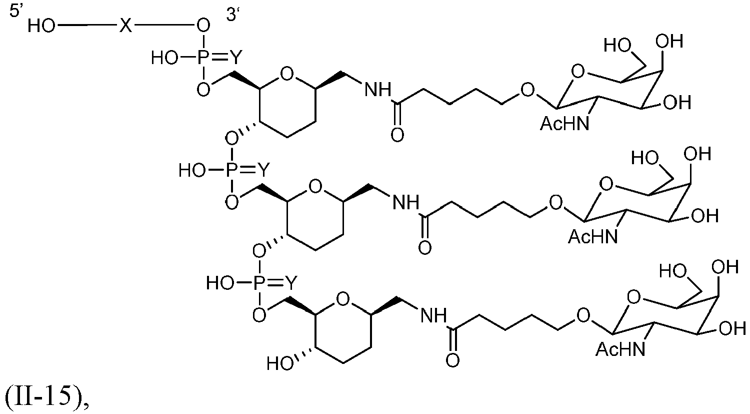

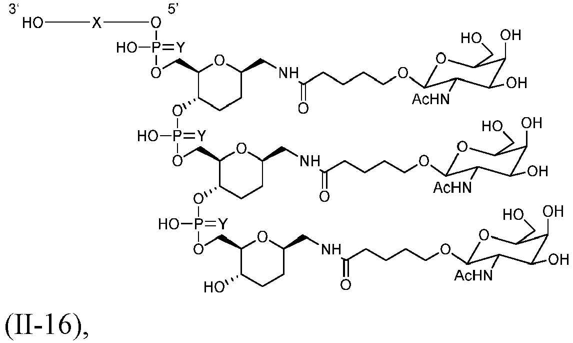

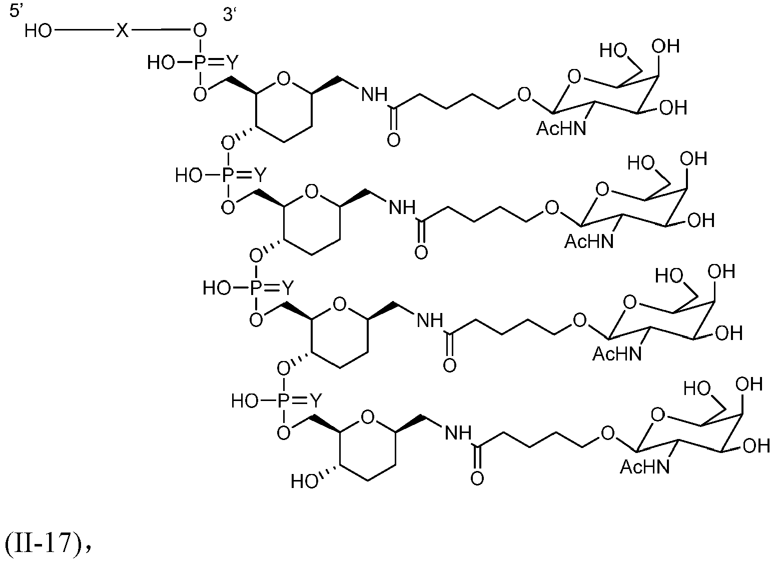

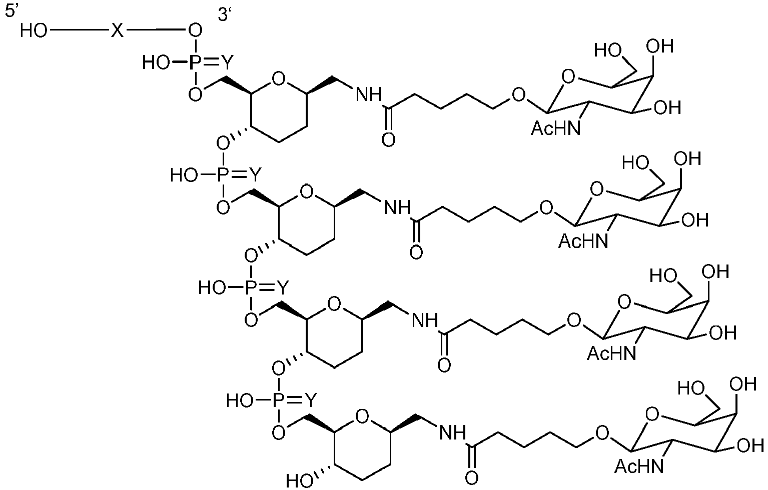

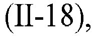

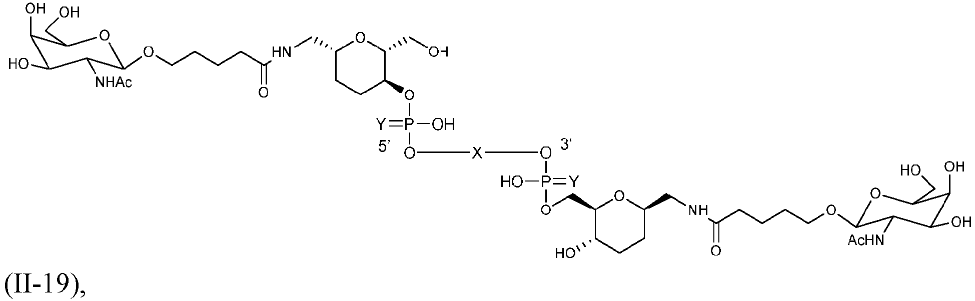

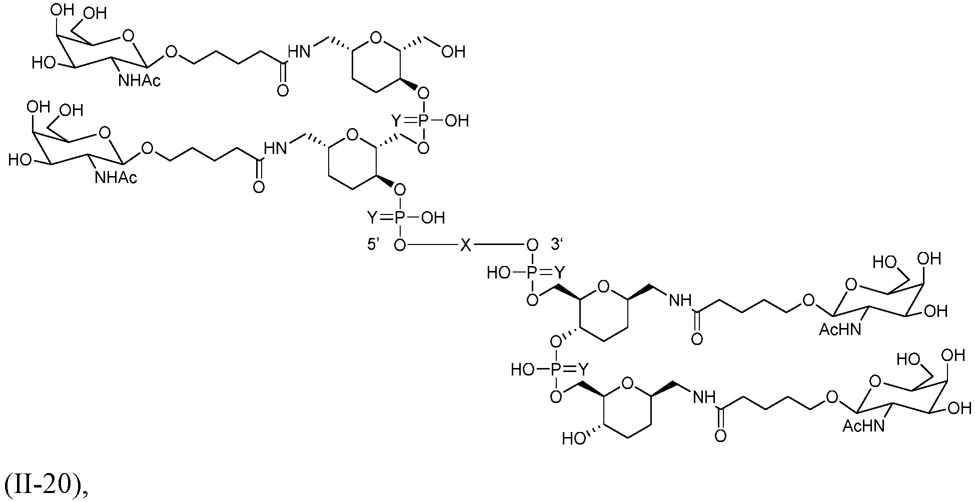

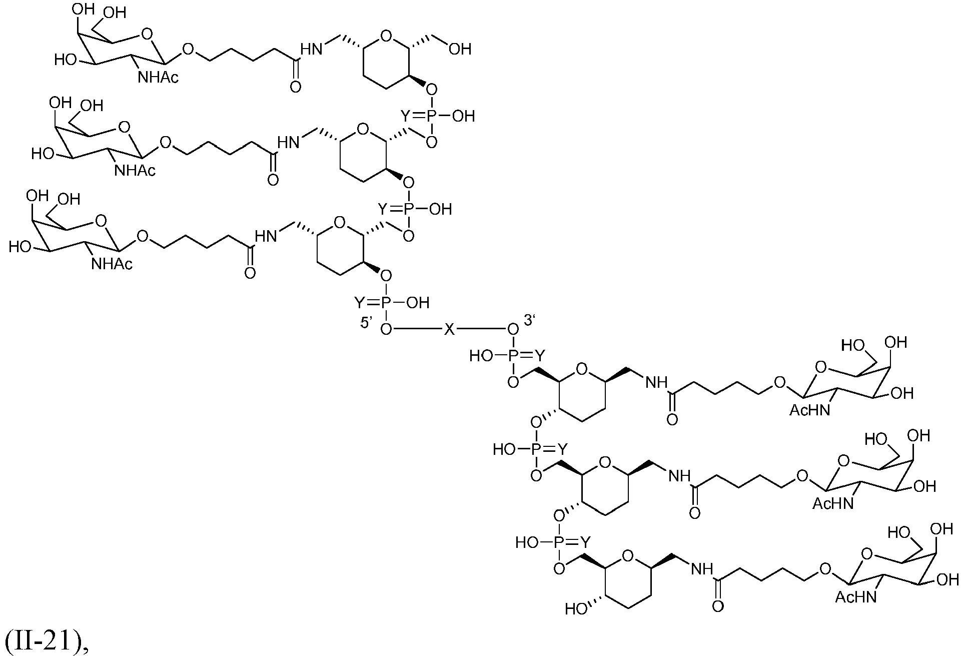

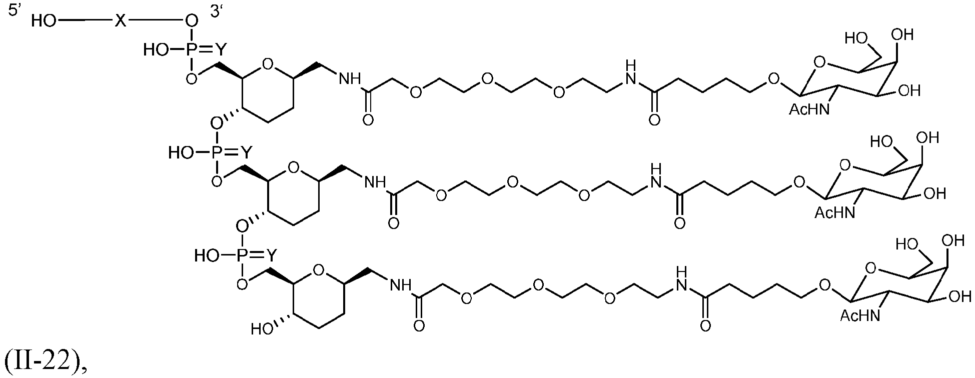

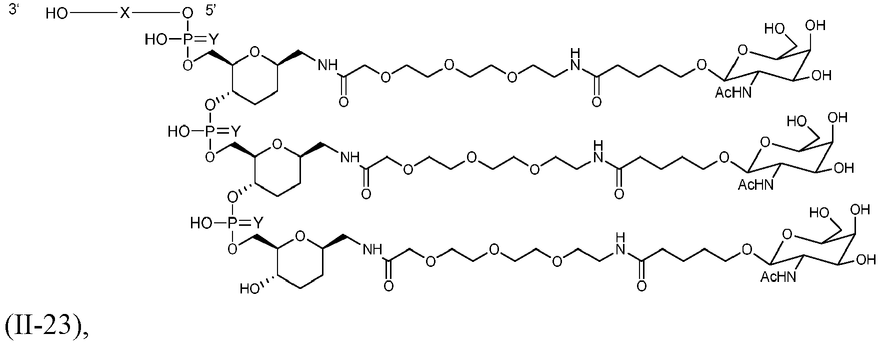

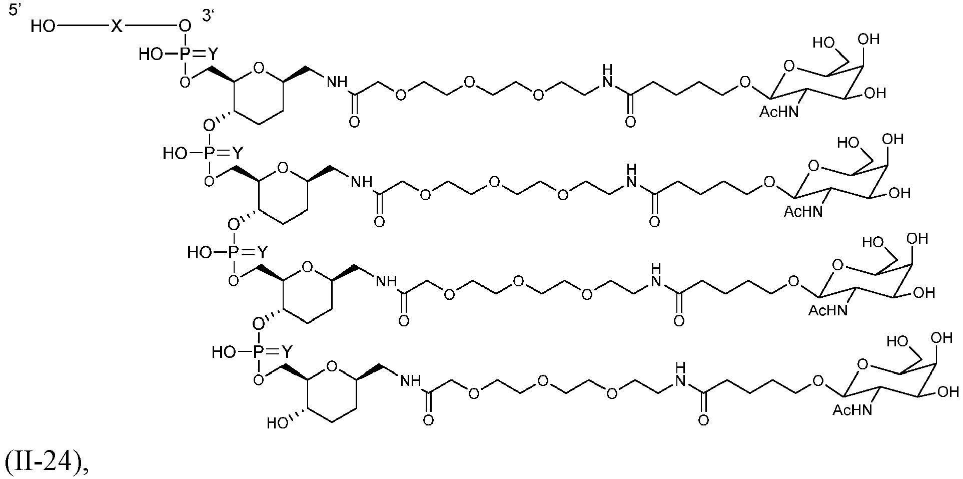

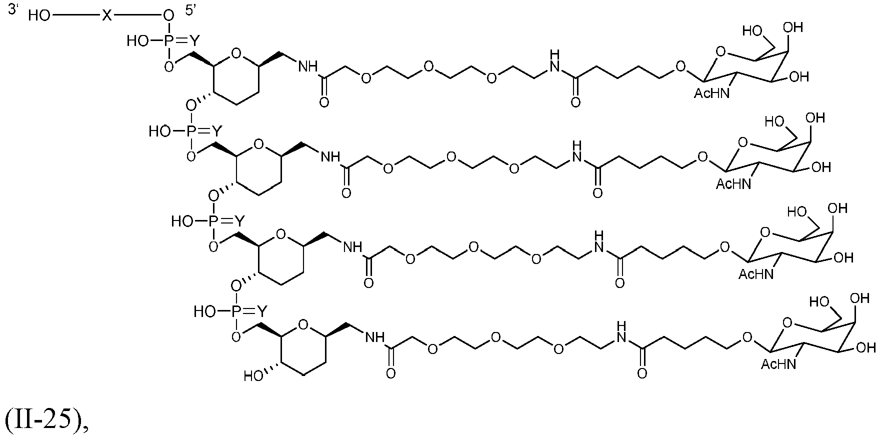

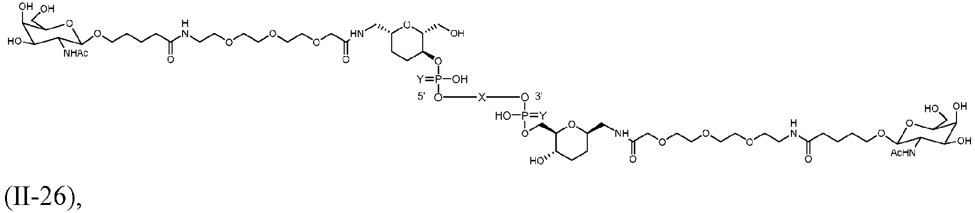

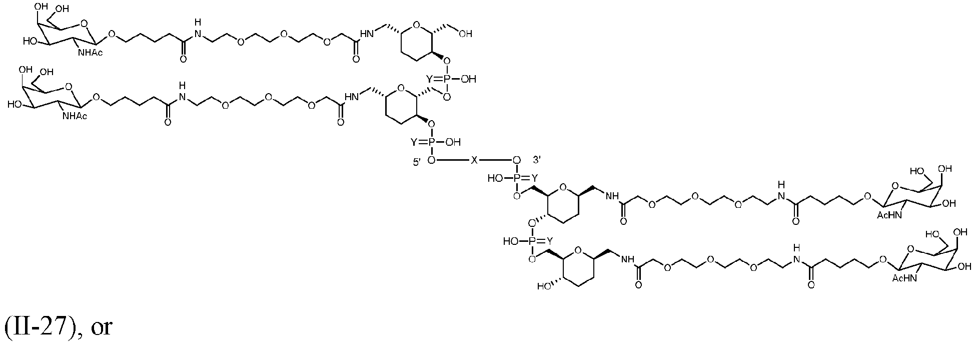

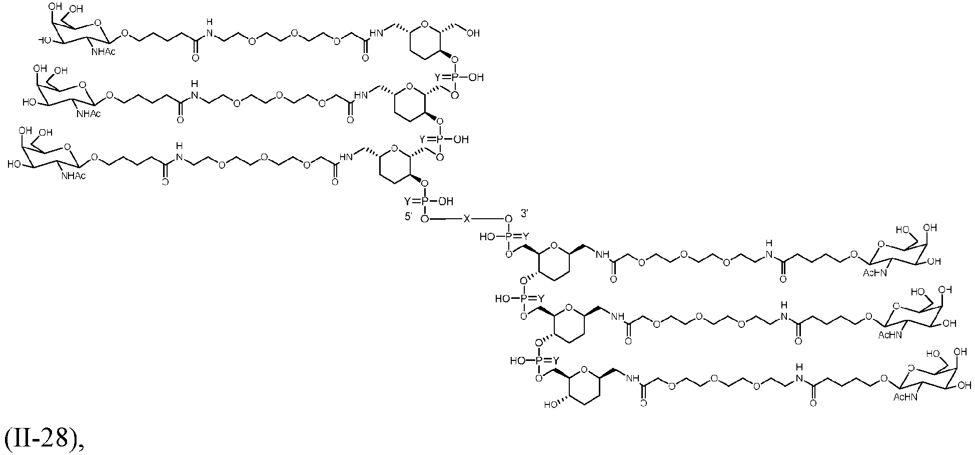

Where Y is O or S.

On the other hand, the above structures bound to the 3' end can all be bound to the 5' end of X.

### Embodiments of pharmaceutical compositions

On the other hand, the present application provides a pharmaceutical composition including the LPA RNAi agent described above and optionally a pharmaceutically acceptable excipient.

In some embodiments, the LPA RNAi agent may be used to inhibit the expression of LPA in cells, cell populations, or tissues of a subject. In some embodiments, the LPA RNAi agent is used to formulate a pharmaceutical composition or medicament for administration to the subject. For example, a pharmaceutical composition or medicament comprises a pharmacologically effective amount of at least one of said LPA RNAi agents and one or more pharmaceutically acceptable excipients. Pharmaceutically acceptable excipients (excipients) are substances other than active pharmaceutical ingredients (API, therapeutic products, such as LPA RNAi agents) that have been appropriately evaluated for safety and are intentionally included in the drug delivery system. Excipients do not exert or are not intended to exert a therapeutic effect at the intendent dosage Excipients may be used to a) assist in the processing of the drug delivery system during manufacture, b) protect, support or enhance the stability, bioavailability or patient acceptability of the API, c) assist in product identification and/or d) enhance any other attributes of the overall safety and effectiveness of delivering API during storage or use. Pharmaceutically acceptable excipients may or may not be inert substances.

The excipients include: absorption enhancer, anti-adherents, defoamer, antioxidants, adhesive, binder, buffer, carrier, coating agent, pigment, delivery accelerator, delivery polymer, dextran, dextrose, diluents, disintegrants, emulsifiers, extenders, fillers, flavors, glidants, humectants r, lubricants, oils, polymers, preservatives, saline salts, solvents, sugars, suspending agents, sustained release matrices, sweeteners, thickeners, tonicity angents, vehicles, hydrophobic agents and wetting agents.

Pharmaceutical compositions may contain other additional components commonly found in pharmaceutical compositions. Such additional components include, but are not limited to: antipruritic, astringents, local anesthetics or anti-inflammatory agents (such as antihistamines, diphenhydramine, etc.). It is also envisioned that cells, tissues or isolated organs expressing or containing RNAi agents as defined in the present application may be used as "pharmaceutical compositions".

In some embodiments, the LPA RNAi agents is combined with one or more additional therapeutic agents or treatments including, but not limited to, small molecule drugs, antibodies, antibody fragments, and/or vaccines. The RNAi agents and pharmaceutical compositions containing the LPA RNAi agents disclosed in the present application may be packaged or included in a kit, container, pack, or dispenser. The LPA RNAi agents and a pharmaceutical composition containing the LPA RNAi agents may be packaged in pre-filled syringes or vials.

The pharmaceutical compositions in the present application can be used to inhibit the expression of LPA gene in cells, tissues or organisms. In some embodiments, the pharmaceutical composition is used to treat subjects having a disease, condition, or disorder that would benefit from reducing or inhibiting of the expression of LPA. In some embodiments, the pharmaceutical compositions is used to treat subjects who are at risk of developing diseases, disorders, or conditions that would benefit from reducing or inhibiting of the expression of LPA. Diseases, disorders, or conditions that will benefit from reducing or inhibiting of the expression of LPA include, but are not limited to: Berger's disease, peripheral artery disease, coronary artery disease, metabolic syndrome, acute coronary syndrome, aortic valve stenosis, aortic valve regurgitation, aortic dissection, retinal artery obstruction, cerebrovascular disease, mesenteric ischemia, superior mesenteric artery obstruction, renal artery stenosis, stable/unstable angina, acute coronary syndrome, heterozygous or homozygous familial hypercholesterolemia, hyperapolipoprotein β lipoproteinemia, cerebrovascular atherosclerosis, cerebrovascular disease and venous thrombosis. In some embodiments, the subjects are mammals, including but not limited to humans.

Considering cells, tissues, and non-human organisms containing at least one of LPA RNAi agents described in present application. The cells, tissues or non-human organisms are made by delivering the LPA RNAi agents to those cells, tissues or non-human organisms by any means available in the field. In some embodiments, the cells are mammalian cells, including but not limited to human cells. Cells, tissues, or non-human organisms may be used in research or as research tools (such as drug testing or diagnostics).

### Use

On the other hand, the present application provides the use of the said LPA RNAi agents or the said pharmaceutical compositions in the preparation of a drug, the drug is intended to prevent and/or treat a disease or condition, or to reduce the risk of a disease or condition.

In some embodiments, the disease or condition includes a disease or condition associated with elevated levels of Lp(a) s. In the present application, the term "Lp(a)" generally refers to LDL-like particles containing apo(a) and containing apoB. apo(a) is connected to apoB by a disulfide bond.

In some embodiments, the disease or condition includes diseases of liver origin.

In some embodiments, the disease or condition includes inflammatory, cardiovascular or metabolic diseases.

In the present application, the term "metabolic disease or condition" generally refers to a condition characterized by an alteration or disorder of metabolic function. "Metabolic" and "metabolism" are well-known terms in the field and usually include the entire range of biochemical processes that occur in living organisms. Metabolic conditions include, but are not limited to, hyperglycemia, prediabetes, diabetes (type I and type II), obesity, insulin resistance, metabolic syndrome, and dyslipidemia due to type II diabetes.

In the present application, the term "inflammatory disease or condition" means a disease, condition, syndrome or other condition that causes inflammation. For example, rheumatoid arthritis and liver fibrosis are inflammatory conditions. Other examples of inflammatory conditions include sepsis, myocardial ischemia/reperfusion injury, adult respiratory distress syndrome, nephritis, transplant rejection, inflammatory bowel disease, multiple sclerosis, arteriosclerosis, atherosclerosis, and vasculitis.

In the present application, the term "symptoms of cardiovascular or cerebrovascular disease or condition" generally refers to the phenomena caused by and accompanied by cardiovascular or cerebrovascular disease or condition, and serves as an indication of cardiovascular or cerebrovascular disease or condition.

On the other hand, the present application provides a method for the prevention and/or treatment of a disease or condition, the method includes the administration to a target subject of an effective amount of the pharmaceutical compositions containing the LPA RNAi agents described above and/or the pharmaceutical compositions described above.

In some embodiments, LPA RNAi agents described in the present application can be used to treat diseases or conditions that benefit from reducing of the expression of LPA. Examples of the diseases or conditions include, but are not limited to: Berger's disease, peripheral artery disease, coronary artery disease, metabolic syndrome, acute coronary syndrome, aortic valve stenosis, aortic valve regurgitation, aortic dissection, retinal artery obstruction, cerebrovascular disease, mesenteric ischemia, superior mesenteric artery obstruction, renal artery stenosis, stable/unstable angina pectoris, acute coronary syndrome, heterozygous or homozygous familial hypercholesterolemia, hyperapolipoprotein β lipoproteinemia, cerebrovascular atherosclerosis, cerebrovascular disease and venous thrombosis. In some embodiments, the method includes the administration of a composition, such as a pharmaceutical composition containing the LPA RNAi agent described in the present application, to a target mammal.

In some embodiments, a therapeutically effective amount of one or more of the LPA RNAi agents is administered to the subject, thereby inhibiting the expression of LPA in the subject (e.g., an amount that effectively inhibits the expression of LPA in the subject).

In some embodiments, the method also includes a step of administering a second therapeutic agent or treatment. In some embodiments, the second therapeutic agent is another LPA RNAi agent (e.g., an LPA RNAi agent that targets a different sequence within the LPA target). In other embodiments, the second therapeutic agent may be selected from: small molecule drugs, antibodies, antibody fragments, and vaccines.

The route of administration is the route by which the RNAi agents are brought into contact with the body. In general, the methods of administration of drugs and nucleic acids used to treat subjects are well known in the field and can be applied to administration of the compositions described in the present application. The compounds described in the present application can be administered by any suitable route in formulations appropriately tailored to a particular route. Therefore, the compounds described in the present application can be administered by injection, for example intravenously, intramuscularly, intracutaneously, subcutaneously or intraperitoneally.

In some embodiments, LPA RNAi agents or compositions described in the present application can be delivered to cells, cell populations, tissues, or subjects using oligonucleotide delivery techniques known in the field. Generally, any suitable method recognized in the field for delivering nucleic acid molecules (in vitro or in vivo) may be adapted to LPA RNAi agents described in the present application. For example, delivery can be by local administration (e.g., direct injection, implantation or topical administering), systemic administration, or subcutaneous, intravenous, oral, intraperitoneal or parenteral routes, including intracranial (e.g. intraventricular, intra-parenchymal, and intrathecal), intramuscular, transdermal, airway (aerosol), nasal, rectal, or topical (including buccal and sublingual) administration. In some embodiments, the compositions are administered by subcutaneous or intravenous infusion or injection.

In some embodiments, LPA RNAi agents can be combined with lipids, nanoparticles, polymers, liposomes, micelles, or other delivery systems available in the field. RNAi agents can also be chemically conjugated to targeted groups, lipids (including but not limited to cholesterol and cholesteryl derivatives), nanoparticles, polymers, liposomes, micelles, or other delivery systems available in the field. The LPA RNAi agents can be conjugated to the delivery polymer. In some embodiments, the delivery polymer is a reversible masked/modified amphiphilic membrane-active polyamine.

### Inhibition of expression

On the other hand, the present application provides a method for reducing the expression of LPA mRNA or protein in cells or tissues, which includes exposing cells or tissues to an effective amount of the LPA RNAi agents described above or the pharmaceutical compositions described above.

In some embodiments, the cells are liver cells. In some embodiments, the tissue is liver tissue.

In some embodiments, the cells and tissues are in vitro. In other embodiments, the cells and tissues are in the body.

On the other hand, the present application provides a method for reducing the expression of LPA mRNA or protein in subjects, which includes administering an effective amount of the LPA RNAi agents described above or the pharmaceutical compositions described above to the target subjects.

As used in the present application, the terms "silence", "reduce", "inhibit", "down-regulate", or "knockdown gene expression" when referring to an LPA gene, mean that when a cell, cell population, or tissue is treated with said LPA RNAi agents, comparing to the same cell, cell population, or tissue prior to the administration of LPA RNAi agents, the gene expression (as measured by the level of RNA transcribed from the gene or the level of polypeptides, proteins or protein subunits translated from mRNA in the cell, cell population or tissue in which the LPA gene is transcribed) is reduced.

In some embodiment, the level of the expression of gene and/or the level of mRNA of LPA in subjects administered the LPA RNAi agents, comparing to subjects prior to the administration of the LPA RNAi agents or subjects without the administration of the LPA RNAi agents, is reduced by at least approximately 5% or more, e.g., 5% to 98%. The level of the expression of gene and/or the level of mRNA in the subject can be reduced in cells, cell populations, and/or tissues in the subjects. In some embodiments, the level of protein of LPA in subjects administered the LPA RNAi agents, comparing to subjects prior to the administration of the LPA RNAi agent or to subjects not receiving the LPA RNAi agents, is reduced by more than 5%, e.g., 5% to 98%. The protein level in the subject can be reduced in cells, cell populations, tissues, blood, and/or other fluids of the subjects. A reduction in gene expression, mRNA, or protein levels can be assessed by any method known in the field. Reduction or decrease in tLPA mRNA level and/or protein level is collectively referred to in the present application as a reduction or decrease of LPA, or inhibition or reduction of the expression of LPA.

Introduction into cells when referring to LPA RNAi agents means the functionally delivery of LPA RNAi agents into cells. Functional delivery means that the RNAi agent is delivered to the cells and has the interested biological activity (e.g., sequence-specific inhibition of gene expression).

the present application is further described by specific examples below.

### Example 1 Synthesis of the compound GENO-Gal-6

### (1) Synthetic route

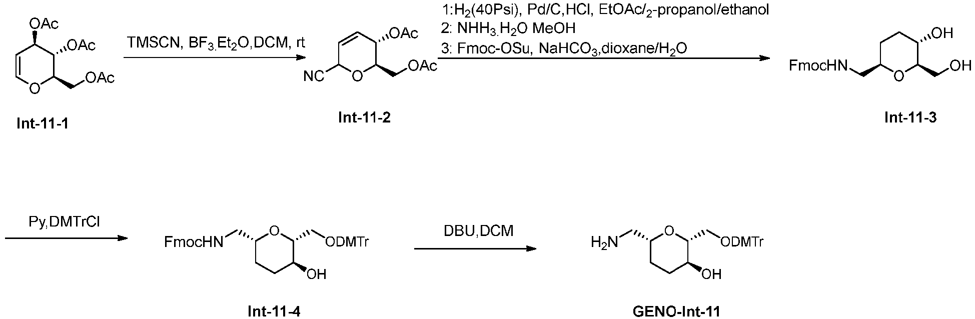

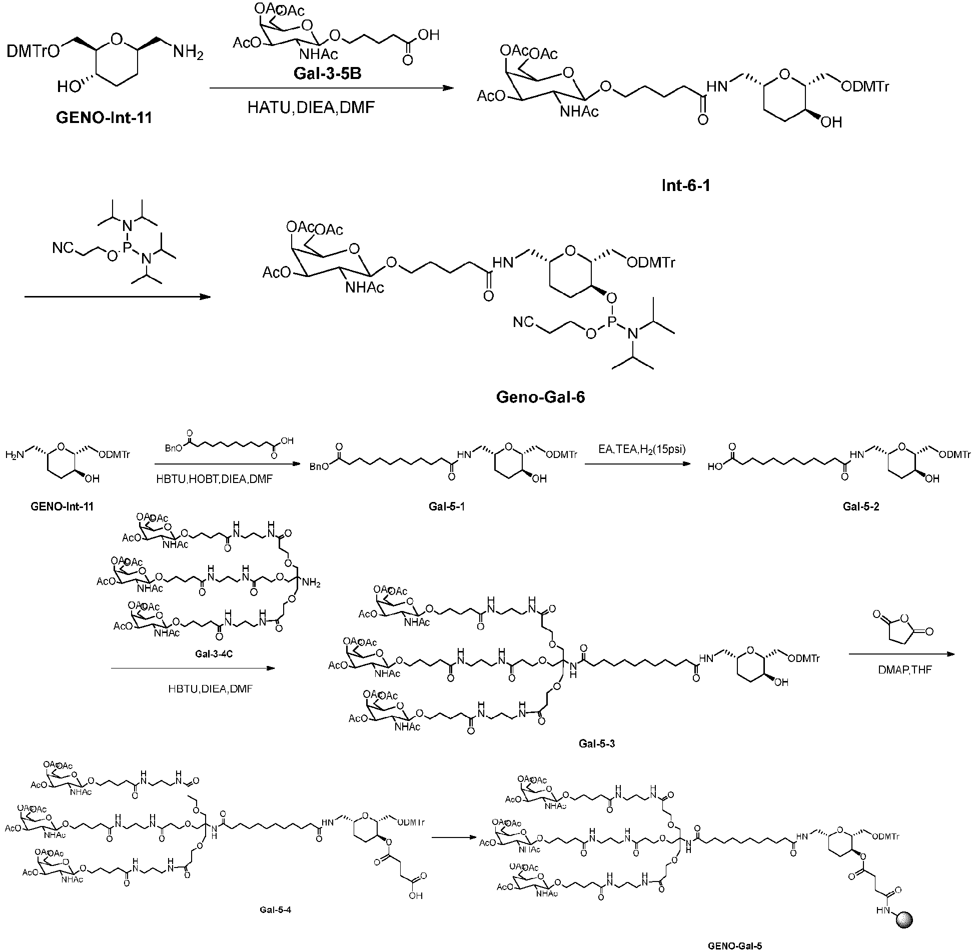

### (2) Specific synthesis steps

### 1) Preparation of compound Int-11-2

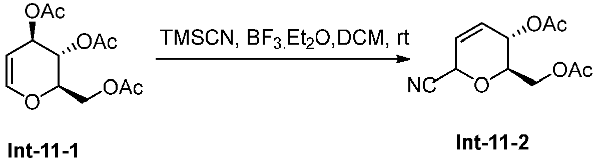

Int-11-1 (10 g) was dissolved in (140 mL) DCM (Dichloromethane) and cooled to 0°C, then TMSCN (Trimethylsilyl cyanide) (4.01g) and BF₃.Et₂O (2.83mL) were dropwise added, and reacting for 10 min. The reaction was monitored by TLC plate (Hexane: EtOAc=5: 1, KMnO₄ color rendering, raw material Rf=0.3, α configuration Rf=0.28, β configuration Rf=0.27) the reaction of raw material was complete. After the reaction was complete, 100 mL saturated NaHCO₃ aqueous solution was added to the reaction solution, and another 100 ml DCM was added to separate the organic phase. The organic phase was washed with saturated salt once to concentrate the organic phase, then dissolved in EtOAc (200 mL) and washed with NaHCO₃ aqueous solution (100 mL) once, saturated salt once, and the organic phase was dried and concentrated with anhydrous sodium sulfate. Then purification by positive silica gel columns with the polarity slowly increasing with Hexane/EtOAc = 20% to obtain α configuration, 25% to obtain β configuration, α configuration obtained (5.3 g) white solid, β configuration obtained (3.7 g) colorless oil. ¹H NMR (400 MHz, DMSO) δ 6.03-6.06 (dt, 1H), 5.91-5.94 (dt, 1H ), 5.35 (dq, 1H), 5.12 (m, 1H), 4.30 (dd, 1H), 4.30 (dd, 1H), 3.82 (ddd, 1H), 2.10-2.12 (2s, 6H).

### 2) Preparation of compound Int-11-3

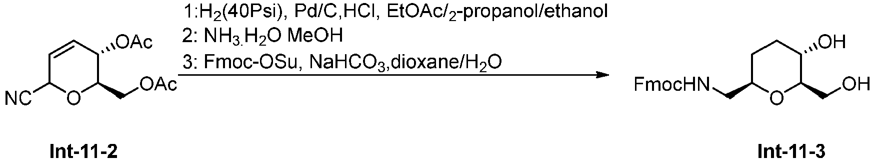

Aqueous HCl solution (1 M, 17.2 mL) was added to the suspension of Int-11-2 (3.7 g, β configuration, colorless oil) and 10% Pd/C (377 mg), and the mixture of ethyl acetate/2-propanol/ethanol (2:1:1, 90 mL) was added. The reaction was stirred at hydrogen (40 Psi) for 48 hours. The reaction was monitored by TLC, the catalyst was removed by diatomite filtration, and the solvent was concentrated under reduced pressure. The obtained crude product was washed twice with toluene, then dissolved in (10 mL) methanol, added 28% NH₃·H₂O(30 mL), stirred at room temperature for 16 hours, and the reaction solution was concentrated and washed with toluene acetonitrile 1:1 solution (50 mL) for 3 times. 1 g of the above crude product was dissolved in (40 mL) H₂O and cooled to 0°C, NaHCO₃ (1.4 g), Na₂CO₃ (0.88 g) were added, and Fmoc-OSu (2.13 g) was dissolved in dioxane (40 mL) solvent. Then it is dropwise added to the above aqueous solution and reacting at room temperature for 1 hour. The reaction was monitored by TLC (DCM: MeOH= 10:1, 254nm, Rf = 0.5). purification by positive column with MeOH/DCM=5% to obtain product, the Int-11-3 (340 mg) colorless solid was obtained. ¹H NMR (CDCl3): δ 7.76-7.78 (d, 2H), 7.58-7.60 (d, 2H ), 7.39-7.42 (t, 2H), 7.30-7.34 (t, 2H), 5.12 (t, 1H), 4.42-4.52 (m, 2H), 4.22 (t, 1H), 3.84 (br. s, 2H). 3.47-3.57 (m, 3H), 3.11-3.24 (m, 2H), 1.30-1.56, 1.67-1.72, 2.05-2.22 (m, 4H).

### 3) Preparation of compound Int-11-4

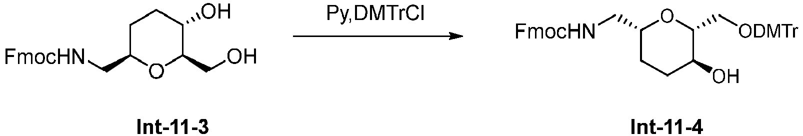

Int-11-3 (340 mg) was dissolved in 2 ml of pyridine and cooled to 0°C, DMTrCl (450 mg) was dissolved in 2 ml of pyridine and then dropwise added to the above solution, monitoring by TLC, the reaction of raw material was complete. 5 ml aqueous solution was added to quench the reaction, and purification by reverse column C18 with MeCN/H₂O=80% to obtain the product int-11-4 (290 mg).

### 4) Preparation of compound GENO-Int-11

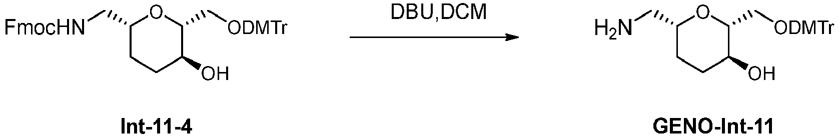

Int-11-4 (700 mg, 1.02 mmol) was dissolved in 2 mL of dichloromethane, then DBU (310 mg, 2.04 mmol) was added to the above solution, monitoring by TLC, the reaction of raw material was complete. 5 ml saturated Na₂CO₃ solution was added to quench the reaction, extracting with 20 mL dichloromethane, and concentrating organic phase with DCM: MeOH=10:1, (254nm, Rf = 0.5). purification by positive column with MeOH/DCM=5% to obtain the product, GENO-Int-11(550 mg) yellow solid was obtained.

### 5) Preparation of compound Int-6-1

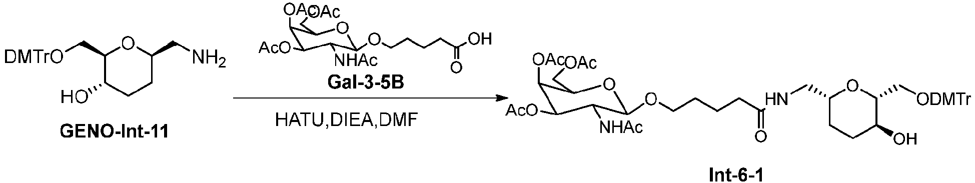

Gal-3-5B (1.45g, 3.24 mmol) was dissolved in anhydrous DMF (10 mL) solvent, then HATU (1.64 g, 4.32 mmol), 3A molecular sieve (1g) and DIEA(1.07 mL, 6.47 mmol)) were added, the reaction liquid was stirred at room temperature for 30min, and then GENO-int-11 (1g, 2.16 mmol) was dissolved in DMF(10 mL) and added to the reaction liquid. The reaction liquid was stirred overnight at room temperature under the protection of argon gas. The reaction solution was filtered, purification by reverse column C18 with MeCN/H₂O=60% to obtain the product Int-6-1 (1.8 g). ¹H NMR: (400 MHz, DMSO-d₆)δ 7.80 (d, *J* = 9.2 Hz, 1H), 7.71-7.73 (m, 1H), 7.39-7.41 (m, 2H), 7.24-7.30 (m, 6H), 7.18-7.21 (m, 2H), 6.87 (d, *J* = 8.8 Hz, 4H), 5.21 (d, *J* = 3.6 Hz, 1H), 4.95-4.98 (m, 1H), 4.59 (d, *J* = 6.4 Hz, 1H), 4.47 (d, *J* = 8.4 Hz, 1H), 4.00-4.05 (m, 2H), 3.83-3.90 (m, 1H), 3.64-3.73 (m, 7H), 3.23-3.38 (m, 8H), 2.97-3.14 (m, 3H), 2.04-2.14 (m, 5H), 1.98 (s, 3H), 1.89 (s, 3H), 1.77 (s, 3H), 1.64-4.66 (m, 1H)1.42-1.50 (m, 4H).

### 5) Preparation of compound Geno-Gal-6

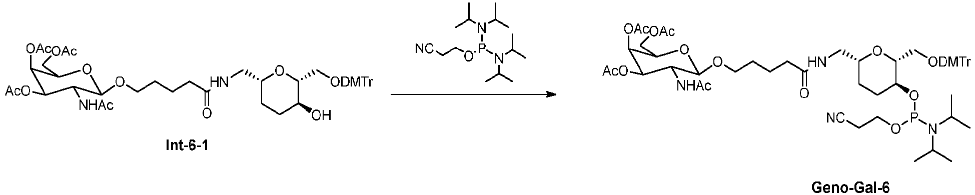

Int-6-1 (2.1g, 2.35 mmol) was dissolved in DCM(30 mL), Tetrazole (33 mg, 0.47 mmol), NMI(77.2 mg, 0.94 mmol) and 2g molecular sieve were added, and the reaction liquid was replaced by argon three times, stirring at room temperature for 20min, then the phosphorus reagent (920.81 mg, 3.06 mmol) was dissolved in a small amount of methylene chloride and added to the reaction liquid, stirring at room temperature for 1 hour. The reaction liquid was washed twice with saturated NaHCO₃ aqueous solution, once with water, and once with salt water, and concentrated at room temperature. purification by reverse column C18 with MeCN/ H₂O=65% to obtain the product GENO-Gal-6 (1.5 g). ¹H NMR: (400 MHz, CD₃CN) δ 7.48-7.50 (m, 2H), 7.20-7.36 (m, 7H), 6.83-6.87 (m, 4H), 6.45-6.51 (m, 1H), 5.28 (d, *J* = 3.2 Hz, 1H), 4.98-5.02 (m, 1H), 4.49 (d, *J* = 8.4 Hz, 1H), 3.90-4.12 (m, 4H), 3.24-3.76 (m, 20H), 3.02-3.11 (m, 1H), 2.49-2.59 (m, 1H), 2.36-2.39 (m, 1H), 2.08-2.25 (m, 9H), 1.97 (s, 3H), 1.91 (s, 3H), 1.83 (s, 3H), 1.71-1.74 (m, 1H), 1.46-1.63 (m, 5H), 0.85-1.39 (m, 20H).

### 6) Preparation of compound Gal-5-1

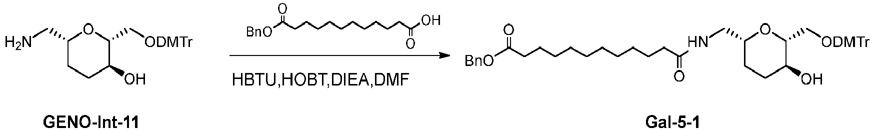

Monobenzyl 1, 12-dodecanediate (387 mg, 1.2 mmol) was dissolved in an anhydrous DMF (N, N-dimethylformamide) (10 mL) solvent, then HBTU (O-Benzotriazole-N,N,N',N'-tetramethyl-uronium-hexafluorophosphate) (546 mg, 1.44 mmol), DIEA(N,N-diisopropylethylamine)(0.65 mL, 3.6 mmol), HOBT(1-Hydroxybenzotriazole) (324 mg, 2.4 mmol) and GENO-Int-11 (560 mg, 1.2 mmol) were added, then stirring overnight at room temperature under argon protection. The reaction liquid was purified by reverse column C18 with MeCN/ H₂O=70% to obtain Gal-5-1 (200 mg). ¹H NMR (400 MHz, DMSO) δ 7.68 (t, 1H), 7.49 - 7.12 (m, 14H), 6.86 (d, 4H), 5.07 (s, 2H), 4.58 (d, 1H), 3.73 (s, 6H), 3.38 (dd, 1H), 3.28 - 3.16 (m, 3H), 3.22-2.85 (m, 3H), 2.41 - 2.25 (m, 2H), 2.11 - 2.00 (m, 2H), 1.92 (dd, 1H), 1.64 (d, 1H), 1.57 - 1.41 (m, 4H), 1.27 - 1.06 (m, 14H).

### 7) Preparation of compound Gal-5-2

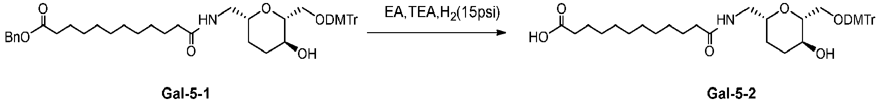

Gal-5-1 (200 mg, 0.26mmol) was dissolved in 5mL ethyl acetate, 50 mg palladium carbon and triethylamine (0.11mL, 0.78 mmol) were added, the reaction liquid was stirred in hydrogen (15psi) at room temperature for 16 hours, and the reaction liquid was filtered and concentrated to obtain Gal-5-2 (150 mg).

### 8) Preparation of compound Gal-5-3

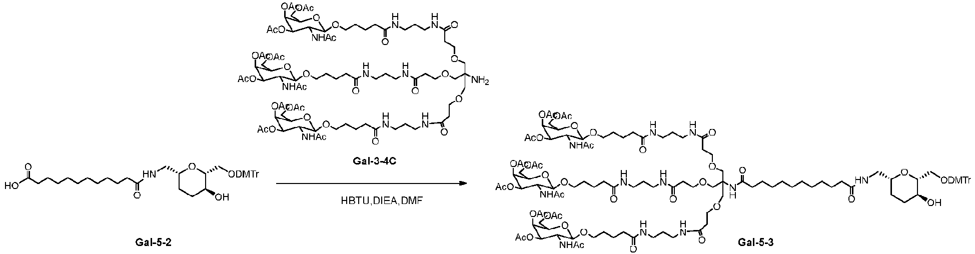

Gal-5-2 (corresponding formula I-1) (150 mg, 0.22 mmol) was dissolved in 5 mL anhydrous DMF, and HBTU (101 mg, 0.22 mmol), DIEA (0.16 mL), 3A molecular sieve (1 g) and Gal-3-4C (441 mg.0.22 mmol) were added to the solution. The mixture was then stirred at room temperature for 16 h, and the reaction liquid was purified by reverse column C18 with MeCN/H₂O=60% to obtain the product Gal-5-3 (380 mg). ¹H NMR (400 MHz, DMSO) δ 7.91 - 7.67 (m, 10H), 7.42 (d, 2H), 7.34 - 7.24 (m, 6H), 7.20 (d, 1H), 6.99 (s, 1H), 6.87 (d, 4H), 5.21 (d, 3H), 4.97 (dd, 3H), 4.60 (s, 1H), 4.48 (d, 3H), 4.09 - 3.95 (m, 10H), 3.93 - 3.82 (m, 3H), 3.77 - 3.66 (m, 9H), 3.61 - 3.48 (m, 12H), 3.45 - 3.20 (m, 16H), 3.09 - 2.95 (m, 15H), 2.27 (t, 6H), 2.10 (s, 9H), 2.05 (dd, 10H), 1.99 (s, 9H), 1.89 (s, 9H), 1.76 (d, 9H), 1.63 - 1.38 (m, 24H), 1.16 (s, 14H).

### 9) Preparation of compound Gal-5-4

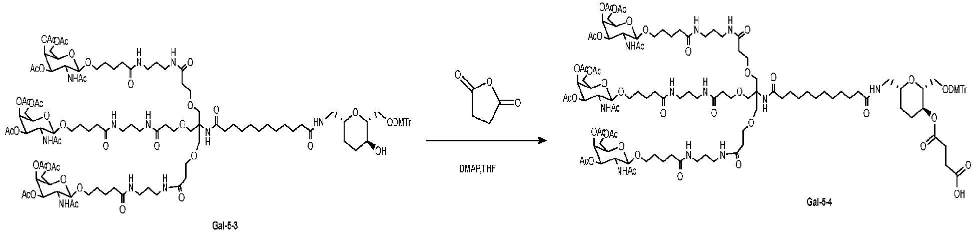

Gal-5-3 (370 mg, 0.15 mmol), succinic anhydride (75 mg, 0.76 mmol), 3A molecular sieves (0.5 g) and DMAP(4-Dimethylaminopyridine) (46 mg, 0.38 mmol) were dissolved in 5mL THF (Tetrahydrofuran), and then the reaction liquid was stirred overnight at 40°C. The reaction liquid was purified by reverse column C18 with MeCN/ H2O=40% to obtain the product Gal-5-4 (220 mg).

### 10) Synthesis of the galactosamine compound GENO-Gal-5 attached to the solid phase carrier

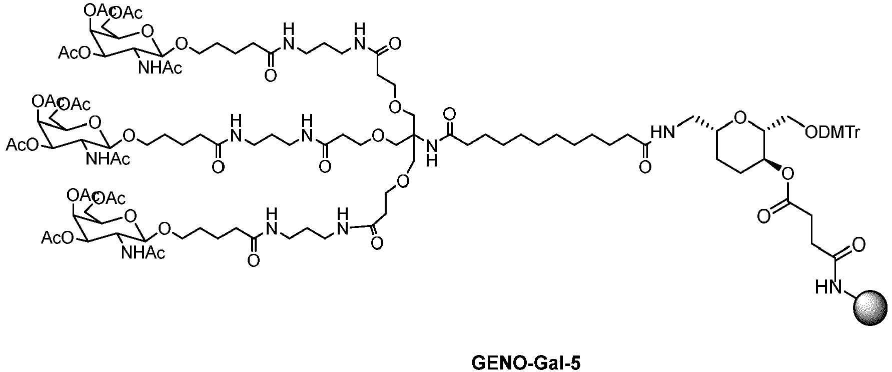

Gal-5-4 (220 mg, 0.086 mmol) was suspended in 4 ml CH₃CN and 2 mL DMF, and DIEA (0.035 mL, 0.216mmol) and HBTU (49.07 mg, 0.129 mmol) were added, stirring at room temperature for 5 minutes, aminomethyl resin (99.51 mg, 100-200 mesh, amino load 250umol/g) was added to the reaction liquid, the reaction was carried out at 25 °C and the rotating speed was 220 RPM, filtering after 16h reaction, the filter cake was washed with DCM for 3 times, 30ml each time, and acetonitrile for 3 times, 30ml each time, 30ml of N-hexane for 3 times, drying in a vacuum oil pump for 2h, and then mixed reagents (CapB1, 4-dimethylaminopyridine, N-methylimidazole and acetonitrile, 11.2 mL/12.4 mg/0.50 mL/4.32 mL) were added for capping reaction. Placeing on a shaking table at 25°C, the rotating speed was 220 r/min, reacting for 16h, the reaction liquid was filtered, the filter cake was washed with acetonitrile three times, 30ml each time, and drained to dry, drying overnight under vacuum oil pump under pressure, the target product, 160 mg GNEO-Gal-5 compound, was obtained.

### Example 2 Synthesis of siRNA

The unmodified siRNA was provided by Wuxi AppTec (see Table 1 for the sequence). The synthesis process of modified siRNA (see Tables 2 and 3) is briefly described as follows: On the Dr. Oligo 48 synthesizer (Biolytic), starting with the Universal CPG solid support, nucleoside phosphoramidites monomers were connected one by one according to the synthesis procedure. The raw materials of RNA phosphoramidites monomers such as 2'-F RNA and 2'-O-methyl RNA and so on were purchased from Huaren of Wuhu and Hongene of Shanghai. 5-ethylsulfur-1H-tetrazole (ETT) was used as an activator (0.6M acetonitrile solution), by using 0.22M PADS dissolved in a mixed solvent with a 1:1 volume ratio of acetonitrile and trimethylpyridine (Shanghai Lingjiang), the solution was obtained and used as a curing reagent, pyridine iodide/aqueous solution (Shanghai Lingjiang) was used as an oxidant.

In oligonucleotides, nucleotide monomers are linked to each other by 5'-3'-phosphodiester bonds, including thiophosphate and phosphodiester bonds.

After solid-phase synthesis, the oligoribonucleotides were cleaved from the solid support and soaked in a 3:1 solution of 28% ammonia and ethanol at 50°C for 15 hours. Then centrifuging, transferring the supernatant to another centrifuge tube, concentrating and evaporating to dry, purification use C18 reverse chromatography purification, mobile phases were 0.1M TEAA and acetonitrile, and use 3% trifluoroacetic acid solution to remove DMTr. Collecting target oligonucleotides, freezedrying, identifying as target products by LC-MS, and quantifying by UV (260nm).

The obtained single-stranded oligonucleotides were annealed according to the complementary pairing and the equal molar ratio. Finally, the obtained double-stranded siRNA was dissolved in 1×PBS or sterile water, and adjusted to the required concentration for use in the experiment.

In the sequences in Tables 1, 2, and 3, symbols are used to represent nucleotides (esters) modified as follows:
A = adenosine-3 '-phosphate
C = cytidine-3 '-phosphate
G = guanosine-3 '-phosphate
U = uridine-3 '-phosphate
Am = 2 '-O-methyladenosine-3' -phosphate
Ams = 2 '-O-methyladenosine-3' -phosphorothioate
Cm = 2 '-O-methylcytidine-3' -phosphate
Cms = 2 '-O-methylcytidine-3' -phosphorothioate
Gm = 2 '-O-methylguanosine-3' -phosphate
Gms = 2 '-O-methylguanosine-3' -phosphorothioate
Um = 2 '-O-methyluridine -3' -phosphate
Urns = 2 '-O-methyluridine -3' -phosphorothioate
Af = 2 '-fluoroadenosine-3' -phosphate
Afs = 2 '-fluoroadenosine-3' -phosphorothioate
Cf = 2 '-fluorocytidine-3' -phosphate
Cfs = 2 '-fluorocytidine-3' -phosphorothioatee
Gf = 2 '-fluoroguanosine-3' -phosphate
Gfs = 2 '-fluoroguanosine-3' -phosphorothioate
Uf = 2 '-fluorouridine -3' -phosphate
Ufs = 2 '-fluorouridine -3' -phosphorothioate
Where: lowercase letter m indicates that a nucleotide adjacent to the left of the letter m is a methoxy-modified nucleotide; The lowercase letter f indicates that a nucleotide adjacent to the left of the letter f is a fluorinated nucleotide;
When the lowercase letter s is in the middle of the uppercase letter, it indicates that the connection between the two nucleotides adjacent to the left and right of the letter s is a phosphonothioate linkage;
When the lowercase letter s is the first in the 3 'end, it indicates that the end of one nucleotide adjacent to the left of the letter s is a phosphorothioate group;

**Table 1 ID numbers and sequences of Unmodified siRNAs targeting human LPA mRNA**

| ID of LPA RNAi agent | Base sequence of antisense strand(5'→3') | SEQ ID NO. | Base sequence of positive- sense strand(5'→3') | SEQ ID NO. |
|---|---|---|---|---|
| Geno-1-001 | UAAUCUCAGCAUCUGGAUUCCUG | 2 | GGAAUCCAGAUGCUGAGAUUA | 93 |
| Geno-1-002 | AAACAGCCGUGGACGUCGCAAGG | 3 | UUGCGACGUCCACGGCUGUUU | 94 |
| Geno-1-003 | AUAGACGACCAAGACUGACAUGU | 4 | AUGUCAGUCUUGGUCGUCUAU | 95 |
| Geno-1-004 | AUUGACAUGUCCUUCCUGUGACA | 5 | UCACAGGAAGGACAUGUCAAU | 96 |
| Geno-1-005 | AUGCUUCCAGGACAUUUCUUCGG | 6 | GAAGAAAUGUCCUGGAAGCAU | 97 |
| Geno-1-006 | UCAUUCUCAAUAACAAGGAGCUG | 7 | GCUCCUUGUUAUUGAGAAUGA | 98 |
| Geno-1-007 | UAACAAACCUUGAAACACGAGCA | 8 | CUCGUGUUUCAAGGUUUGUUA | 99 |
| Geno-1-008 | AUAACUCUGUCCAUUACCAUU | 9 | UGGUAAUGGACAGAGUUAUUU | 100 |
| Geno-1-009 | AUAACUCUGUCCAUUACCAUGGU | 10 | CAUGGUAAUGGACAGAGUUAU | 101 |
| Geno-1-010 | AACUCUGUCCAUCACCAUGGUAG | 11 | ACCAUGGUGAUGGACAGAGUU | 102 |
| Geno-1-011 | AUAACUCUGUCCAUCACCAUGGU | 12 | CAUGGUGAUGGACAGAGUUAU | 103 |
| Geno-1-012 | AUGUGCCUCGAUAACUCUGUCCA | 13 | GACAGAGUUAUCGAGGCACAU | 104 |
| Geno-1-014 | AAGUAACAAACCUUGAAACACGA | 14 | GUGUUUCAAGGUUUGUUACUU | 105 |
| Geno-1-015 | UUCAUUCUCAAUAACAAGGAGCU | 15 | CUCCUUGUUAUUGAGAAUGAA | 106 |
| Geno-1 - 016 | UACAAUGCUUCCAGGACAUUUCU | 16 | AAAUGUCCUGGAAGCAUUGUA | 107 |
| Geno-1-017 | AAGAUUGACAUGUCCUUCCUGUG | 17 | CAGGAAGGACAUGUCAAUCUU | 108 |
| Geno-1-018 | AUUCUGUCACUGGACAUCGUGUC | 18 | CACGAUGUCCAGUGACAGAAU | 109 |
| Geno-1-019 | AUGUCCUUCCUGUAACAGUGGUG | 19 | CCACUGUUACAGGAAGGACAU | 110 |
| Geno-1-020 | AAGGACUAAUCUCAGCAUCUGGA | 20 | CAGAUGCUGAGAUUAGUCCUU | 111 |
| Geno-1-021 | AACACCAAGGACUAAUCUCAGCA | 21 | CUGAGAUUAGUCCUUGGUGUU | 112 |
| Geno-1-022 | AUUCUGUGGUUCUCUGAUGCCAG | 22 | GGCAUCAGAGAACCACAGAAU | 113 |
| Geno-1-023 | UUUCUCAGGUGGUGCUUGUUCAG | 23 | GAACAAGCACCACCUGAGAAA | 114 |
| Geno-1-024 | UUCCUGCAGUAGUUCAUUGUCAG | 24 | GACAAUGAACUACUGCAGGAA | 115 |
| Geno-1-025 | UUGUUCUGAGACUGACUUGCCAG | 25 | GGCAAGUCAGUCUCAGAACAA | 116 |
| Geno-1-026 | UUGCACACUUCAUUCUCAAUAAC | 26 | UAUUGAGAAUGAAGUGUGCAA | 117 |
| Geno-1-027 | UGAGCCUCGAUAACUCUGUUU | 27 | ACAGAGUUAUCGAGGGCUCATT | 118 |
| Geno-1-029 | UAUGUGCCUCGAUAACUCUGUCC | 28 | ACAGAGUUAUCGAGGCACAUA | 119 |
| Geno-1-030 | AUGAGCCUCGAUAACUCUGUCCA | 29 | GACAGAGUUAUCGAGGCUCAU | 120 |
| Geno-1-031 | AAUGAGCCUCGAUAACUCUGUCC | 30 | ACAGAGUUAUCGAGGCUCAUU | 121 |
| Geno-1-032 | UUCUUCCUGUGACAGUGGUGGAG | 31 | CCACCACUGUCACAGGAAGAA | 122 |
| Geno-1-034 | UUCGUUUGAUUGCUGUCUAUUAU | 32 | AAUAGACAGCAAUCAAACGAA | 123 |
| Geno-1-035 | UAUAGUGAUUGCACACUUCAUUC | 33 | AUGAAGUGUGCAAUCACUAUA | 124 |
| Geno-1-036 | UUCCAAACCUUGUUCUGAGACUG | 34 | GUCUCAGAACAAGGUUUGGAA | 125 |
| Geno-1-037 | UACAGUCUUGUUCAGAAGGAGGC | 35 | CUCCUUCUGAACAAGACUGUA | 126 |
| Geno-1-038 | UUUUCUCAGGUGGUGCUUGUUCA | 36 | AACAAGCACCACCUGAGAAAA | 127 |
| Geno-1-039 | UAGUAUUCUGUGGUUCUCUGAUG | 37 | UCAGAGAACCACAGAAUACUA | 128 |
| Geno-1-040 | UAACACCAAGGACUAAUCUCAGC | 38 | UGAGAUUAGUCCUUGGUGUUA | 129 |
| Geno-1-041 | AUAACACCAAGGACUAAUCUCAG | 39 | GAGAUUAGUCCUUGGUGUUAU | 130 |
| Geno-1-042 | UUGAUUCCAUCACUGGACAUUGC | 40 | AAUGUCCAGUGAUGGAAUCAA | 131 |
| Geno-1-043 | AUAUGCCUCGAUAACUCCGUCCA | 41 | GACGGAGUUAUCGAGGCAUAU | 132 |
| Geno-1-044 | AUACAGUCUUGUUCAGAAGGAGG | 42 | UCCUUCUGAACAAGACUGUAU | 133 |
| Geno-1-045 | UUCAAGCAGUGAGCAGCAGUCAG | 43 | GACUGCUGCUCACUGCUUGAA | 134 |
| Geno-1-046 | UACUUAUAGUGAUUGCACACUUC | 44 | AGUGUGCAAUCACUAUAAGUA | 135 |
| Geno-1-047 | UACAGAAUUUGUCAGUCAGACCU | 45 | GUCUGACUGACAAAUUCUGUA | 136 |
| Geno-1-049 | UUCAUGAUCAAGCCAGCAUUUGG | 46 | AAAUGCUGGCUUGAUCAUGAA | 137 |
| Geno-1-050 | UUCCUGUGACAGUGGUAGAGAAU | 47 | UCUCUACCACUGUCACAGGAA | 138 |
| Geno-1-051 | AAUAUUCUGUUGUCCUCUGAUGC | 48 | AUCAGAGGACAACAGAAUAUU | 139 |
| Geno-1-052 | CAGUAGUUCAUGAUCAAGCCAGC | 49 | UGGCUUGAUCAUGAACUACUG | 140 |
| Geno-1-054 | AUACAGAAUUUGUCAGUCAGACC | 50 | UCUGACUGACAAAUUCUGUAU | 141 |
| Geno-1-055 | UAUUUGUCCUUCUCGAAGCAAAC | 51 | UUGCUUCGAGAAGGACAAAUA | 142 |
| Geno-1-056 | UUCUUCAAGCAGUGAGCAGCAGU | 52 | UGCUGCUCACUGCUUGAAGAA | 143 |
| Geno-1-057 | AACAUACAGUCUUGUUCAGAAGG | 53 | UUCUGAACAAGACUGUAUGUU | 144 |
| Geno-1-058 | UUCUGUUUCUGAGCAUUGUGUCA | 54 | ACACAAUGCUCAGAAACAGAA | 145 |
| Geno-1-059 | UUCAGUUGGUGCUUCUUCAGAAG | 55 | UCUGAAGAAGCACCAACUGAA | 146 |
| Geno-1-060 | UAUAACACCAAGGACUAAUCUCA | 56 | AGAUUAGUCCUUGGUGUUAUA | 147 |
| Geno-1-061 | UUCUGUCACUGGACAUUGUGUCA | 57 | ACACAAUGUCCAGUGACAGAA | 148 |
| Geno-1-062 | AUAACUCUGUCCAUCACCUCGGU | 58 | CGAGGUGAUGGACAGAGUUAU | 149 |
| Geno-1-063 | AUUCUGUUUCUGAGCAUUGUGUC | 59 | CACAAUGCUCAGAAACAGAAU | 150 |
| Geno-1-064 | UUUCUUGGAUUCAUUGUGUAGCA | 60 | CUACACAAUGAAUCCAAGAAA | 151 |
| Geno-1-065 | UAUUUCCUGAACAUGAGAUUCGA | 61 | GAAUCUCAUGUUCAGGAAAUA | 152 |
| Geno-1-066 | AAUGUAUUUGUCCUUCUCGAAGC | 62 | UUCGAGAAGGACAAAUACAUU | 153 |
| Geno-1-067 | AAUACAGAAUUUGUCAGUCAGAC | 63 | CUGACUGACAAAUUCUGUAUU | 154 |
| Geno-1-069 | AUUCCUGCAGUAGUUCAUGAUCA | 64 | AUCAUGAACUACUGCAGGAAU | 155 |
| Geno-1-070 | UAAUAUUCUGUUGUCCUCUGAUG | 65 | UCAGAGGACAACAGAAUAUUA | 156 |
| Geno-1-071 | AUAAUAUUCUGUUGUCCUCUGAU | 66 | CAGAGGACAACAGAAUAUUAU | 157 |
| Geno-1-072 | AUAACAAUAAGGAGCUGCCACAG | 67 | GUGGCAGCUCCUUAUUGUUAU | 158 |
| Geno-1-074 | UAAUACAGAAUUUGUCAGUCAGA | 68 | UGACUGACAAAUUCUGUAUUA | 159 |
| Geno-1-075 | AAACACGAGCAUAGACACCAGGC | 69 | CUGGUGUCUAUGCUCGUGUUU | 160 |
| Geno-1-076 | UCUAUUUCCUGAACAUGAGAUUC | 70 | AUCUCAUGUUCAGGAAAUAGA | 161 |
| Geno-1-077 | AUCACAGUAGUCAAAAAGUUUUC | 71 | AAACUUUUUGACUACUGUGAU | 162 |
| Geno-1-078 | UCUAGGACACCUGAUUCUGUUUC | 72 | AACAGAAUCAGGUGUCCUAGA | 163 |
| Geno-1-079 | UUCUUCCUGUGAUAGUGGUGGAG | 73 | CCACCACUAUCACAGGAAGAA | 164 |
| Geno-1-080 | AUUCUGUCACUGGACAUUGUGUC | 74 | CACAAUGUCCAGUGACAGAAU | 165 |
| Geno-1-081 | UUGAUUCUGUCACUGGACAUUGU | 75 | AAUGUCCAGUGACAGAAUCAA | 166 |
| Geno-1-082 | UGUUCUUCCUGUGAUAGUGGUGG | 76 | ACCACUAUCACAGGAAGAACA | 167 |
| Geno-1-083 | UUCAGAAUGAGCCUCCAUGCUUG | 77 | AGCAUGGAGGCUCAUUCUGAA | 168 |
| Geno-1-084 | AAUCAAAUGAAGAGGAUGCACAG | 78 | GUGCAUCCUCUUCAUUUGAUU | 169 |
| Geno-1-085 | AUUACUUUGUCAGUGAUGACGGC | 79 | CGUCAUCACUGACAAAGUAAU | 170 |
| Geno-1-086 | UGAAACACGAGCAUAGACACCAG | 80 | GGUGUCUAUGCUCGUGUUUCA | 171 |
| Geno-1-087 | UUAAUACAGAAUUUGUCAGUCAG | 81 | GACUGACAAAUUCUGUAUUAA | 172 |
| Geno-1-089 | UAUAACAAUAAGGAGCUGCCACA | 82 | UGGCAGCUCCUUAUUGUUAUA | 173 |
| Geno-1-090 | AUCUCAGCAUCUGGAUUCCUGCA | 83 | CAGGAAUCCAGAUGCUGAGAU | 174 |
| Geno-1-091 | AAUCUCAGCAUCUGGAUUCCUGC | 84 | AGGAAUCCAGAUGCUGAGAUU | 175 |
| Geno-1-092 | UCGUAUAACAAUAAGGAGCUGCC | 85 | CAGCUCCUUAUUGUUAUACGA | 176 |
| Geno-1-094 | UUCAAAUCAAAAUAAGUGCAGAG | 86 | CUGCACUUAUUUUGAUUUGAA | 177 |
| Geno-1-095 | UUGAAACACGAGCAUAGACACCA | 87 | GUGUCUAUGCUCGUGUUUCAA | 178 |
| Geno-1-096 | AUGUAACAUUCAGUCCUGGCGGU | 88 | CGCCAGGACUGAAUGUUACAU | 179 |
| Geno-1-097 | UUCCCACAAUCAAAUGAAGAGGA | 89 | CUCUUCAUUUGAUUGUGGGAA | 180 |
| Geno-1-098 | AUGUCCUUCCUGUGACAGUGGUG | 90 | CCACUGUCACAGGAAGGACAU | 181 |
| Geno-1-099 | AUGUUCUUCCUGUGAUAGUGGUG | 91 | CCACUAUCACAGGAAGAACAU | 182 |
| Geno-1-100 | AAGGACACUUGAUUCUGUCACUG | 92 | GUGACAGAAUCAAGUGUCCUU | 183 |

**Table 2 ID numbers and sequences of modified siRNAs targeting human LPA mRNA**

| Number of double strand | Number of antisens e strand | antisense strand 5'→3' | Origin al SEQ ID NO. | Numbe r of sense strand | sense strand 5'→3' | Origin al SEQ ID NO. |
|---|---|---|---|---|---|---|
| Geno-1-010M | LPA-1AS | AmsAfsCm UmCmUf GmUmCm CmAmUm CmAfCm CfAmUm GmGmUms AmsGm | 11 | LPA-69SS | AmsCmsCm AmUmGm GfUmGf AfUfGm GmAmCm AmGmAm GmUmUm | 102 |
| Geno-1-011M | LPA-2AS | AmsUfsAm AmCmUf CmUmGm UmCmCm AmUfCm AfCmCm AmUmGms GmsUm | 12 | LPA-70SS | CmsAmsUm GmGmUm GfAmUf GfGfAm CmAmGm AmGmUm UmAmUm | 103 |
| Geno-1-019M | LPA-3AS | AmsUfsGm UmCmCf UmUmCm CmUmGm UmAfAm CfAmGm UmGmGms UmsGm | 19 | LPA-71SS | CmsCmsAm CmUmGm UfUmAf CfAfGm GmAmAm GmGmAm CmAmUm | 110 |
| Geno-1-022M | LPA-4AS | AmsUfsUm CmUmGf UmGmGm UmUmCm UmCfUm GfAmUm GmCmCms AmsGm | 22 | LPA-72SS | GmsGmsCm AmUmCm AfGmAf GfAfAm CmCmAm CmAmGm AmAmUm | 113 |
| Geno-1-023M | LPA-5AS | UmsUfsUm CmUmCf AmGmGm UmGmGm UmGfCm UfUmGm UmUmCms AmsGm | 23 | LPA-73SS | GmsAmsAm CmAmAm GfCmAf CfCfAm CmCmUm GmAmGm AmAmAm | 114 |
| Geno-1-030M | LPA-6AS | AmsUfsGm AmGmCf CmUmCm GmAmUm AmAfCm UfCmUm GmUmCms CmsAm | 29 | LPA-74SS | GmsAmsCm AmGmAm GfUmUf AfUfCm GmAmGm GmCmUm CmAmUm | 120 |
| Geno-1-031M | LPA-7AS | AmsAfsUm GmAmGf CmCmUm CmGmAm UmAfAm CfUmCm UmGmUms CmsCm | 30 | LPA-75SS | AmsCmsAm GmAmGm UfUmAf UfCfGm AmGmGm | 121 |
| | | | | | CmUmCm AmUmUm | |
| Geno-1-03 5M | LPA-8AS | UmsAfsUm AmGmUf GmAmUm UmGmCm AmCfAm CfUmUm CmAmUms UmsCm | 35 | LPA-76SS | AmsUmsGm AmAmGm UfGmUf GfCfAm AmUmCm AmCmUm AmUmAm | 20126 |
| Geno-1-041M | LPA-9AS | AmsUfsAm AmCmAf CmCmAm AmGmGm AmCfUm AfAmUm CmUmCms AmsGm | 039 | LPA-77SS | GmsAmsGm AmUmUm AfGmUf CfCfUm UmGmGm UmGmUm UmAmUm | 130 |
| Geno-1-050M | LPA-10 AS | UmsUfsCm CmUmGf UmGmAm CmAmGm UmGfGm UfAmGm AmGmAms AmsUm | 47 | LPA-78SS | UmsCmsUm CmUmAm CfCmAf CfUfGm UmCmAm CmAmGm GmAmAm | 138 |
| Geno-1-051M | LPA-11AS | AmsAfsUm AmUmUf CmUmGm UmUmGm UmCfCm UfCmUm GmAmUms GmsCm | 48 | LPA-79SS | AmsUmsCm AmGmAm GfGmAf CfAfAm CmAmGm AmAmUm AmUmUm | 139 |
| Geno-1-060M | LPA-12AS | UmsAfsUm AmAmCf AmCmCm AmAmGm GmAfCm UfAmAm UmCmUms CmsAm | 56 | LPA-80SS | AmsGmsAm UmUmAm GfUmCf CfUfUm GmGmUm GmUmUm AmUmAm | 147 |
| Geno-1-061M | LPA-13AS | UmsUfsCm UmGmUf CmAmCm UmGmGm AmCfAm UfUmGm UmGmUms CmsAm | 57 | LPA-81SS | AmsCmsAm CmAmAm UfGmUf CfCfAm GmUmGm AmCmAm GmAmAm | 148 |
| Geno-1-064M | LPA-14AS | UmsUfsUm CmUmUf GmGmAm UmUmCm | 60 | LPA-82SS | CmsUmsAm CmAmCm AfAmUf GfAfAm | 151 |
| | | AmUfUm GfUmGm UmAmGms CmsAm | | | UmCmCm AmAmGm AmAmAm | |
| Geno-1-070M | LPA-15AS | UmsAfsAm UmAmUf UmCmUm GmUmUm GmUfCm CfUmCm UmGmAms UmsGm | 65 | LPA-83SS | UmsCmsAm GmAmGm GfAmCf AfAfCm AmGmAm AmUmAm UmUmAm | 156 |
| Geno-1-076M | LPA-16AS | UmsCfsUm AmUmUf UmCmCm UmGmAm AmCfAm UfGmAm GmAmUms UmsCm | 70 | LPA-84SS | AmsUmsCm UmCmAm UfGmUf UfCfAm GmGmAm AmAmUm AmGmAm | 161 |
| Geno-1-080M | LPA-17AS | AmsUfsUm CmUmGf UmCmAm CmUmGm GmAfCm AfUmUm GmUmGms UmsCm | 74 | LPA-85SS | CmsAmsCm AmAmUm GfUmCf CfAfGm UmGmAm CmAmGm AmAmUm | 165 |
| Geno-1-081M | LPA-18AS | UmsUfsGm AmUmUf CmUmGm UmCmAm CmUfGm GfAmCm AmUmUms GmsUm | 75 | LPA-86SS | AmsAmsUm GmUmCm CfAmGf UfGfAm CmAmGm AmAmUm CmAmAm | 166 |
| Geno-1-082M | LPA-19AS | UmsGfsUm UmCmUf UmCmCm UmGmUm GmAfUm AfGmUm GmGmUms GmsGm | 76 | LPA-87SS | AmsCmsCm AmCmUm AfUmCf AfCfAm GmGmAm AmGmAm AmCmAm | 167 |
| Geno-1-086M | LPA-20AS | UmsGfsAm AmAmCf AmCmGm AmGmCm AmUfAm GfAmCm AmCmCms AmsGm | 80 | LPA-88SS | GmsGmsUm GmUmCm UfAmUf GfCfUm CmGmUm GmUmUm UmCmAm | 171 |
| Geno-1-072M | LPA-21AS | AmsUfsAm AmCmAf AmUmAm AmGmGm | 67 | LPA-89SS | GmsUmsGm GmCmAm | 158 |
| | | AmGfCm UfGmCm CmAmCms AmsGm | | | GfCmUf CfCfUm UmAmUm UmGmUm UmAmUm | |
| Geno-1-089M | LPA-22AS | UmsAfsUm AmAmCf AmAmUm AmAmGm GmAfGm CfUmGm CmCmAms CmsAm | 82 | LPA-90SS | UmsGmsGm CmAmGm CfUmCf CfUfUm AmUmUm GmUmUm AmUmAm | 173 |
| Geno-1-001M | LPA-23AS | UmsAfsAm UmCmUf CmAmGm CmAmUm CmUfGm GfAmUm UmCmCms UmsGm | 2 | LPA-91SS | GmsGmsAm AmUmCm CfAmGf AfUfGm CmUmGm AmGmAm UmUmAm | 93 |
| Geno-1-018M | LPA-24AS | AmsUfsUm CmUmGf UmCmAm CmUmGm GmAfCm AfUmCm GmUmGms UmsCm | 18 | LPA-92SS | CmsAmsCm GmAmUm GfUmCf CfAfGm UmGmAm CmAmGm AmAmUm | 109 |
| Geno-1-055M | LPA-25AS | UmsAfsUm UmUmGf UmCmCm UmUmCm UmCfGm AfAmGm CmAmAms AmsCm | 51 | LPA-93SS | UmsUmsGm CmUmUm CfGmAf GfAfAm GmGmAm CmAmAm AmUmAm | 142 |
| Geno-1-100M | LPA-26AS | AmsAfsGm GmAmCf AmCmUm UmGmAm UmUfCm UfGmUm CmAmCms UmsGm | 92 | LPA-94SS | GmsUmsGm AmCmAm GfAmAf UfCfAm AmGmUm GmUmCm CmUmUm | 183 |
| Geno-1-099M | LPA-27AS | AmsUfsGm UmUmCf UmUmCm CmUmGm UmGfAm UfAmGm UmGmGms UmsGm | 91 | LPA-95SS | CmsCmsAm CmUmAm UfCmAf   CfAfGm GmAmAm GmAmAm CmAmUm | 182 |
| Geno-1-005M | LPA-28AS | AmsUfsGm CmUmUf CmCmAm GmGmAm CmAfUm UfUmCm UmUmCms GmsGm | 6 | LPA-96SS | GmsAmsAm GmAmAm AfUmGf UfCfCm UmGmGm AmAmGm CmAmUm | 97 |
| Geno-1-075M | LPA-29AS | AmsAfsAm CmAmCf GmAmGm CmAmUm AmGfAm CfAmCm CmAmGms GmsCm | 69 | LPA-97SS | CmsUmsGm GmUmGm UfCmUf AfUfGm CmUmCm GmUmGm UmUmUm | 160 |
| Geno-1-085M | LPA-30AS | AmsUfsUm AmCmUf UmUmGm UmCmAm GmUfGm AfUmGm AmCmGms GmsCm | 79 | LPA-98SS | CmsGmsUm CmAmUm CfAmCf UfGfAm CmAmAm AmGmUm AmAmUm | 170 |
| Geno-1-084M | LPA-31AS | AmsAfsUm CmAmAf AmUmGm AmAmGm AmGfGm AfUmGm CmAmCms AmsGm | 78 | LPA-99SS | GmsUmsGm CmAmUm CfCmUf CfUfUm CmAmUm UmUmGm AmUmUm | 169 |
| Geno-1-092M | LPA-32AS | UmsCfsGm UmAmUf AmAmCm AmAmUm AmAfGm GfAmGm CmUmGms CmsCm | 85 | LPA-100SS | CmsAmsGm CmUmCm CfUmUf AfUfUm GmUmUm AmUmAm CmGmAm | 176 |
| Geno-1-026M | LPA-33AS | UmsUfsGm CmAmCf AmCmUm UmCmAm UmUfCm UfCmAm AmUmAms AmsCm | 26 | LPA-101SS | UmsAmsUm UmGmAm GfAmAf UfGfAm AmGmUm GmUmGm CmAmAm | 117 |
| Geno-1-059M | LPA-34AS | UmsUfsCm AmGmUf UmGmGm UmGmCm UmUfCm UfUmCm AmGmAms AmsGm | 55 | LPA-102SS | UmsCmsUm GmAmAm GfAmAf GfCfAm CmCmAm | 146 |
| | | | | | AmCmUm GmAmAm | |
| Geno-1-071M | LPA-35AS | AmsUfsAm AmUmAf UmUmCm UmGmUm UmGfUm CfCmUm CmUmGms AmsUm | 66 | LPA-103SS | CmsAmsGm AmGmGm AfCmAf AfCfAm GmAmAm UmAmUm UmAmUm | 157 |
| Geno-1-037M | LPA-36AS | UmsAfsCm AmGmUf CmUmUm GmUmUm CmAfGm AfAmGm GmAmGms GmsCm | 35 | LPA-104SS | CmsUmsCm CmUmUm CfUmGf AfAfCm AmAmGm AmCmUm GmUmAm | 124 |
| Geno-1-066M | LPA-37AS | AmsAfsUm GmUmAf UmUmUm GmUmCm CmUfUm CfUmCm GmAmAms GmsCm | 62 | LPA-105SS | UmsUmsCm GmAmGm AfAmGf GfAfCm AmAmAm UmAmCm AmUmUm | 153 |
| Geno-1-036M | LPA-38AS | UmsUfsCm CmAmAf AmCmCm UmUmGm UmUfCm UfGmAm GmAmCms UmsGm | 34 | LPA-106SS | GmsUmsCm UmCmAm GfAmAf CfAfAm GmGmUm UmUmGm GmAmAm | 125 |
| Geno-1-098M | LPA-39AS | AmsUfsGm UmCmCf UmUmCm CmUmGm UmGfAm CfAmGm UmGmGms UmsGm | 90 | LPA-107SS | CmsCmsAm CmUmGm UfCmAf CfAfGm GmAmAm GmGmAm CmAmUm | 181 |
| Geno-1-058M | LPA-40AS | UmsUfsCm UmGmUf UmUmCm UmGmAm GmCfAm UfUmGm UmGmUms CmsAm | 54 | LPA-108SS | AmsCmsAm CmAmAm UfGmCf UfCfAm GmAmAm AmCmAm GmAmAm | 145 |
| Geno-1-090M | LPA-41AS | AmsUfsCm UmCmAf GmCmAm UmCmUm | 83 | LPA-109SS | CmsAmsGm GmAmAm UfCmCf AfGfAm | 174 |
| | | GmGfAm UfUmCm CmUmGms CmsAm | | | UmGmCm UmGmAm GmAmUm | |
| Geno-1-039M | LPA-42AS | UmsAfsGm UmAmUf UmCmUm GmUmGm GmUfUm CfUmCm UmGmAms UmsGm | 37 | LPA-110SS | UmsCmsAm GmAmGm AfAmCf CfAfCm AmGmAm AmUmAm CmUmAm | 128 |
| Geno-1-003M | LPA-43AS | AmsUfsAm GmAmCf GmAmCm CmAmAm GmAfCm UfGmAm CmAmUms GmsUm | 4 | LPA-111SS | AmsUmsGm UmCmAm GfUmCf UfUfGm GmUmCm GmUmCm UmAmUm | 95 |
| Geno-1-014M | LPA-44AS | AmsAfsGm UmAmAf CmAmAm AmCmCm UmUfGm AfAmAm CmAmCms GmsAm | 14 | LPA-112SS | GmsUmsGm UmUmUm CfAmAf GfGfUm UmUmGm UmUmAm CmUmUm | 105 |
| Geno-1-040M | LPA-45AS | UmsAfsAm CmAmCf CmAmAm GmGmAm CmUfAm AfUmCm UmCmAms GmsCm | 38 | LPA-113SS | UmsGmsAm GmAmUm UfAmGf UfCfCm UmUmGm GmUmGm UmUmAm | 129 |
| Geno-1-047M | LPA-46AS | UmsAfsCm AmGmAf AmUmUm UmGmUm CmAfGm UfCmAm GmAmCms CmsUm | 45 | LPA-114SS | GmsUmsCm UmGmAm CfUmGf AfCfAm AmAmUm UmCmUm GmUmAm | 136 |
| Geno-1-045M | LPA-47AS | UmsUfsCm AmAmGf CmAmGm UmGmAm GmCfAm GfCmAm GmUmCms AmsGm | 43 | LPA-115SS | GmsAmsCm UmGmCm UfGmCf UfCfAm CmUmGm CmUmUm GmAmAm | 134 |
| Geno-1-057M | LPA-48AS | AmsAfsCm AmUmAf CmAmGm UmCmUm | 53 | LPA-116SS | UmsUmsCm UmGmAm | 144 |
| | | UmGfUm UfCmAm GmAmAms GmsGm | | | AfCmAf AfGfAm CmUmGm UmAmUm GmUmUm | |
| Geno-1-097M | LPA-49AS | UmsUfsCm CmCmAf CmAmAm UmCmAm AmAfUm GfAmAm GmAmGms GmsAm | 89 | LPA-117SS | CmsUmsCm UmUmCm AfUmUf UfGfAm UmUmGm UmGmGm GmAmAm | 180 |
| Geno-1-046M | LPA-50AS | UmsAfsCm UmUmAf UmAmGm UmGmAm  UmUfGm CfAmCm AmCmUms UmsCm | 44 | LPA-118SS | AmsGmsUm GmUmGm CfAmAf UfCfAm CmUmAm UmAmAm GmUmAm | 135 |
| Geno-1-021M | LPA-51AS | AmsAfsCm AmCmCf AmAmGm GmAmCm UmAfAm UfCmUm CmAmGms CmsAm | 21 | LPA-119SS | CmsUmsGm AmGmAm UfUmAf GfUfCm CmUmUm GmGmUm GmUmUm | 112 |
| Geno-1-024M | LPA-52AS | UmsUfsCm CmUmGf CmAmGm UmAmGm UmUfCm AfUmUm GmUmCms AmsGm | 24 | LPA-120SS | GmsAmsCm AmAmUm GfAmAf CfUfAm CmUmGm CmAmGm GmAmAm | 115 |
| Geno-1-020M | LPA-53AS | AmsAfsGm GmAmCf UmAmAm UmCmUm CmAfGm CfAmUm CmUmGms GmsAm | 20 | LPA-121SS | CmsAmsGm AmUmGm CfUmGf AfGfAm UmUmAm GmUmCm CmUmUm | 111 |
| Geno-1-091M | LPA-54AS | AmsAfsUm CmUmCf AmGmCm AmUmCm UmGfGm AfUmUm CmCmUms GmsCm | 84 | LPA-122SS | AmsGmsGm AmAmUm CfCmAf GfAfUm GmCmUm GmAmGm AmUmUm | 175 |
| Geno-1-004M | LPA-55AS | AmsUfsUm GmAmCf AmUmGm UmCmCm UmUfCm CfUmGm UmGmAms CmsAm | 5 | LPA-123SS | UmsCmsAm CmAmGm GfAmAf GfGfAm CmAmUm GmUmCm AmAmUm | 96 |
| Geno-1-009M | LPA-56AS | AmsUfsAm AmCmUf CmUmGm UmCmCm AmUfUm AfCmCm AmUmGms GmsUm | 10 | LPA-124SS | CmsAmsUm GmGmUm AfAmUf GfGfAm CmAmGm AmGmUm UmAmUm | 101 |
| Geno-1-062M | LPA-57AS | AmsUfsAm AmCmUf CmUmGm UmCmCm AmUfCm AfCmCm UmCmGms GmsUm | 58 | LPA-125SS | CmsGmsAm GmGmUm GfAmUf GfGfAm CmAmGm AmGmUm UmAmUm | 149 |
| Geno-1-079M | LPA-58AS | UmsUfsCm UmUmCf CmUmGm UmGmAm UmAfGm UfGmGm UmGmGms AmsGm | 73 | LPA-126SS | CmsCmsAm CmCmAm CfUmAf UfCfAm CmAmGm GmAmAm GmAmAm | 164 |
| Geno-1-006M | LPA-59AS | UmsCfsAm UmUmCf UmCmAm AmUmAm AmCfAm AfGmGm AmGmCms UmsGm | 7 | LPA-127SS | GmsCmsUm CmCmUm UfGmUf UfAfUm UmGmAm GmAmAm UmGmAm | 98 |
| Geno-1-017M | LPA-60AS | AmsAfsGm AmUmUf GmAmCm AmUmGm UmCfCm UfUmCm CmUmGms UmsGm | 17 | LPA-128SS | CmsAmsGm GmAmAm GfGmAf   CfAfUm GmUmCm AmAmUm CmUmUm | 108 |
| Geno-1-063M | LPA-61AS | AmsUfsUm CmUmGf UmUmUm CmUmGm AmGfCm AfUmUm GmUmGms UmsCm | 59 | LPA-129SS | CmsAmsCm AmAmUm GfCmUf CfAfGm AmAmAm | 150 |
| | | | | | CmAmGm AmAmUm | |
| Geno-1-096M | LPA-62AS | AmsUfsGm UmAmAf CmAmUm UmCmAm GmUfCm CfUmGm GmCmGms GmsUm | 88 | LPA-130SS | CmsGmsCm CmAmGm GfAmCf UfGfAm AmUmGm UmUmAm CmAmUm | 179 |
| Geno-1-044M | LPA-63AS | AmsUfsAm CmAmGf UmCmUm UmGmUm UmCfAm GfAmAm GmGmAms GmsGm | 42 | LPA-131SS | UmsCmsCm UmUmCm UfGmAf AfCfAm AmGmAm CmUmGm UmAmUm | 133 |
| Geno-1-015M | LPA-64AS | UmsUfsCm AmUmUf CmUmCm AmAmUm AmAfCm AfAmGm GmAmGms CmsUm | 15 | LPA-132SS | CmsUmsCm CmUmUm GfUmUf AfUfUm GmAmGm AmAmUm GmAmAm | 106 |
| Geno-1-077M | LPA-65AS | AmsUfsCm AmCmAf GmUmAm GmUmCm AmAfAm AfAmGm UmUmUms UmsCm | 71 | LPA-133SS | AmsAmsAm CmUmUm UfUmUf GfAfCm UmAmCm UmGmUm GmAmUm | 162 |
| Geno-1-042M | LPA-66AS | UmsUfsGm AmUmUf CmCmAm UmCmAm CmUfGm GfAmCm AmUmUms GmsCm | 40 | LPA-134SS | AmsAmsUm GmUmCm CfAmGf UfGfAm UmGmGm AmAmUm CmAmAm | 131 |
| Geno-1-027M | LPA-67AS | UGAGCCUmCGAU AACU CUmGUdTdT | 27 | LPA-135SS | ACmAGAmGmU UA CGmAGmGm CmUmCAmdTdT | 118 |
| Geno-1-008M | LPA-68AS | AUAACUCmUGUC CAUU ACmCAdTdT | 9 | LPA-136SS | UGmGUAmAmU GG ACAmGAmGm UmUmAUmdTdT | 100 |

**Table 3 ID numbers and sequences of modified siRNAs with conjugated ligands targeting human LPA mRNA**

| Numb er of doubl e strand | Num ber of antise use strand | Antisense strand 5'→3' | Orig inal SEQ ID NO. | Num ber of sense strand | sense strand 5'→3' | Origi nal SEQ ID NO. |
|---|---|---|---|---|---|---|
| Geno-1-105M | 359A S | AmsUfsAm AmCfUmCmUmGm UmCmCm AmUfUm AfCmCm AmUmGms GmsUm | 10 | 368S S | [Gal-6]sCmsAmsUm GmGmUm AmAmUf GfGfAm CmAmGm AmGmUm UmAmUms[Gal-6] | 101 |
| Geno-1-106M | 359A S | AmsUfsAm AmCfUmCmUmGm UmCmCm AmUfUm AfCmCm AmUmGms GmsUm | 10 | 369S S | [Gal-6]s[Gal- 6]sCmAmUm GmGmUm AmAmUf GfGfAm CmAmGm AmGmUm UmAmUm[Gal-6][Gal-6] | 101 |
| Geno-1-107M | 359A S | AmsUfsAmAmCfUmC mUmGm UmCmCm AmUfUm AfCmCm AmUmGms GmsUm | 10 | 370S S | CmsAmsUm GmGmUm AmAmUf GfGfAm CmAmGm AmGmUm UmAmUms[Gal-6]s[Gal- 6]s[Gal-6] | 101 |
| Geno-1-108M | 359A S | AmsUfsAm AmCfUmCmUmGm UmCmCm AmUfUm AfCmCm AmUmGms GmsUm | 10 | 371S S | CmsAmsUm GmGmUm AmAmUf GfGfAm CmAmGm AmGmUm UmAmUms[Gal-5] | 101 |
| Geno-1-109M | 382A S | AmsUfsAmAmCmUfC mUmGm UmCmCm AmUfUm AfCmCm AmUmGms GmsUm | 10 | 396S S | CmsAmsUm GmGmUm AfAmUf GfGfAm CmAmGm AmGmUm UmAmUms[Gal-6]s[Gal- 6]s[Gal-6] | 101 |
| Geno-1-110M | 383A S | AmsUfsAm AmCmUfCmUfGf UmCmCm AmUfUm AfCmCm AmUmGms GmsUm | 10 | 396S S | CmsAmsUm GmGmUm AfAmUf GfGfAm CmAmGm AmGmUm UmAmUms[Gal-6]s[Gal- 6]s[Gal-6] | 101 |
| Geno-1-111M | 384A S | VP(U)sUfsAmAmCfU mCmUmGm UmCmCm AmUfUm AfCmCm AmUmGms GmsUm | 10 | 370S S | CmsAmsUm GmGmUm AmAmUf GfGfAm CmAmGm AmGmUm UmAmUms[Gal-6]s[Gal- 6]s[Gal-6] | 101 |
| Geno-1-112M | 385A S | AmsUfsAm AmCfUmCmUmGm UmCmCm AmUfUm AfCmCf AmUmGms GmsUm | 10 | 370S S | CmsAmsUm GmGmUm AmAmUf GfGfAm CmAmGm AmGmUm UmAmUms[Gal-6]s[Gal- 6]s[Gal-6] | 101 |
| Geno-1-113M | 386A S | AmsUfsAm AmCmUmCmUmGm UmCmCm AmUfUm AfCmCf AmUmGms GmsUm | 10 | 370S S | CmsAmsUm GmGmUm AmAmUf GfGfAm CmAmGm AmGmUm UmAmUms[Gal-6]s[Gal- 6]s[Gal-6] | 101 |
| Geno-1-114M | 387A S | AmsUfsAm AmCfUfCmUmGm UmCmCm AmUfUm AfCmCm AmUmGms GmsUm | 10 | 370S S | CmsAmsUm GmGmUm AmAmUf GfGfAm CmAmGm AmGmUm UmAmUms[Gal-6]s[Gal- 6]s[Gal-6] | 101 |
| Geno-1-115M | 388A S | AmsUfsAm AmCmUfCfUmGm UmCmCm AmUfUm AfCmCm AmUmGms GmsUm | 10 | 370S S | CmsAmsUm GmGmUm AmAmUf GfGfAm CmAmGm AmGmUm UmAmUms[Gal-6]s[Gal- 6]s[Gal-6] | 101 |
| Geno-1-116M | 389A S | AmsUfsAf AmCmUfCmUmGm UmCmCm AmUfUm AfCmCm AmUmGms GmsUm | 10 | 370S S | CmsAmsUm GmGmUm AmAmUf GfGfAm CmAmGm AmGmUm UmAmUms[Gal-6]s[Gal- 6]s[Gal-6] | 101 |
| Geno-1-117M | 359A S | AmsUfsAm AmCfUmCmUmGm UmCmCm AmUfUm AfCmCm AmUmGms GmsUm | 10 | 397S S | CmsAmsUm GmGmUm AfAmUf GfGfAf CmAmGm AmGmUm UmAmUms[Gal-6]s[Gal- 6]s[Gal-6] | 101 |
| Geno-1-118M | LPA-60AS | AmsAfsGm AmUmUf GmAmCm AmUmGm UmCfCm UfUmCm CmUmGms UmsGm | 17 | 398S S | CmsAmsGm GmAmAm GfGmAf CfAfUm GmUmCm AmAmUm CmUmUms[Gal-6]s[Gal- 6]s[Gal-6] | 108 |
| Geno-1-119M | 494A S | AmsUfsAm AmCfUm(UNA- C)UmGm UmCmCm AmUfUm AfCmCm AmUmGms GmsUm | 10 | 370S S | CmsAmsUm GmGmUm AmAmUf GfGfAm CmAmGm AmGmUm UmAmUms[Gal-6]s[Gal- 6]s[Gal-6] | 101 |
| Geno-1-120M | 498A S | AmsAfsGm AmUfUm GmAmCm AmUmGm UmCfCm UfUmCm CmUmGms UmsGm | 17 | 511S S | CmsAmsGm GmAmAm GmGmAf CfAfUm GmUmCm AmAmUm CmUmUms[Gal-6]s[Gal- 6]s[Gal-6] | 108 |
| Geno-1-1001 M | LPA-56AS | AmsUfsAm AmCmUf CmUmGm UmCmCm AmUfUm AfCmCm AmUmGms GmsUm | 10 | 330S S | CmsAmsUm GmGmUm AfAmUf GfGfAm CmAmGm AmGmUm UmAmUm[L96] | 101 |
| Geno-1-1002 M | 331A S | AmsUfsAm AfCmUf CmUfGm UfCmCf AmUfUm AfCmsCfs Gm | 184 | 332S S | CfsGmsGfUm AfAmUf GmGfAm CfAmGf AmGfUm UfAmUfs[L96] | 187 |
| Geno-1-1003 M | AM-AS | UmsCfsGmUfAmUfAm AmCmAmAmUfAmAf GmGfGmGfCmsUfsGm | 185 | AM-SS | [NAG25]sCmsAmGmCm CmCmCmUmUfAfUfUm GmUmUmAmUmAmCm Gms[invdA] | 188 |
| Geno-1-1004 M | 331A S | AmsUfsAm AfCmUf CmUfGm UfCmCf AmUfUm AfCmsCfs Gm | 186 | 365S S | [ST23sST23sST23sC6XL T]sCfsGmsGf UmAf AmUfGmGfAmCfAmGf AmGfUmUfsAmsUf | 189 |

The above Geno-1-1002M unmodified sequence is: AUAACUCUGUCCAUUACCG (SEQ ID No. 184); the corresponding unmodified sequence of the sense strand is CGGUAAUGGACAGAGUUAU (SEQ ID No. 188). The above Geno-1-1003M unmodified sequence is UCGUAUAACAAUAAGGGGCUG (SEQ ID No. 185); The corresponding unmodified sequence the sense strand is CAGCCCCUUAUUGUUAUACGA (SEQ ID No. 189). The above Geno-1-1004M unmodified sequence is: AUAACUCUGUCCAUUACCG (SEQ ID No. 186); The corresponding unmodified sequence of the sense strand is CGGUAAUGGACAGAGUUAU (SEQ ID No. 189).[Gal-5] Structural formula:

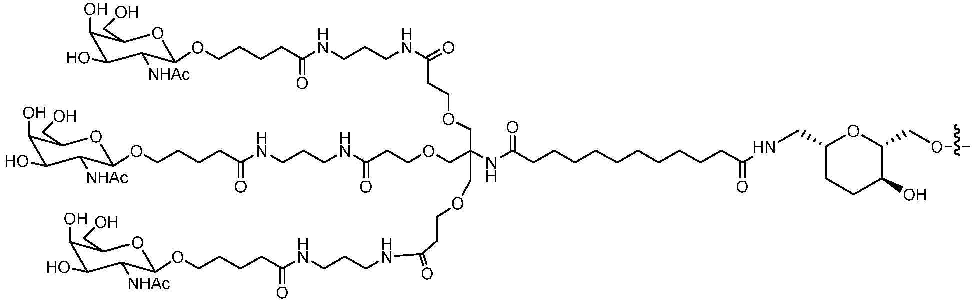

Gal-6 is shorthand for Geno-Gal-6, the target product prepared in step 5 of Example 1;

[Gal-6]s[Gal-6]s[Gal-6] Structural formula:

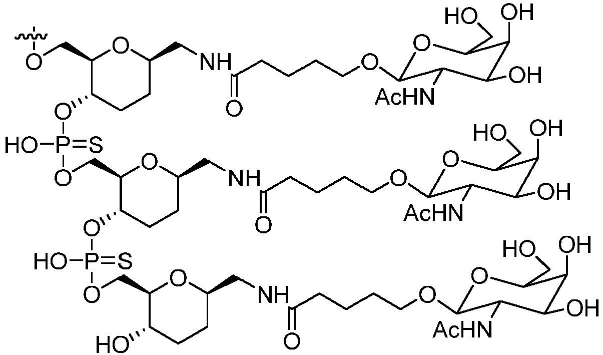

[ST23sST23sST23sC6XLT] Structural formula:

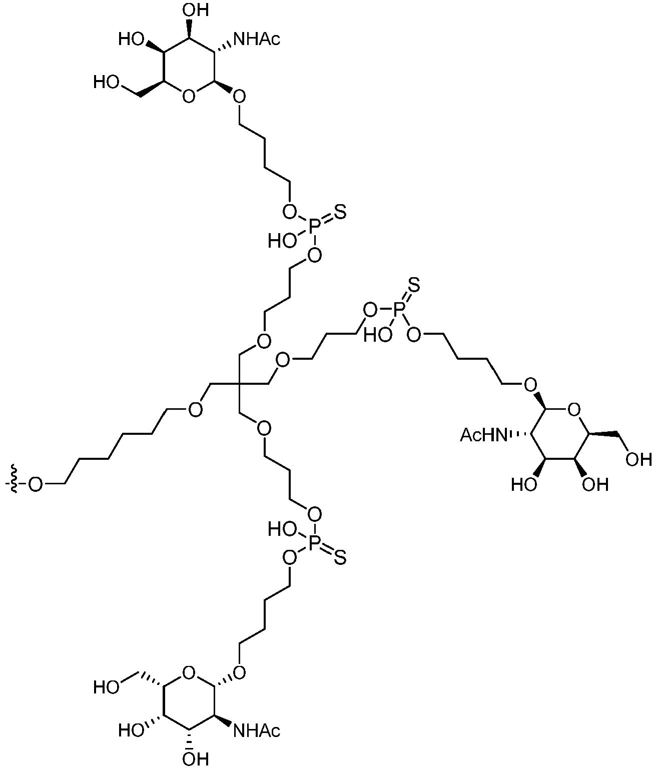

### Example 3 In vitro experiment of LPA RNAi agent

91 in silico-identifed potential LPA RNAi agents (Table 1) synthesized and screened efficacy in vitro .

For screening purposes, the human LPA cDNA sequence (accession #NM_005577.1) or its fragment was subcloned from the commercially available mammalian expression vector (Origene) into the commercially available reporter-based screening plasmid, psiCHECK2(Promega) which generated the*Renilla* luciferase /LPA fusion mRNA. For the efficacy of LPA RNAi agent in a human background, Huh7 cells (human hepatocellular carcinoma line) were plated in a 96-well format with approximately 10,000 cells/well. Each LPA RNAi agent was co-transfected with 25ng psiCHECK2-LPA plasmid DNA per well and 0.2µL LipoFectamine 2000 per well at two concentrations (1nM and 0.1nM). Gene knock-down was determined by measuring *Renilla* luciferase levels normalized to the levels of constitutively expressed firefly luciferase, also presented on the psiCHECK2 plasmid, using the double luciferase reporter assays (Promega) (Table 4).

As shown in Table 4, 66 of the 91 sequences showed a knockdown efficiency greater than 60% at 1nM; 61 sequences showed greater than 50% knock-down efficiency at 0.1nM.

**Table 4. Screening results of In vitro efficacy of LPA RNAi agents determined by double luciferase reporter assay**

| **Compound ID** | **Inhibition%** | |
|---|---|---|
| | **1nM** | **0.1nM** |
| Geno-1-001 | 78.18 | 60.57 |
| Geno-1-002 | 18.19 | 0.79 |
| Geno-1-003 | 65.09 | 55.86 |
| Geno-1-004 | 69.45 | 56.78 |
| Geno-1-005 | 76.00 | 67.92 |
| Geno-1-006 | 75.74 | 57.81 |
| Geno-1-007 | -10.00 | -9.37 |
| Geno-1-010 | 93.06 | 86.75 |
| Geno-1-011 | 86.65 | 77.88 |
| Geno-1-014 | 82.13 | 69.88 |
| Geno-1-015 | 62.17 | 40.78 |
| Geno-1-016 | -0.04 | -5.14 |
| Geno-1-017 | 71.17 | 55.74 |
| Geno-1-018 | 68.30 | 64.22 |
| Geno-1-019 | 84.44 | 81.88 |
| Geno-1-020 | 67.32 | 43.77 |
| Geno-1-021 | 77.85 | 60.29 |
| Geno-1-022 | 75.88 | 68.58 |
| Geno-1-023 | 83.04 | 80.59 |
| Geno-1-024 | 77.55 | 60.52 |
| Geno-1-025 | 51.88 | 25.06 |
| Geno-1-026 | 78.57 | 65.11 |
| Geno-1-030 | 86.03 | 80.89 |
| Geno-1-031 | 83.81 | 75.38 |
| Geno-1-035 | 86.58 | 82.78 |
| Geno-1-036 | 70.26 | 60.91 |
| Geno-1-037 | 81.46 | 74.44 |
| Geno-1-038 | 5.10 | 3.16 |
| Geno-1-039 | 77.03 | 66.79 |
| Geno-1-040 | 69.92 | 55.90 |
| Geno-1-041 | 81.97 | 73.79 |
| Geno-1-042 | 63.01 | 44.87 |
| Geno-1-043 | -6.86 | -11.04 |
| Geno-1-044 | 69.39 | 48.30 |
| Geno-1-045 | 73.01 | 61.20 |
| Geno-1-046 | 66.89 | 53.89 |
| Geno-1-050 | 72.83 | 65.84 |
| Geno-1-051 | 86.40 | 74.10 |
| Geno-1-055 | 73.37 | 66.42 |
| Geno-1-056 | -6.78 | -5.78 |
| Geno-1-057 | 74.65 | 57.12 |
| Geno-1-058 | 86.67 | 73.20 |
| Geno-1-059 | 77.75 | 70.33 |
| Geno-1-060 | 91.34 | 81.49 |
| Geno-1-061 | 90.34 | 83.21 |
| Geno-1-062 | 67.81 | 51.32 |
| Geno-1-063 | 75.74 | 64.82 |
| Geno-1-064 | 94.24 | 88.92 |
| Geno-1-065 | 11.71 | -0.21 |
| Geno-1-066 | 71.04 | 60.00 |
| Geno-1-070 | 80.00 | 67.11 |
| Geno-1-071 | 78.50 | 57.74 |
| Geno-1-075 | 71.01 | 58.21 |
| Geno-1-076 | 82.21 | 72.31 |
| Geno-1-077 | 78.06 | 60.96 |
| Geno-1-078 | 41.18 | 14.44 |
| Geno-1-079 | 63.34 | 47.73 |
| Geno-1-080 | 90.15 | 80.39 |
| Geno-1-081 | 93.02 | 86.90 |
| Geno-1-082 | 80.40 | 74.00 |
| Geno-1-083 | 5.59 | 2.06 |
| Geno-1-084 | 78.77 | 68.91 |
| Geno-1-085 | 77.73 | 66.81 |
| Geno-1-086 | 79.34 | 74.16 |
| Geno-1-090 | 69.09 | 55.55 |
| Geno-1-091 | 60.89 | 33.31 |
| Geno-1-095 | -10.72 | -9.86 |
| Geno-1-096 | 75.27 | 55.43 |
| Geno-1-097 | 77.78 | 71.38 |
| Geno-1-098 | 81.48 | 72.03 |
| Geno-1-099 | 75.57 | 60.84 |
| Geno-1-100 | 74.28 | 60.79 |
| Geno-1-027 | 84.03 | 75.13 |
| Geno-1-009 | 64.75 | 50.17 |
| Geno-1-012 | 3.07 | -7.03 |
| Geno-1-029 | 5.67 | 0.99 |
| Geno-1-032 | 7.26 | -0.51 |
| Geno-1-049 | 7.32 | 6.57 |
| Geno-1-052 | 2.96 | 4.52 |
| Geno-1-069 | 5.85 | -1.42 |
| Geno-1-072 | 83.39 | 80.69 |
| Geno-1-089 | 86.55 | 74.21 |
| Geno-1-092 | 80.11 | 69.67 |
| Geno-1-008 | 50.89 | 37.03 |
| Geno-1-034 | 5.35 | -10.01 |
| Geno-1-047 | 55.57 | 62.42 |
| Geno-1-054 | -13.83 | -13.81 |
| Geno-1-067 | -18.62 | -13.62 |
| Geno-1-074 | -12.83 | -15.93 |
| Geno-1-087 | -16.56 | -17.15 |
| Geno-1-094 | -22.42 | -19.66 |

### Example 4 Determination of EC50 of LPA RNAi agent

The same cell and transfection conditions as in Example 3 were used to generate a seven-point EC50 curve, with the LPA RNAi agents concentration range from 0.1fM to 10M. The EC₅₀ determined through the GraphPad Prism software using a dose-response curve fitted with "Sigmoidal dose-response (Variable slope)" (Table 5).

As shown in Table 5, the EC50 of the measured LPA RNAi agents ranges from 1pM to 60pM.

**Table 5. EC50 values (pM) of specified LPA RNAi agents determined in vitro**

| **LPA RNAi agent ID** | **EC50 (pM)** | **LPA RNAi agent ID** | **EC50 (pM)** |
|---|---|---|---|
| Geno-1-001 | 13.16 | Geno-1-050 | 8.102 |
| Geno-1-003 | NA | Geno-1-051 | 5.861 |
| Geno-1-004 | 19.72 | Geno-1-055 | 9.449 |
| Geno-1-005 | 10.46 | Geno-1-057 | 16.05 |
| Geno-1-006 | 17.01 | Geno-1-058 | 11.55 |
| Geno-1-010 | 3.495 | Geno-1-059 | 8.994 |
| Geno-1-011 | 12.61 | Geno-1-060 | 18.58 |
| Geno-1-014 | 10.32 | Geno-1-061 | 8.115 |
| Geno-1-015 | NA | Geno-1-062 | 55.48 |
| Geno-1-017 | NA | Geno-1-063 | 15.12 |
| Geno-1-018 | NA | Geno-1-064 | 3.104 |
| Geno-1-019 | 6.852 | Geno-1-066 | 16.99 |
| Geno-1-020 | 8.324 | Geno-1-070 | 14.51 |
| Geno-1-021 | 16.65 | Geno-1-071 | 16.84 |
| Geno-1-022 | 5.928 | Geno-1-072 | 18.87 |
| Geno-1-023 | 3.388 | Geno-1-075 | 11.3 |
| Geno-1-024 | 21.88 | Geno-1-076 | 9.823 |
| Geno-1-026 | 9.508 | Geno-1-077 | 20.88 |
| Geno-1-027 | 5.44 | Geno-1-079 | 27.85 |
| Geno-1-030 | 3.114 | Geno-1-080 | 8.466 |
| Geno-1-031 | 5.853 | Geno-1-081 | 11.46 |
| Geno-1-035 | 6.414 | Geno-1-082 | 9.531 |
| Geno-1-036 | 7.389 | Geno-1-084 | 7.988 |
| Geno-1-037 | 1.974 | Geno-1-085 | 11.92 |
| Geno-1-039 | 13.55 | Geno-1-086 | 6.002 |
| Geno-1-040 | 5.001 | Geno-1-090 | 19.78 |
| Geno-1-041 | 4.361 | Geno-1-096 | 39.39 |
| Geno-1-042 | 26 | Geno-1-097 | 10.19 |
| Geno-1-044 | 8.591 | Geno-1-098 | 4.67 |
| Geno-1-045 | 3.016 | Geno-1-099 | 15.63 |
| Geno-1-046 | 27.33 | Geno-1-100 | 38.62 |

### Example 5 Analysis of in vitro test results of modified LPA RNAi agent

After preliminary screening, LPA RNAi agents with modified sequence were synthesized., Each LPA RNAi agent was co-transfected with 25ng psiCHECK2-LPA plasmid DNA per well and 0.2µL LipoFectamine 2000 per well at a concentration of 1nM using the same cell and transfection conditions as in Example 3. Gene knock-down was determined by measuring *Renilla* luciferase levels normalized to the level of constitutively expressed firefly luciferase also presented on the psiCHECK2 plasmid using the double luciferase reporter assays (Promega, Madison, WI) (Table 6).

As shown in Table 6, 41 of the 71 sequences showed greater than 60% knock-down efficiency.

**Table 6. Screening results of in vitro efficacy of LPA RNAi agents determined by double luciferase reporter assay**

| **Compound ID** | **Inhibition % (1nM)** |
|---|---|
| Geno-1-001M | 58.00 |
| Geno-1-003M | 47.13 |
| Geno-1-004M | 71.11 |
| Geno-1-005M | 52.21 |
| Geno-1-006M | 70.00 |
| Geno-1-009M | 89.39 |
| Geno-1-010M | 78.45 |
| Geno-1-011M | 84.58 |
| Geno-1-014M | 80.29 |
| Geno-1-015M | 73.55 |
| Geno-1-017M | 71.98 |
| Geno-1-018M | 67.59 |
| Geno-1-019M | 61.85 |
| Geno-1-020M | 61.80 |
| Geno-1-021M | 44.08 |
| Geno-1-022M | 62.18 |
| Geno-1-023M | 32.89 |
| Geno-1-024M | 55.27 |
| Geno-1-026M | 73.35 |
| Geno-1-030M | 84.39 |
| Geno-1-031M | 56.31 |
| Geno-1-035M | 68.26 |
| Geno-1-036M | 64.07 |
| Geno-1-037M | 52.63 |
| Geno-1-039M | 72.51 |
| Geno-1-040M | 58.20 |
| Geno-1-041M | 54.25 |
| Geno-1-042M | 65.20 |
| Geno-1-044M | 65.44 |
| Geno-1-045M | 33.35 |
| Geno-1-046M | 75.78 |
| Geno-1-047M | 3.80 |
| Geno-1-050M | 55.11 |
| Geno-1-051M | 61.79 |
| Geno-1-055M | 55.97 |
| Geno-1-057M | 54.52 |
| Geno-1-058M | 60.63 |
| Geno-1-059M | 64.08 |
| Geno-1-060M | 47.53 |
| Geno-1-061M | 56.16 |
| Geno-1-062M | 80.93 |
| Geno-1-063M | 71.69 |
| Geno-1-064M | 69.74 |
| Geno-1-066M | 65.90 |
| Geno-1-070M | 71.81 |
| Geno-1-071M | 55.91 |
| Geno-1-072M | -10.82 |
| Geno-1-075M | 61.53 |
| Geno-1-076M | 53.98 |
| Geno-1-077M | 79.55 |
| Geno-1-079M | 29.32 |
| Geno-1-080M | 65.69 |
| Geno-1-081M | 75.32 |
| Geno-1-082M | 42.32 |
| Geno-1-084M | 50.80 |
| Geno-1-085M | 51.56 |
| Geno-1-086M | 71.99 |
| Geno-1-089M | 7.90 |
| Geno-1-090M | 61.57 |
| Geno-1-091M | 52.38 |
| Geno-1-092M | 1.15 |
| Geno-1-096M | 45.30 |
| Geno-1-097M | 62.95 |
| Geno-1-098M | 57.05 |
| Geno-1-099M | 62.93 |
| Geno-1-100M | 66.52 |
| Geno-1-009M | 80.82 |
| Geno-1-072M | 62.73 |
| Geno-1-089M | 53.75 |
| Geno-1-092M | 62.58 |
| Geno-1-047M | 66.07 |

### Example 6 Screening of unconjugated LPA RNAi agents by inhibiting expression of LPA mRNA in human RT-4 cells

Each LPA RNAi agent was incubated at 50nM with 0.4µL RNAiMax (Invitrogen) per well. RT-4 cells (human bladder transitional cell papilloma cell line) were plated in a 24-well format with approximately 70,000 cells/well and were inoculated on the incubated transfection complex. Total RNA was extracted 24 hours after transfection using the RNeasy 96 kit (Qiagen). Using Vazyme cDNA reverse transcription kit and qPCR kit, cDNA synthesis and real-time PCR were performed to detect mRNA expression level of housekeeping gene GAPDH and LPA in the corresponding sample (Table 7).

**Table 7. Screening results of in vitro efficacy of LPA RNAi agents determined by qRT-PCR**

| **Compound ID** | **Inhibition % (50 nM)** |
|---|---|
| Geno-1-004M | -60.71 |
| Geno-1-006M | -51.12 |
| Geno-1-009M | 46.29 |
| Geno-1-010M | -0.08 |
| Geno-1-011M | 44.31 |
| Geno-1-014M | 2.73 |
| Geno-1-015M | 22.70 |
| Geno-1-017M | 14.12 |
| Geno-1-026M | -91.64 |
| Geno-1-030M | 13.43 |
| Geno-1-039M | 23.94 |
| Geno-1-046M | 1.06 |
| Geno-1-062M | 25.41 |
| Geno-1-063M | -11.46 |
| Geno-1-064M | 1.71 |
| Geno-1-070M | -18.22 |
| Geno-1-077M | -108.01 |
| Geno-1-081M | -45.73 |
| Geno-1-086M | 11.98 |
| Geno-1-092M | 6.23 |

### Example 7 Distribution proportion of conjugated LPA RNAi agents in the liver and kidney of wild-type mice after subcutaneous administration

30 C57BL6/J mice were divided into 10 groups, and the designated 5 groups of LPA RNAi agents were injected subcutaneously at a dose of 10mg/kg on the first day for each group of mice. Liver and kidney were collected at 1 hour or 24h post administration in respective five groups,. The collected tissues samples were quickly frozen and transferred to -80 °C for storage. After tissues homogenization, the concentrations of LPA RNAi agents in liver tissue homogenate and kidney tissue homogenate were measured by hybridization enzyme-linked immunosorbent (ELISA) assay. The concentration of liver tissue homogenate was divided by the concentration of kidney tissue homogenate to calculate the liver-to-kidney ratio. The results are shown in Table 8.

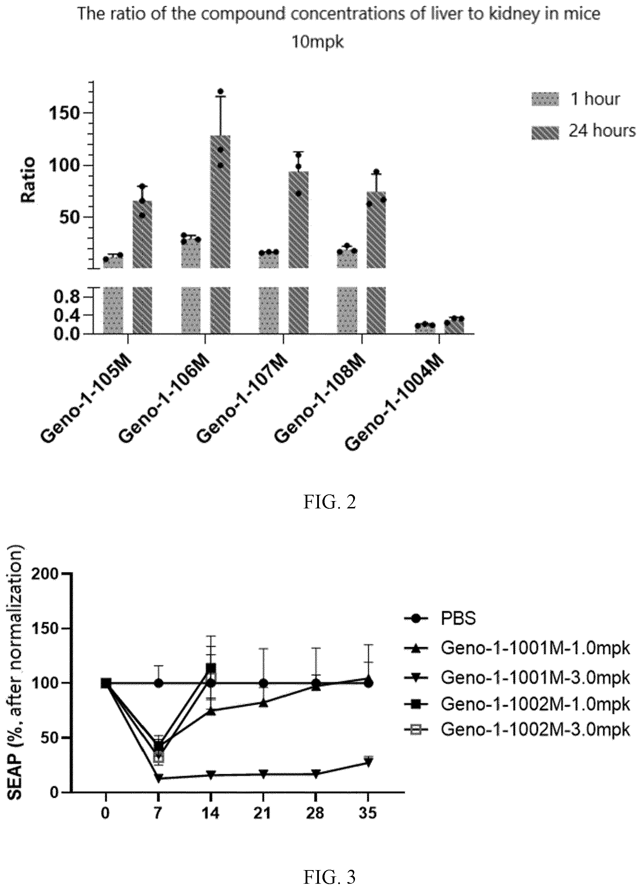

FIG. 2 shows the ratio of the compound concentrations of liver to kidney in wild-type mice after 10mg/kg LPA RNAi administered by SC. The results showed that the concentration of Geno-1-105M~Geno-1-108M in the liver (targeted organ) was much higher than that in the kidney (nontargeted organ) at different time points, and the ratio of drug concentration in liver to kidney was much greater than 1. At the same time, the concentrations of Geno-1-1004M at two time points in the kidney was greater than that in the liver, and the ratio of drug concentration in liver to kidney was less than 1.

**Table 8 Ratio of compounds concentrations of liver and kidney in wild-type mice after 10mg/kg LPA RNAi agent was administered by SC**

| | Ratio of compounds concentrations of liver and kidney of 1 hour | SD% | Ratio of compounds concentrations of liver and kidney of 24 hours | SD% |
|---|---|---|---|---|
| Geno-1-105M | 12.1 | 21% | 65.9 | 30% |
| Geno-1-106M | 29.5 | 14% | 128.6 | 39% |
| Geno-1-107M | 16.5 | 1% | 94.2 | 8% |
| Geno-1-108M | 19.3 | 24% | 74.8 | 25% |
| Geno-1-1004M | 0.2 | 13% | 0.3 | 21% |

### Example 8 In Vivo detection of efficacy of LPA RNAi agents in C57 transient transgenic mice

Two weeks before the LPA RNAi agents was administered, wild-type mice (male, C57 BL/6) were injected with a plasmid containing the SEAP gene under the control of a mouse albumin promoter (this plasmid contains a clone of the human LPA gene target sequence) by hydrodynamic tail-vein injection, which were called SEAP-LPA-HDI mice. On day 0, SEAP-LPA-HDI mice were subcutaneously injected with 1mg/Kg or 3mg/Kg of Geno-1-1001M or Geno-1-1002M solution of LPA RNAi of 5ml/Kg, and mice of the control group were given an equal volume of phosphate buffer (PBS). Mouse serum was collected at 14 days before administration (Day-14), the day of administration (Day0), and days 7, 14, 21, 28, and 35 post administration (only Geno-1-1001M group was collected at days 21-35).

Knock-down of LPA was evaluated by detecting SEAP protein levels in mouse serum detected by the chemiluminescent reporter gene system Phospha-LightTM (Life Technologies). For normalization, the SEAP level of each animal at a given point was divided by the pre-treatment level of expression in that animal to determine the ratio of expression "normalized to pre-treatment"; Expression at a specific time point was then normalized to the expression of the control group by dividing the ratio of "normalized to pre-treatment" for each animal by the average ratio for of "normalized to preconditioning" of all mice in the control group. This resulted in expression for each time point normalized to that in the control group.

FIG. 3 shows the results of detection for this example.

The conjugated LPA RNAi agents were administered to the SEAP-LPA-HDI mice as described above. Each mouse received a single subcutaneous injection (SC) dose of 1mg/kg or 3 mg/kg Geno-1-1001M or Geno-1-1002M solutions of LPA RNAi agents, and the levels of SEAP protein in serum were monitored for up to 35 days. Knock-down levels and response duration are shown in Table 9. The levels of SEAP protein showed the maximum knock-down of ≥ 57% and ≥67% on the seventh day post administration of 1mg/kg and 3 mg/kg Geno-1-1002M of LPA RNAi agents, and had recovered to the level of the control group on the 14^{th} day post administration, without showing knock-down effect. The levels of SEAP protein showed maximum knockdowns of ≥ 87% and ≥57% on the seventh day post administration of 1mg/kg and 3 mg/kg Geno-1-1001M of LPA RNAi agents, and still showed knockdowns of ≥ 72% to the 35th day post the administration of 3 mg/kg Geno-1-1001M of LPA RNAi agents. The knock-down effect and duration of Geno-1-1001M of LPA RNAi agents on SEAP protein level were significantly better than that of Geno-1-1002M of LPA RNAi agents at the same dose.

**Table 9 Relative levels of SEAP in serum of mice following subcutaneous administration of conjugated LPA RNAi agents at different doses**

| Treatment | Day0 | | Day7 | | Day14 | | Day21 | | Day2 8 | | Day3 5 | |
|---|---|---|---|---|---|---|---|---|---|---|---|---|
| | Mean value | SD | Mean value | SD | Mean value | SD | Mean value | SD | Mean value | SD | Mean value | SD |
| PBS | 1.00 | 0.00 | 1.00 | 0.39 | 1.00 | 0.64 | 1.00 | 0.78 | 1.00 | 0.79 | 1.00 | 0.86 |
| Geno-1-1001M-1.0mpk | 1.00 | 0.00 | 0.42 | 0.16 | 0.75 | 0.28 | 0.82 | 0.33 | 0.97 | 0.25 | 1.04 | 0.36 |
| Geno-1-1001M-3.0mpk | 1.00 | 0.00 | 0.13 | 0.07 | 0.16 | 0.08 | 0.17 | 0.09 | 0.17 | 0.08 | 0.27 | 0.15 |
| Geno-1-1002M-1.0mpk | 1.00 | 0.00 | 0.43 | 0.23 | 1.14 | 0.71 | / | / | / | / | / | / |
| Geno-1-1002M-3.0mpk | 1.00 | 0.00 | 0.32 | 0.17 | 1.05 | 0.71 | / | / | / | / | / | / |

### Example 9 In Vivo detection of the efficacy of LPA RNAi agents in NOD SCID transient transgenic mice

Mini-Circle DNA (MC hLPA) containing clones of human LPA gene target sequence was injected into NOD SCID mice (males) by hydrodynamic tail-vein injection 10 days before the LPA RNAi agent was administered, these mice were called MC hLPA HDI mice. On day 0, these mice were subcutaneously injected with 0.3mg/Kg,1mg/Kg, 3mg/Kg, or 9mg/kg LPA RNAi agents (Geno-1-107M, Geno-1-1003M, and Geno-1-1004M) solutions at volume of 5ml/Kg, and mice of the control group was given an equal volume of phosphate buffer (PBS). Mouse serum was collected 3 days before RNAi administration (day-3), on the Day of administration (Day0), and on days 4, 7, 14, 21, 28, and 35 post administration.

The levels of serum Apo(a) protein in mice was detected by ELISA (Apo(a), Abeam) to evaluate the knockdown of LPA expression. For normalization, the Apo(a) level of each animal at a given point in time is divided by the pre-treatment level (Day0) in that animal to determine the ratio of expression of "normalized to pre-treatment". Expression at a specific time point was then normalized to the expression of the control group by dividing the ratio of "normalized to pre-treatment" for each animal by the average ratio for of "normalized to pre-treatment" of all mice in the control group. This resulted in expression for each time point normalized to that in the control group.

FIG. 3 shows the results of detection for this example.

The conjugated LPA RNAi agents were administered to MC hLPA HDI mice as described above. Each mouse received a single subcutaneous injection (SC) dose of 0.3mg/Kg, 1mg/Kg, 3mg/Kg, or 9mg/kg LPA RNAi agent (Geno-1-107M, Geno-1-1003M, and Geno-1-1004M) solutions. The levels of Apo(a) protein in serum were monitored for up to 35 days. Knock-down levels and response duration are shown in Table 10. The levels of Apo(a) protein showed maximum knockdowns on day 7 post administration of the 0.3mg/Kg, 1mg/Kg, or 3mg/Kg LPA RNAi agent (Geno-1-1004M) solution, was 33%, 58%, and 79%, respectively. At day 28 post administration, 0.3mg/Kg, 1mg/Kg or 3mg/Kg LPA RNAi agents (Geno-1-1004M) knocked down the levels of Apo(a) proteinwas 20%, 42% and 45%, respectively. The 3mg/Kg LPA RNAi agent (Geno-1-1004M) showed knockdowns of the levels of Apo(a) protein of ≥ 20% continuing to the 35^{th} day post administration. The levels of Apo(a) protein showed maximum knockdowns on day 7 post administration of either 1mg/Kg or 3mg/Kg LPA RNAi agent (Geno-1-1003M) solutions, was 79% and 92%, respectively. At day 28 post administration, 1mg/Kg or 3mg/Kg LPA RNAi agents (Geno-1-1003M) knocked down the levels of Apo(a) protein was 52% and 90%, respectively. The 3mg/Kg LPA RNAi agent (Geno-1-1003M) showed knockdowns of the levels of Apo(a) protein of ≥ 83% continuing to the 35^{th} day post the administration. The levels of Apo(a) protein showed maximum knockdowns on day 7 post administration of 0.3mg/Kg, 1mg/Kg, 3mg/Kg or 9mg/kg LPA RNAi (Geno-1-107M) solutions, was45%, 71%, 96% and 99%, respectively. At day 28 post administration, 0.3mg/Kg, 1mg/Kg, 3mg/Kg or 9mg/kg LPA RNAi agents (Geno-1-107M) knocked down the levels of Apo(a) protein was 33%, 45%, 88% and 98%, respectively. In addition, 3mg/Kg Geno-1-107M of LPA RNAi agent showed knockdowns of the levels of Apo(a) protein of ≥ 74% continuing to the 35^{th} day post the administration. The 9 mg/kg Geno-1-107M of LPA RNAi agent also showed knockdown of the level of Apo(a) protein of ≥ 97% continuing to the 35^{th} day post the administration. After the same dose of 3mg/kg LPA RNAi agent was administrated, the knockdown effect of Geno-1-107M on the level of Apo(a) protein was significantly better than that of Geno-1-1004M at the same time point, and was equivalent to that of Geno-1-1003M.

**Table 10 Relative levels of Apo(a) in serum of mice following subcutaneous administration of conjugated LPA RNAi agents at different doses**

| treatment | Day0 | | Day4 | | Day7 | | Day14 | | Day21 | | Day28 | | Day35 | |
|---|---|---|---|---|---|---|---|---|---|---|---|---|---|---|
| | Mean value | SD | Mean value | SD | Mean value | SD | Mean value | SD | Mean value | SD | Mean value | SD | Mean value | SD |
| PBS | 1.00 | 0.00 | 1.00 | 0.22 | 1.00 | 0.34 | 1.00 | 0.32 | 1.00 | 0.44 | 1.00 | 0.29 | 1.00 | 0.29 |
| Geno-1-107M 0.3mpk | 00 | 0.00 | 0.56 | 0.11 | 0.55 | 0.18 | 0.55 | 0.12 | 0.61 | 0.21 | 0.67 | 0.08 | 0.93 | 0.22 |
| Geno-1-107M 1mpk | 1.00 | 0.00 | 0.35 | 0.13 | 0.29 | 0.12 | 0.33 | 0.18 | 0.35 | 0.26 | 0.55 | 0.29 | 0.75 | 0.44 |
| Geno-1-107M 3mpk | 1.00 | 0.00 | 0.06 | 0.06 | 0.04 | 0.02 | 0.04 | 0.02 | 0.07 | 0.03 | 0.12 | 0.05 | 0.26 | 0.09 |
| Geno-1-107M 9mpk | 1.00 | 0.00 | 0.02 | 0.01 | 0.01 | 0.01 | 0.02 | 0.03 | 0.04 | 0.02 | 0.02 | 0.03 | 0.03 | 0.05 |
| Geno-1-1004M 0.3mpk | 1.00 | 0.00 | 0.85 | 0.15 | 0.67 | 0.08 | 0.81 | 0.11 | 0.72 | 0.20 | 0.80 | 0.26 | 1.20 | 0.27 |
| Geno-1-1004M 1mpk | 1.00 | 0.00 | 0.52 | 0.0 | 0.42 | 0.11 | 0.47 | 0.12 | 0.42 | 0.10 | 0.58 | 0.11 | 0.70 | 0.15 |
| Geno-1-1004M 3mpk | 1.00 | 0.00 | 0.23 | 0.2 | 0.21 | 0.10 | 0.23 | 0.11 | 0.28 | 0.06 | 0.55 | 0.15 | 0.80 | 0.20 |
| Geno-1-1003M 1mpk | 1.00 | 0.00 | 0.40 | 0.06 | 0.21 | 0.05 | 0.21 | 0.03 | 0.25 | 0.09 | 0.48 | 0.15 | 0.61 | 0.16 |
| Geno-1-1003M 3mpk | 1.00 | 0.00 | 0.17 | 0.05 | 0.08 | 0.01 | 0.04 | 0.03 | 0.07 | 0.03 | 0.10 | 0.03 | 0.17 | 0.09 |

### Example 10 In Vivo detection of efficacy of LPA RNAi agents in NOD SCID transient transgenic mice (Single dose)

Mini-Circle DNA (MC hLPA) containing clones of LPA gene target sequence was injected into NOD SCID mice (male) by hydrodynamic tail-vein injection 10 days before administration of LPA RNAi agent. These mice are called MC hLPA HDI mice. On day 0, these mice were injected subcutaneously with 1mg/Kg LPA RNAi agent solution at a volume of 5ml/Kg, and mice of the control group were given an equal volume of phosphate buffer (PBS). Mouse serum was collected 3 days before administration (day-3), on the Day of administration (DayO), and on days 4, 7, 14, 21, and 28 post administration (only some groups received samples at days 21 and 28).

The levels of Apo(a) protein in serum of mice were detected by ELISA (Apo(a), Abeam) to evaluate the knockdown of LPA expression. For normalization, the Apo(a) level of each animal at a given point in time is divided by the pre-treatment level(Day0) in that animal to determine the ratio of expression of "normalized to pre-treatment"; Expression at a specific time point was then normalized to the expression of the control group by dividing the ratio of "normalized to pre-treatment" for each animal by the average ratio for of "normalized to pre-treatment" of all mice in the control group. This resulted in expression for each time point normalized to that in the control group.

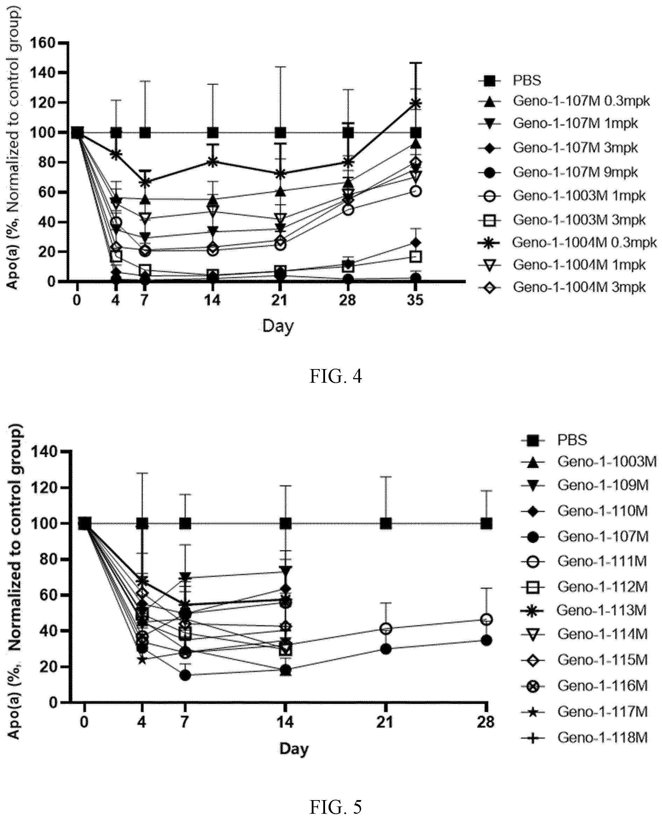

FIG. 5 shows the results of detection for this example.

The conjugated LPA RNAi agents were administered to MC hLPA HDI mice as described above. Each mouse received a single subcutaneous injection (SC) dose of 1mg/Kg LPA RNAi agent solution, the levels of Apo(a) protein in serum were monitored for up to 28 days. Knock down levels and response duration are shown in Table 11. On the 7^{th} day post administration, 10 LPA RNAi agents showed knockdowns of more than 50%; Among them, 3 LPA RNAi agents showed knockdowns of more than 70%; And Geno-1-107M showed knocked down level of more than 84%. On the 14^{th} day postadministration, 8 LPA RNAi agents showed knockdowns of more than 50%, among them, Geno-1-112M showed knockdown of more than 70%; Geno-1-107M and Geno-1-1003M showed knockdown the levels of more than 80%. On the 28^{th} day post administration, Geno-1-107M and Geo-1-111M still knocked down the levels of Apo(a) protein by 65% and 54%.

**Table 11 Relative levels of Apo(a) in serum of mice with subcutaneous administration of 1mg/Kg of conjugated LPA RNAi agents**

| Treatment | Day0 | | Day4 | | Day7 | | Day14 | | Day21 | | Day28 | |
|---|---|---|---|---|---|---|---|---|---|---|---|---|
| | Mean value | SD | Mean value | SD | Mean value | SD | Mean value | SD | Mean value | SD | Mean value | SD |
| PBS | 1.00 | 0.00 | 1.00 | 0.28 | 1.00 | 0.16 | 1.00 | 0.21 | 1.00 | 0.26 | 1.00 | 0.18 |
| Geno-1-1003M | 1.00 | 0.00 | 0.46 | 0.24 | 0.30 | 0.11 | 0.18 | 0.03 | / | / | / | / |
| Geno-1-109M | 1.00 | 0.00 | 0.50 | 0.20 | 0.70 | 0.19 | 0.73 | 0.26 | / | / | / | / |
| Geno-1-110M | 1.00 | 0.00 | 0.55 | 0.17 | 0.50 | 0.15 | 0.64 | 0.21 | / | / | / | / |
| Geno-1-107M | 1.00 | 0.00 | 0.31 | 0.08 | 0.15 | 0.06 | 0.18 | 0.06 | 0.30 | 0.10 | 0.35 | 0.11 |
| Geno-1-111M | 1.00 | 0.00 | 0.34 | 0.08 | 0.28 | 0.07 | 0.32 | 0.12 | 0.41 | 0.14 | 0.46 | 0.17 |
| Geno-1-112M | 1.00 | 0.00 | 0.49 | 0.19 | 0.39 | 0.12 | 0.30 | 0.06 | / | / | / | / |
| Geno-1-113M | 1.00 | 0.00 | 0.68 | 0.31 | 0.55 | 0.15 | 0.58 | 0.18 | / | / | / | / |
| Geno-1-114M | 1.00 | 0.00 | 0.45 | 0.15 | 0.47 | 0.15 | 0.30 | 0.12 | / | / | / | / |
| Geno-1-115M | 1.00 | 0.00 | 0.61 | 0.22 | 0.44 | 0.10 | 0.43 | 0.14 | / | / | / | / |
| Geno-1-116M | 1.00 | 0.00 | 0.37 | 0.18 | 0.50 | 0.18 | 0.56 | 0.24 | / | / | / | / |
| Geno-1-117M | 1.00 | 0.00 | 0.24 | 0.05 | 0.28 | 0.09 | 0.35 | 0.19 | / | / | / | / |
| Geno-1-118M | 1.00 | 0.00 | 0.45 | 0.23 | 0.35 | 0.12 | 0.40 | 0.21 | / | / | / | / |

### Example 11 In Vivo detection of the efficacy of LPA RNAi agents in NOD SCID transient transgenic mice

Mini-Circle DNA (MC hLPA) containing clones of human LPA gene target sequence was injected into NOD SCID mice (males) by hydrodynamic tail-vein injection 27 days before the LPA RNAi agent was administered, these mice were called MC hLPA HDI mice. On day 0, these mice were subcutaneously administrated with 1mg/Kg LPA RNAi test substance solution at a volume of 5ml/Kg, and mice of the control group were given an equal volume of phosphate buffer (PBS). Mouse serum was collected 3 days before administration (day-3), on the Day of administration (DayO), and on days 4, 11, and 18 post administration.

The levels of Apo(a) protein in serum of mice were detected by ELISA (Apo(a), Abeam) to evaluate the knockdown of LPA expression. For normalization, the Apo(a) level of each animal at a given point in time is divided by the pre-treatment level(Day0) in that animal to determine the ratio of expression of "normalized to pre-treatment "; Expression at a specific time point was then normalized to the expression of the control group by dividing the ratio of "normalized to pre-treatment " for each animal by the average ratio for of "normalized to pre-treatment" of all mice in the control group. This resulted in expression for each time point normalized to that in the control group.

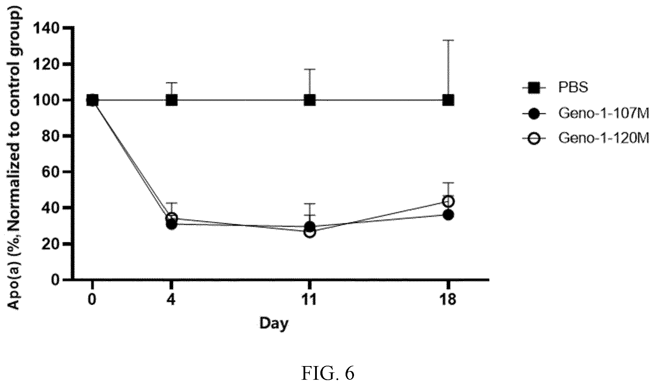

FIG. 6 shows the results of detection for this example.

The Gal-6-conjugated LPA RNAi agents were administered to MC hLPA HDI mice as described above. Each mouse received a single subcutaneous injection (SC) dose of 1mg/Kg LPA RNAi agent solution, the levels of Apo(a) protein in serum were monitored for up to 18 days. Knock down levels and response duration are shown in Table 12. On the 4^{th} day post administration, Geno-1-107M and Geno-1-120M showed knockdowns of 69% and 66%; On the 11^{th} day post administration, Geno-1-107M and Geno-1-120M of showed knockdowns of 70% and 73%. On the 18th day post administration, Geno-1-107M and Geno-1-1003M of LPA RNAi agents showed knockdowns of 64% and 56%.

**Table 12 Relative levels of Apo(a) in serum of mice after subcutaneous administration of 1mg/Kg of Gal-6-conjugated LPA RNAi agents**

| Treatment | Day0 | | Day4 | | Day11 | | Day18 | |
|---|---|---|---|---|---|---|---|---|
| | Mean value | SD | Mean value | SD | Mean value | SD | Mean value | SD |
| PBS | 1.00 | 0.00 | 1.00 | 0.10 | 1.00 | 0.17 | 1.00 | 0.33 |
| Geno-1-107M | 1.00 | 0.00 | 0.31 | 0.05 | 0.30 | 0.13 | 0.36 | 0.11 |
| Geno-1-120M | 1.00 | 0.00 | 0.34 | 0.08 | 0.27 | 0.09 | 0.44 | 0.10 |

The above is only the preferred examples of the invention and is not intended to limit the invention.nd any modification, equivalent replacement, improvement, etc. made within the principles of the invention shall be included in the scope of protection of the invention.

## Claims

1. An LPA RNA interference agent, comprising:
a first nucleotide sequence comprising at least 12 consecutive nucleotides of any one selected from the group consisting of the nucleotide sequences of SEQ ID NO: 2 to SEQ ID NO: 183 or a nucleotide sequence that is complementary thereto, or a sequence that differs not more than 3 nucleotides therefrom,
wherein each of the nucleotides is independently in a modified or unmodified state.

2. The LPA RNA interference agent of claim 1, wherein the first nucleotide sequence consists of 12 to 30 nucleotides, preferably 19 to 23 nucleotides.

3. The LPA RNA interference agent of claim 1 or 2, wherein the first nucleotide sequence is a single-stranded oligonucleotide or a double-stranded nucleotide.

4. The LPA RNA interference agent of any of claims 1 to 3, wherein one or more nucleotides of the first nucleotide sequence are modified to form modified nucleotide(s).

5. The LPA RNA interference agent of claim 4, wherein the first nucleotide sequence comprises any one of the modified nucleotide sequences listed in Table 2 or a sequence that differs not more than 3 nucleotides therefrom.

6. The LPA RNA interference agent of any of claims 1 to 5, wherein the first nucleotide sequence is a sense strand or an antisense strand.

7. The LPA RNA interference agent of any of claims 1 to 6, wherein the first nucleotide sequence comprises a double-stranded structure of an antisense strand and a sense strand.

8. The LPA RNA interference agent of claim 7, wherein the sense strand and/or antisense strand independently comprises one or more modified nucleotides.

9. The LPA RNA interference agent of claim 4 or 5, wherein the modified nucleotide is at least one selected from the group consisting of 2'-fluoro-modified nucleotide, 2'-O-methyl modified nucleotide, 2'-O-methoxyethyl modified nucleotide, 2'-O-alkyl modified nucleotide, 2'-O-allyl modified nucleotide, bicyclic nucleic acid, deoxyribonucleotide, EVP, UNA, and GNA.

10. The LPA RNA interference agent of any of claims 6 to 9, wherein the antisense strand has at least one position, selected from the group consisting of positions 2, 5, 6, 8, 10, 14, and 16 from the 5' end, has a 2' - fluoro-modified nucleotide or O-methyl modified nucleotide, and preferably, 5-7 of these positions have 2' -fluoro-modified nucleotides or O-methyl modified nucleotide.

11. The LPA RNA interference agent of any of claims 6 to 9, wherein the sense strand and/or antisense strand independently comprises one or more thiophosphate ester bonds; preferably, the sense strand comprises two consecutive thiophosphate ester bonds between the end nucleotides at the 3' and 5' ends, or the antisense strand comprises two consecutive thiophosphate ester bonds between the end nucleotides at the 3' and 5' ends.

12. The LPA RNA interference agent of claim 7, wherein the antisense strand comprises any one of the modified antisense strand nucleotide sequences in Table 2 or a nucleotide sequence differs not more than 3 nucleotides therefrom.

13. The LPA RNA interference agent of any of claims 1 to 12, wherein the RNA interfering agent also comprises a ligand.

14. The LPA RNA interference agent of claim 13, wherein the ligand comprises a targeting group, preferably, the targeting group comprises a sialoglycoprotein receptor ligand; more preferably, the sialoglycoprotein receptor ligand comprises a galactose cluster.

15. The LPA RNA interference agent of claim 13 or 14, wherein the ligand is configured to conjugate to the sense strand and/or the antisense strand, and preferably, to conjugate to the sense strand.

16. The LPA RNA interference agent of any of claims 13 to 15, wherein the ligand comprises the following structure:
Z-X (I),
wherein Z is the first connector part of the nucleotide sequence, which has a general formula of Z, as shown by (Z-1): 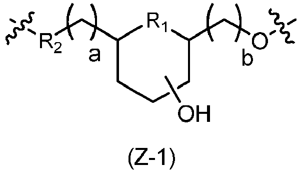
wherein R₁ is O, S, NR₃ or CR₃R₄, wherein R₃ and R₄ are independently hydrogen, halogen, substituted or unsubstituted aliphatic group, substituted or unsubstituted aryl group, substituted or unsubstituted heteraryl group, substituted or unsubstituted heterocyclic ring or substituted or unsubstituted cycloalkyl group;
wherein R₂ is -O-, -S-, -NH-, -CH₂-, -C(O)-, -OC(O)-, -C(O)O-, - NHC(O)-, -C(O)NH-, -CH₂NH-, -CH₂O-, -NH-C(O)-CH₂- and -C(O)-CH₂- NH- or -NH(CO)NH-, where the -CH₂- is optionally substituted by one selected from the group consisting of halogen, alkyl, alkoxy, and alkane amino group; The a and a are the same or different, and are selected from integers of 0 to 20 respectively, preferably selected from integers of 1 to 10.

17. The LPA RNA interference agent of claim 16, wherein the first connector part Z is connected to the second connector part L₁, and the second connector part L₁ is connected to a branch point group E.

18. The LPA RNA interference agent of claim 17, wherein the branch point group E is further connected with a of tethered parts L₂ which each is connected with the targeting part, and the *a* is an integer selected from 0 to 10, preferably an integer from 1 to 5.

19. The LPA RNA interference agent of any of claims 16 to 18, wherein the ligand has a structure as follows: 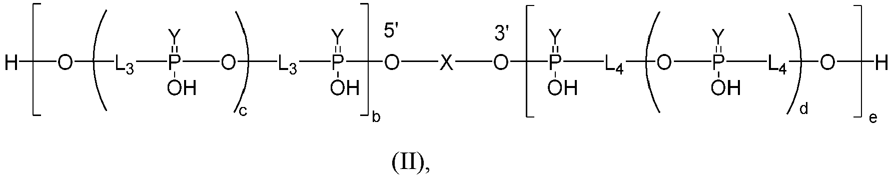 [00303] in the general formula (II):
wherein L₄ is a targeting part,
[00304] X is a nucleotide sequence,
[00305] Y is O or S,
where b, c, d, and e are integers from 0 to 10, and b and e are not both 0 at the same time.

20. The LPA RNA interference agent of any of claims 16 to 19, wherein the second connector part L₁ has a following structure: 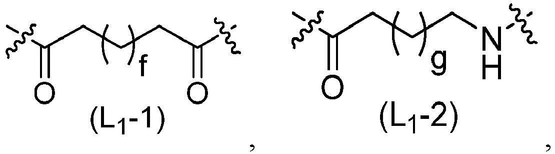 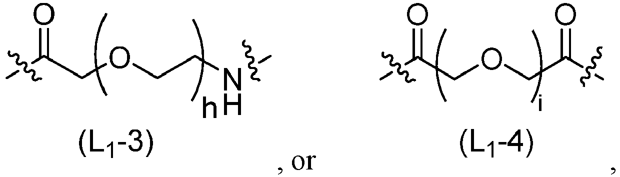 wherein f, g, h, and i are integers from 1 to 20 respectively, preferably integers from 1 to 10.

21. The LPA RNA interference agent of claim 18, wherein the tethered part L₂ has a following structure: 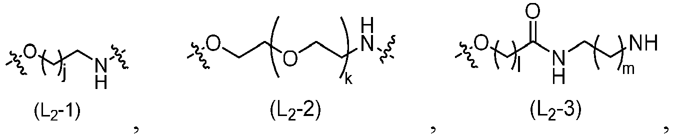 or 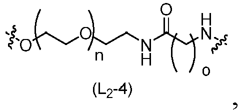 wherein j, k, 1, m, n, and o are integers from 1 to 20 respectively, preferably integers from 1 to 10.

22. The LPA RNA interference agent of any of claims 13 to 21, wherein the ligand comprises at least one of chemical formulas 1-1 to I-16: 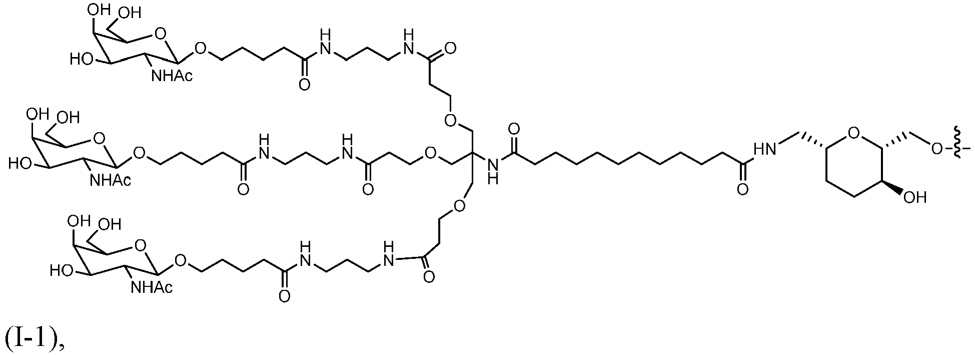 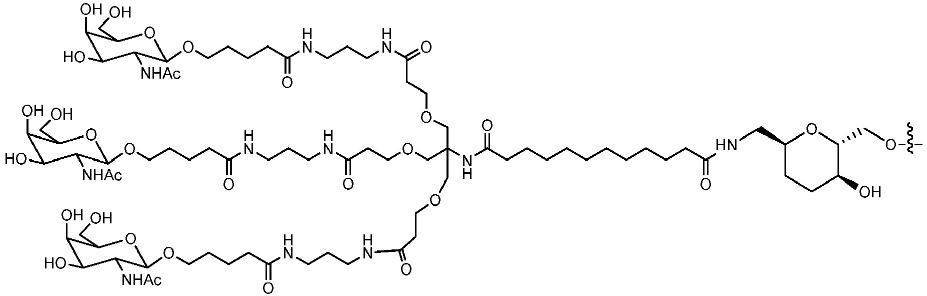 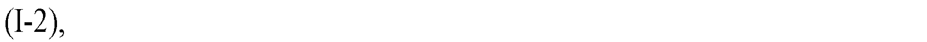 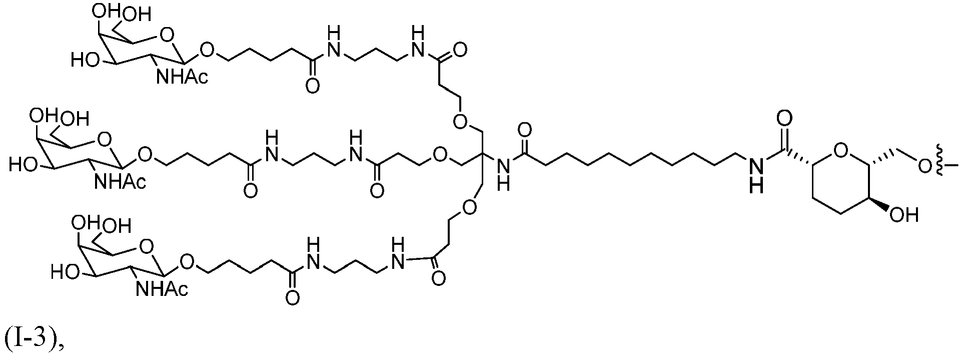 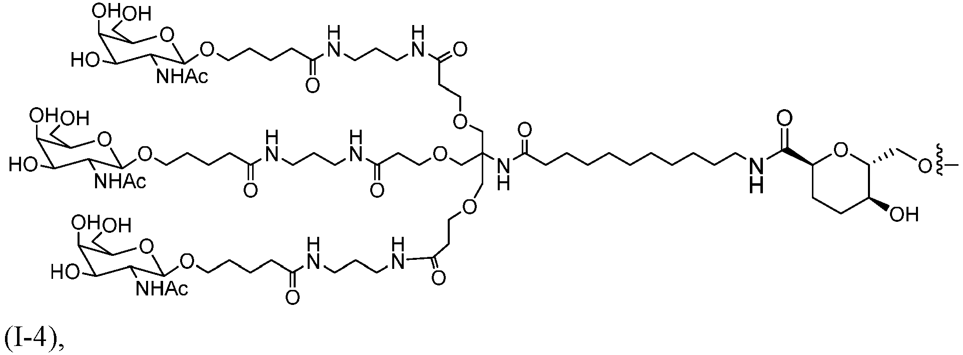 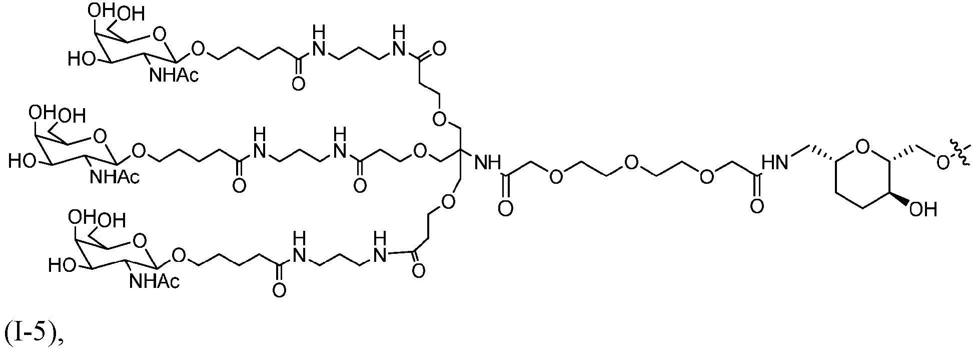 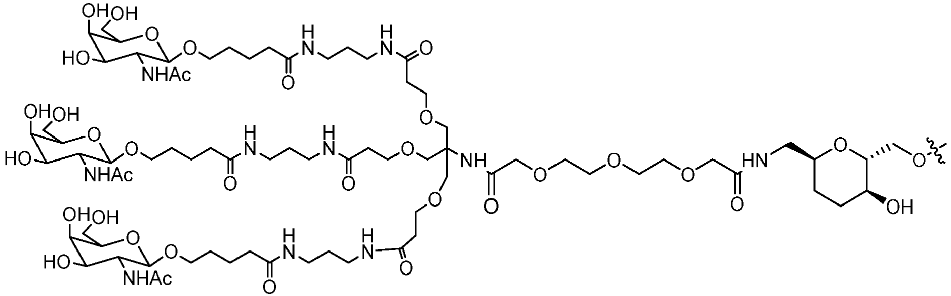 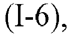 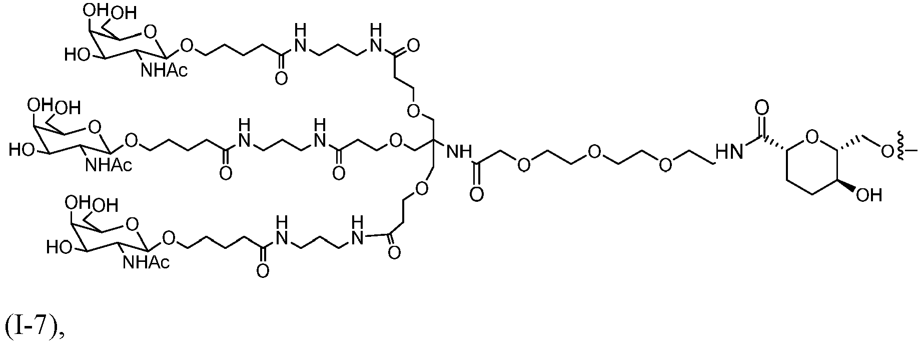 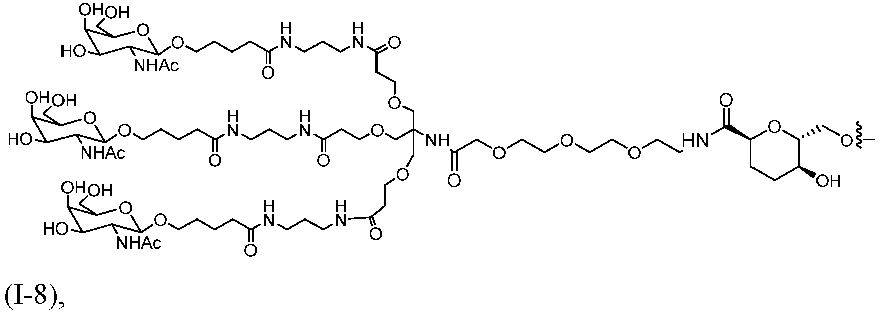 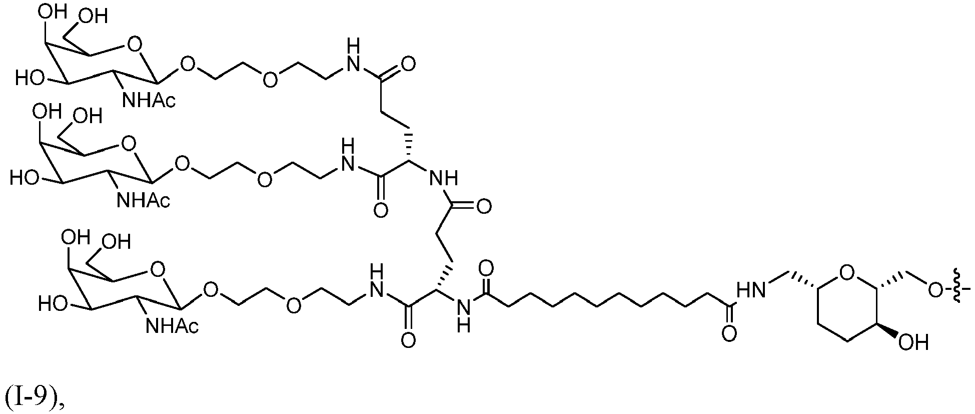 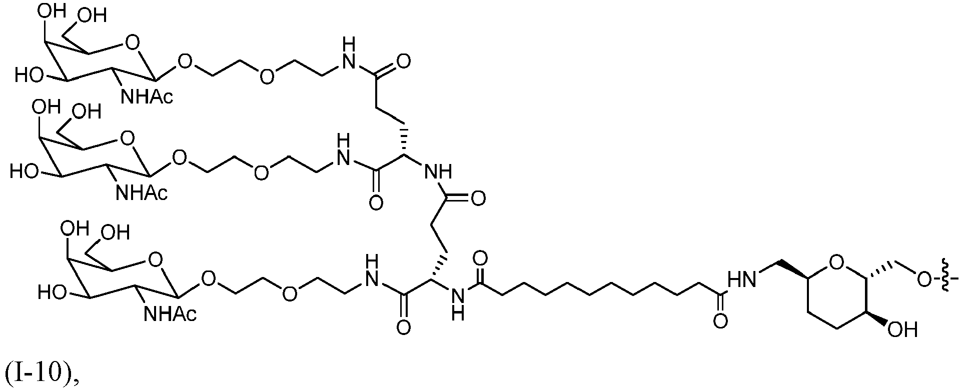 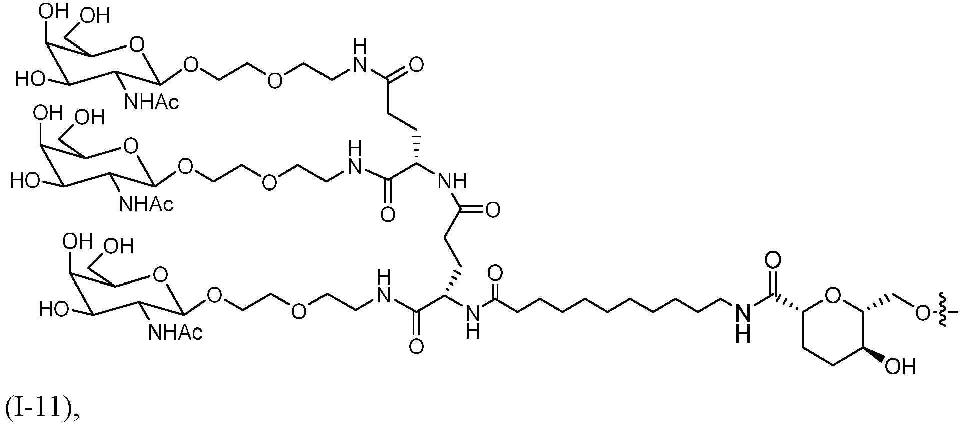 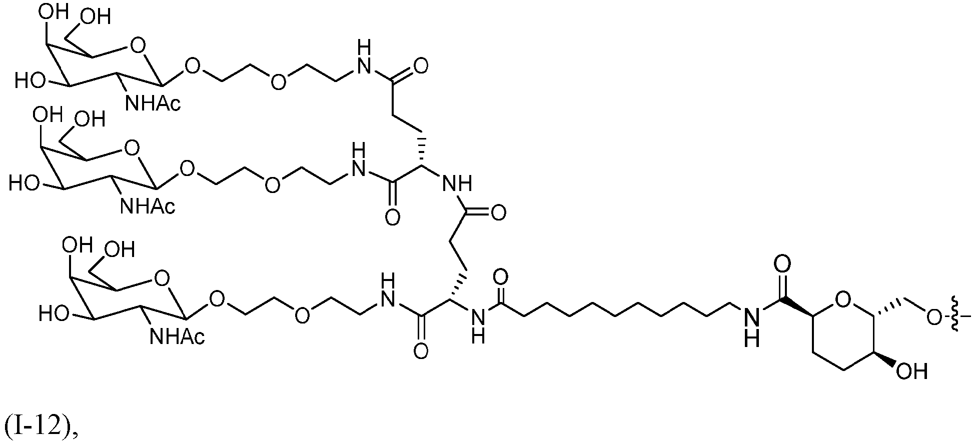 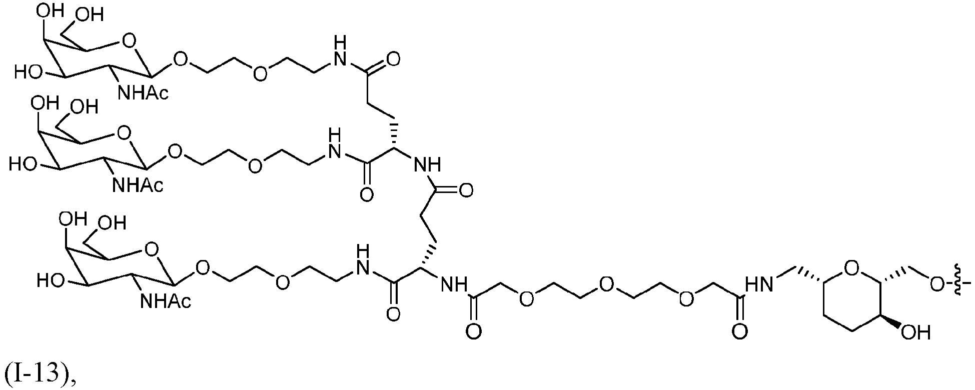 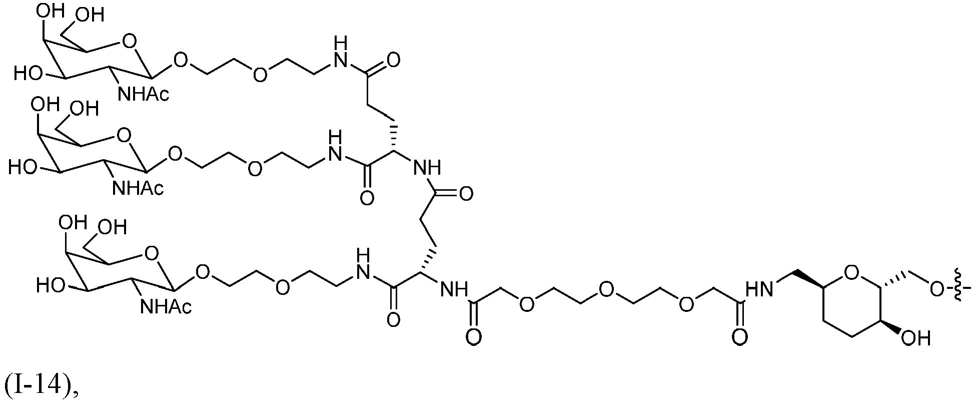 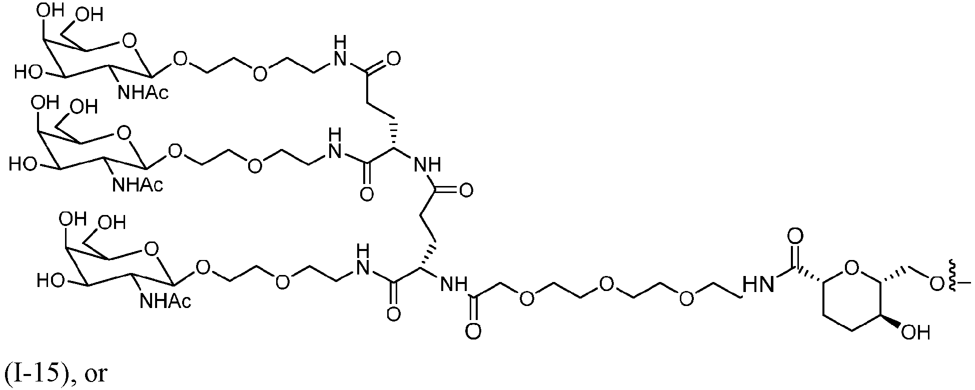 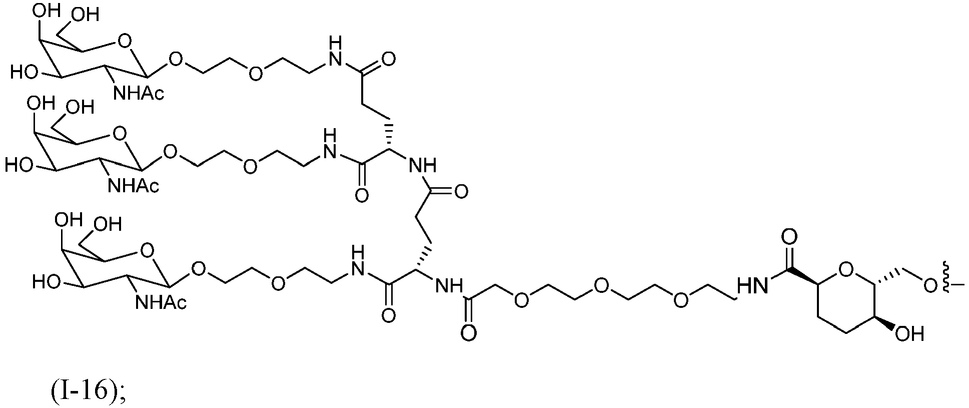 or at least one of chemical formulas II-1 to II-28: 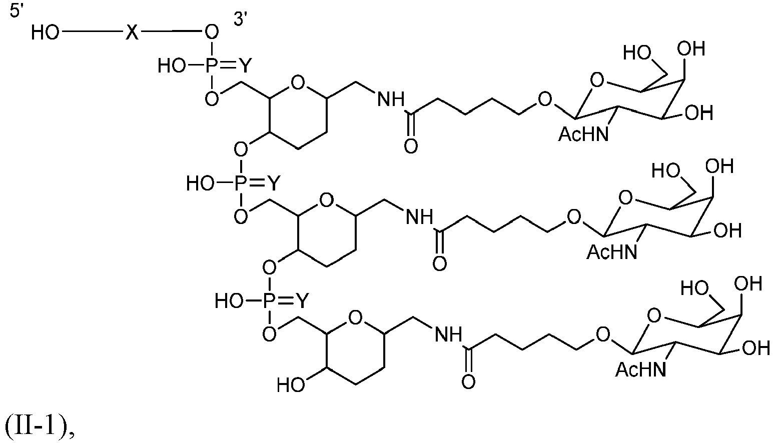 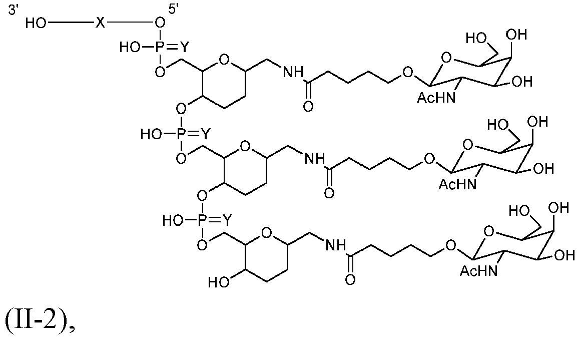 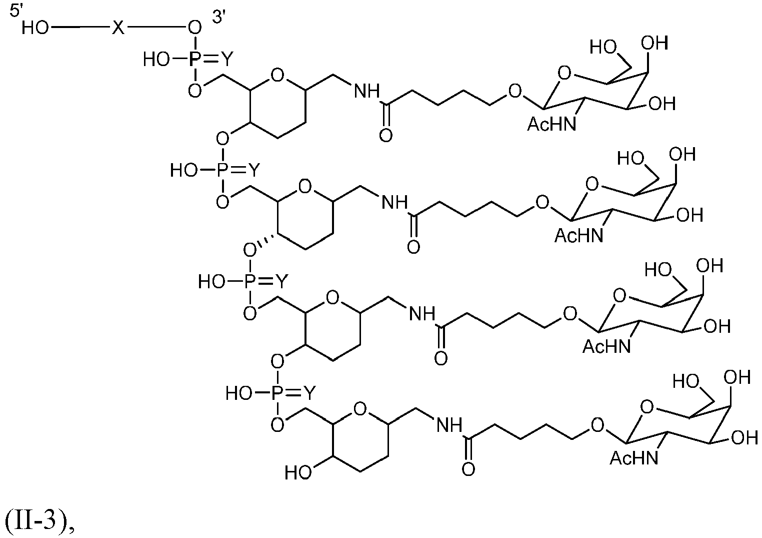 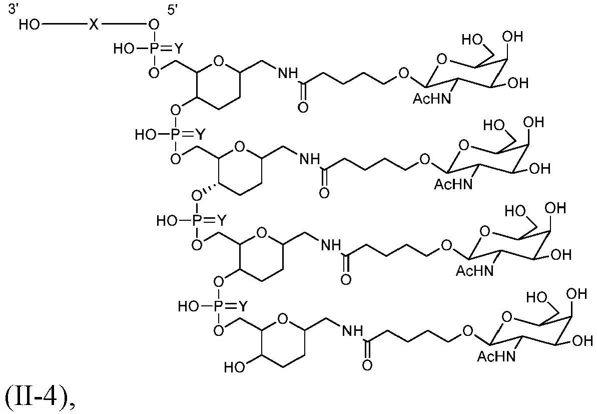 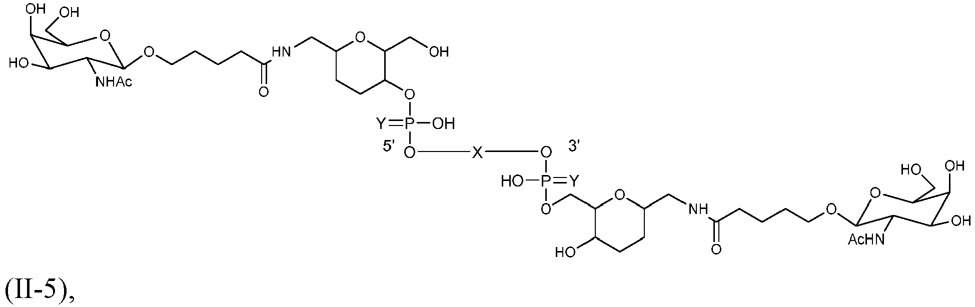 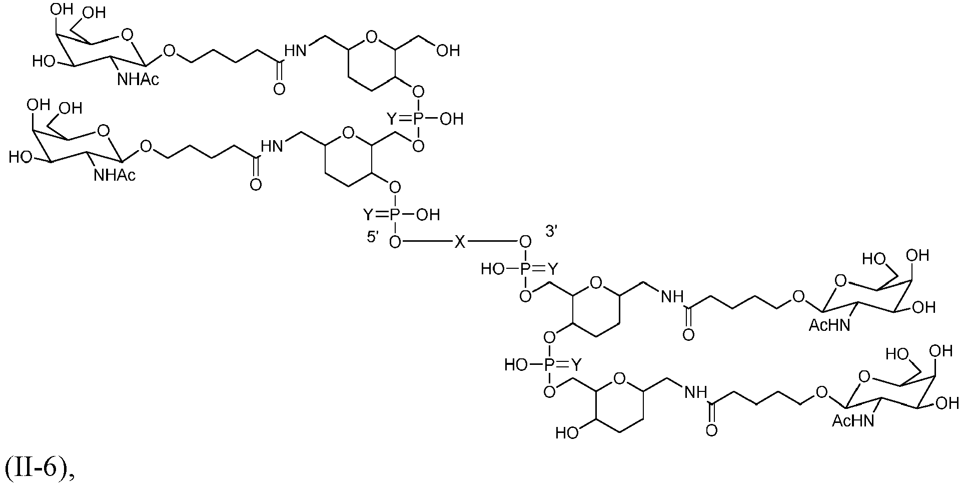 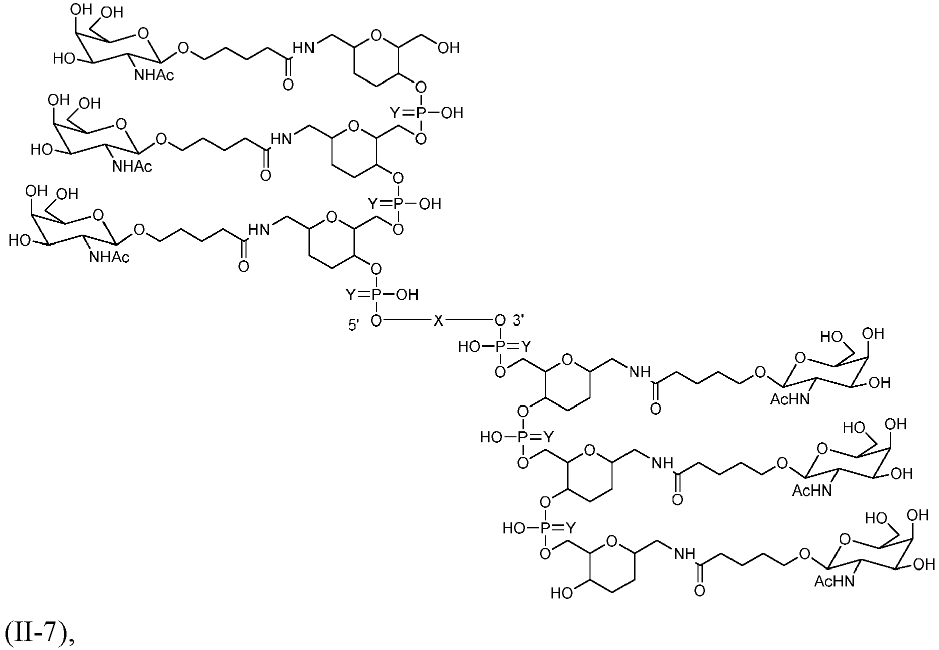 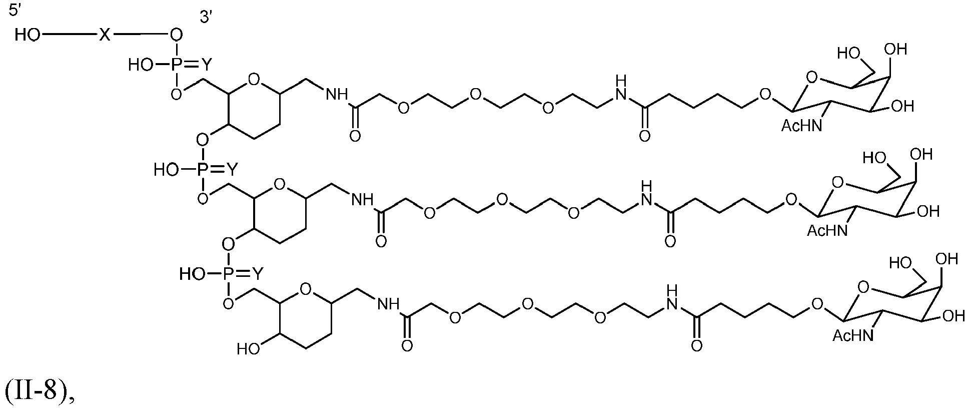 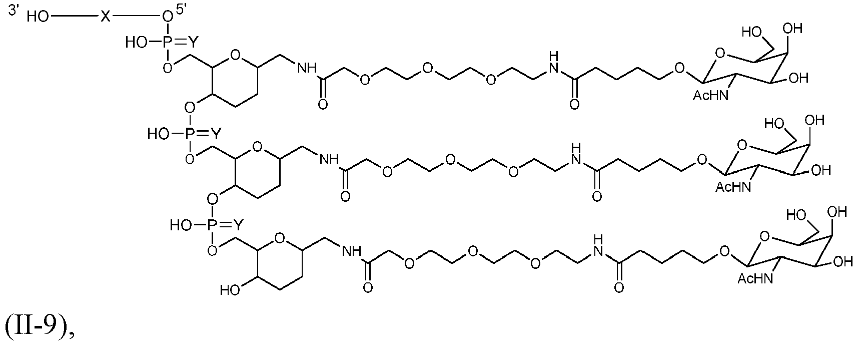 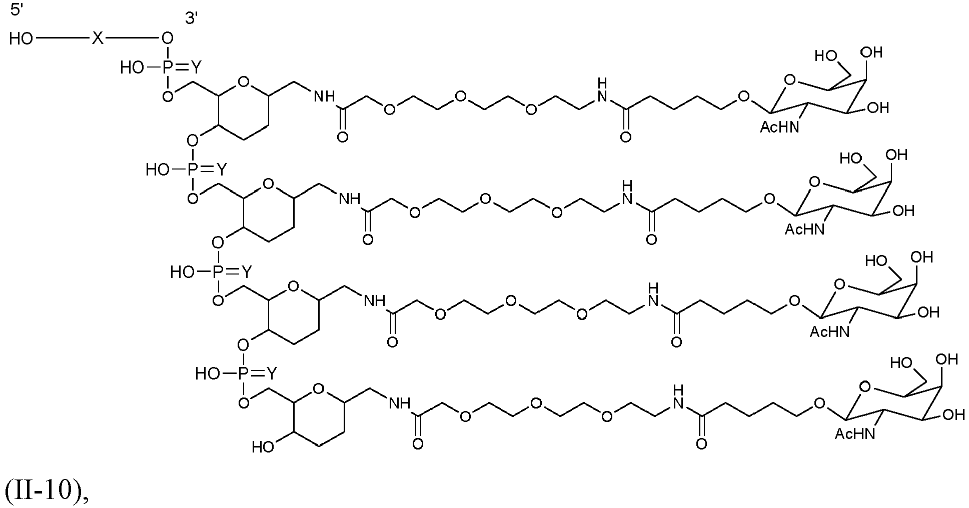 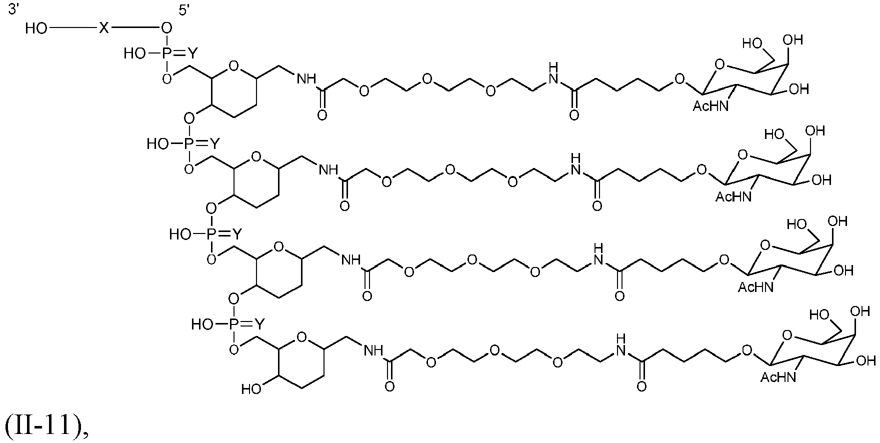 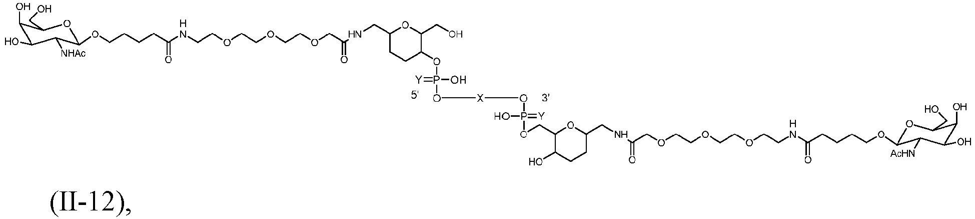 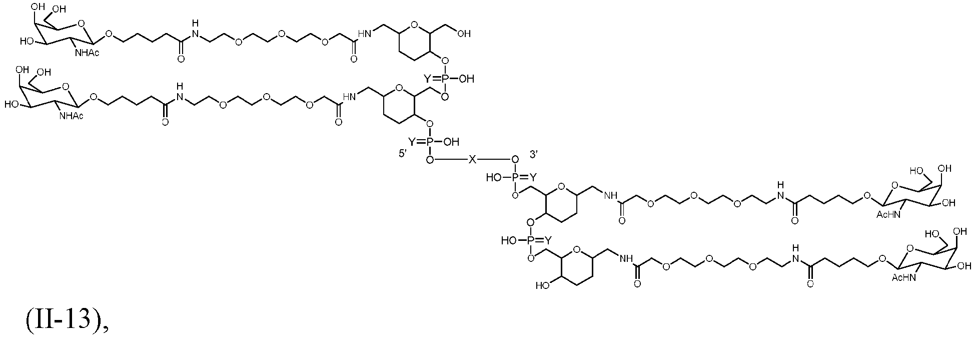 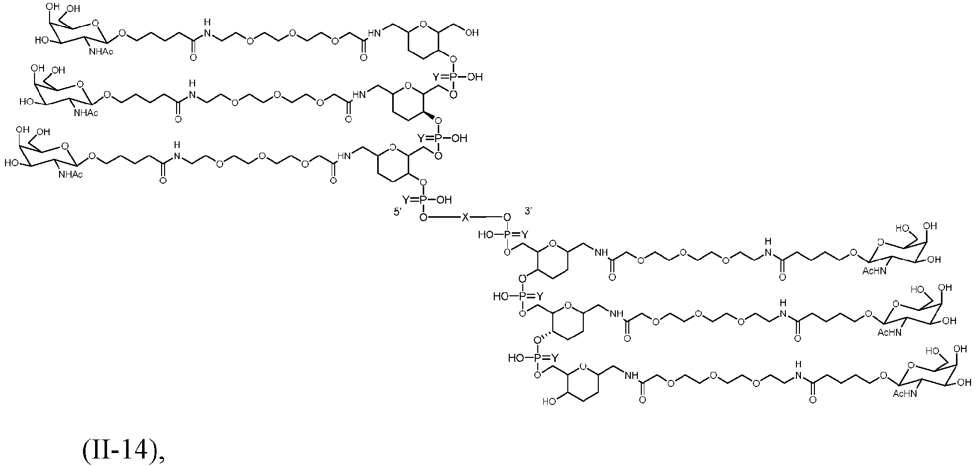 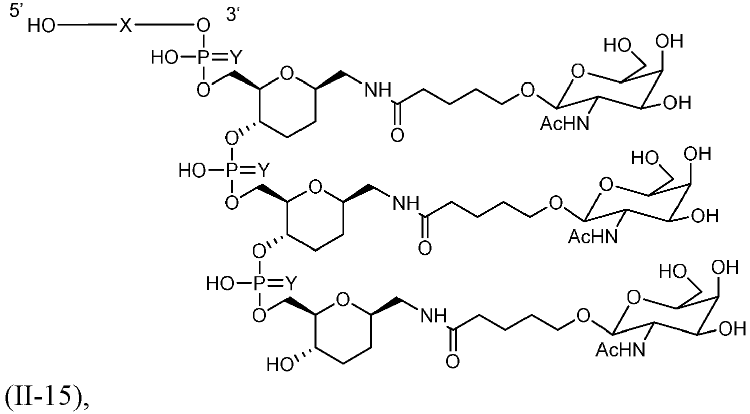 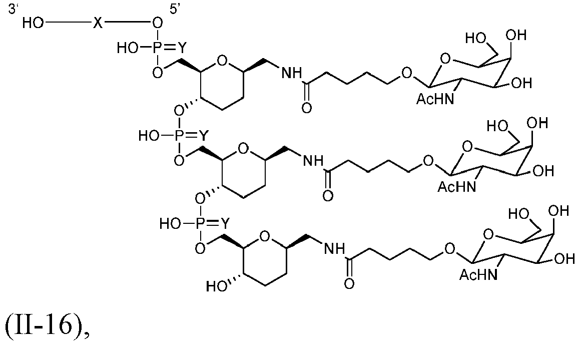 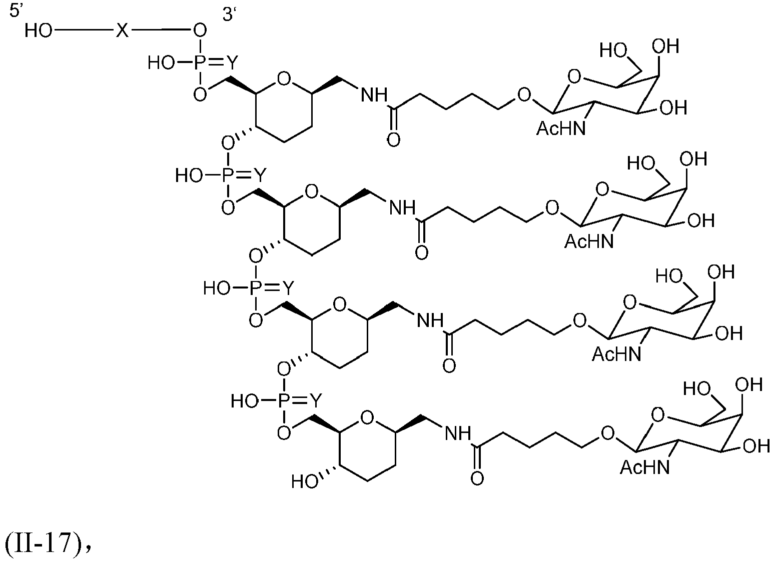 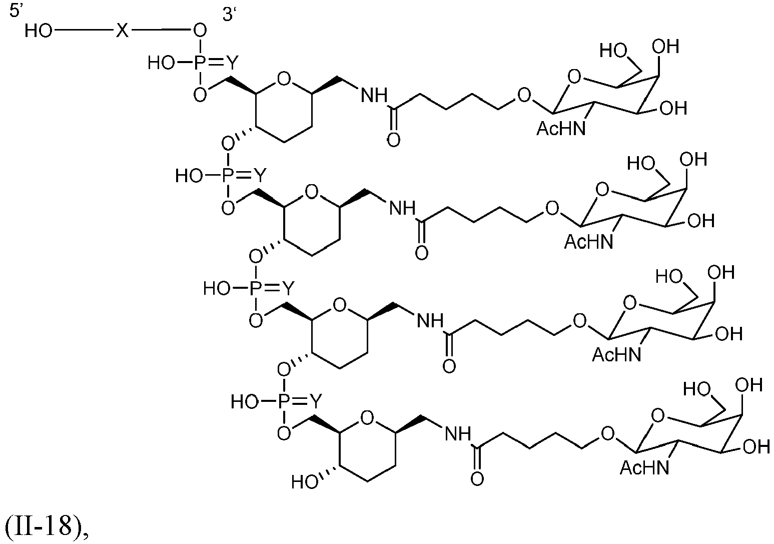 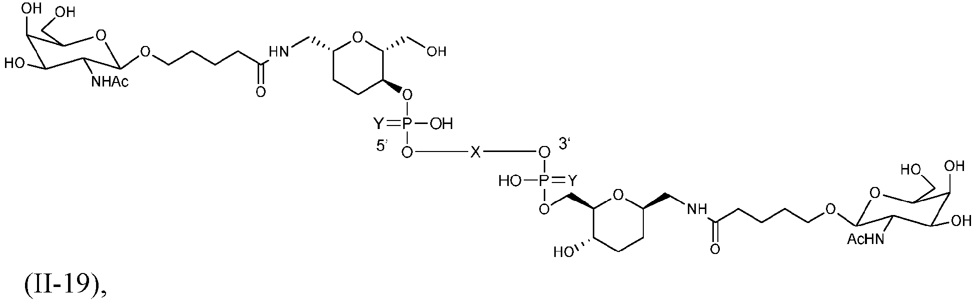 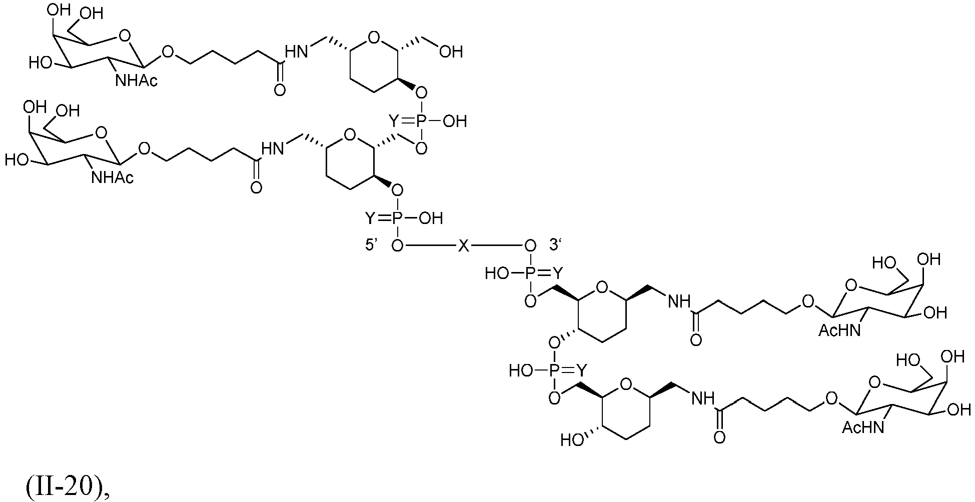 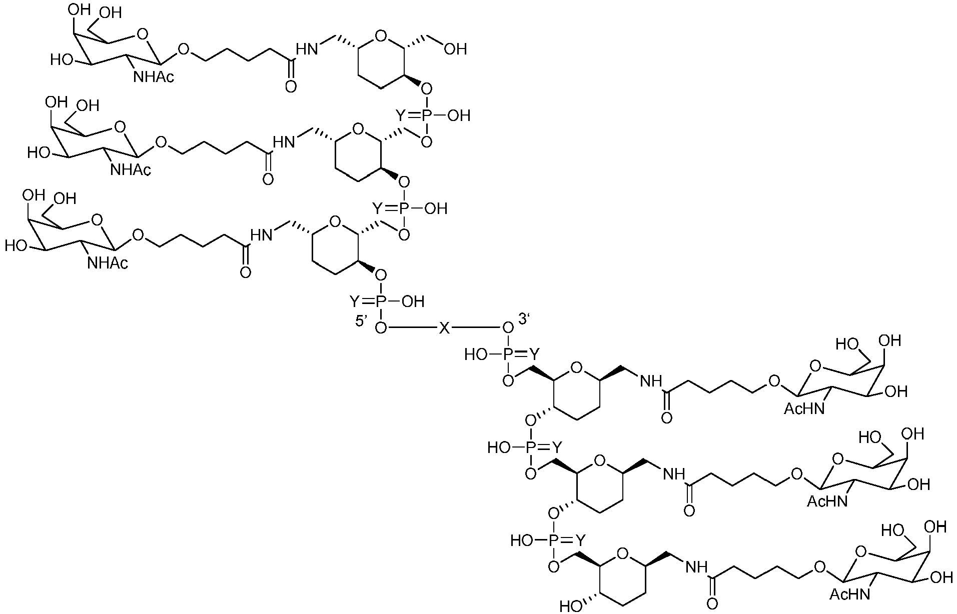 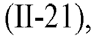 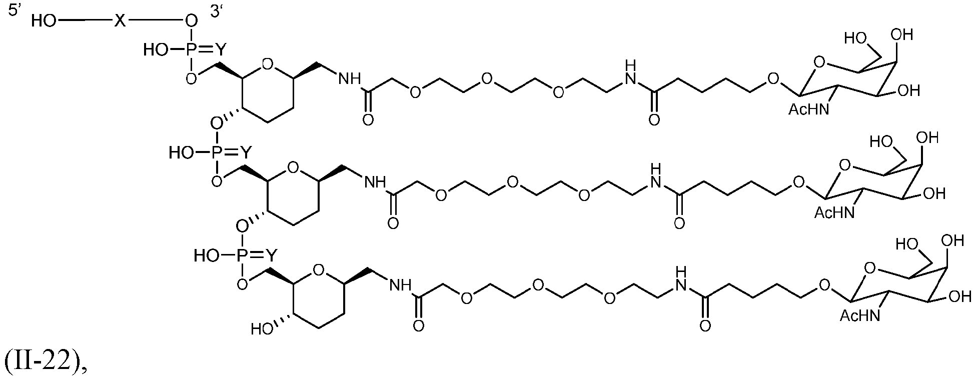 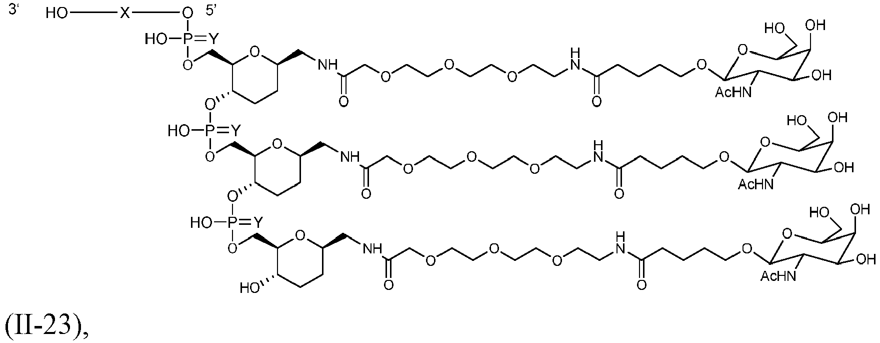 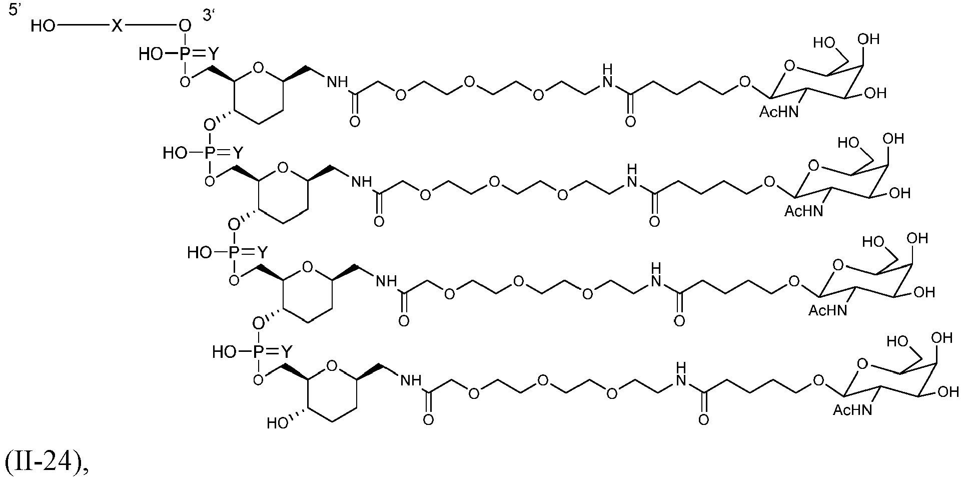 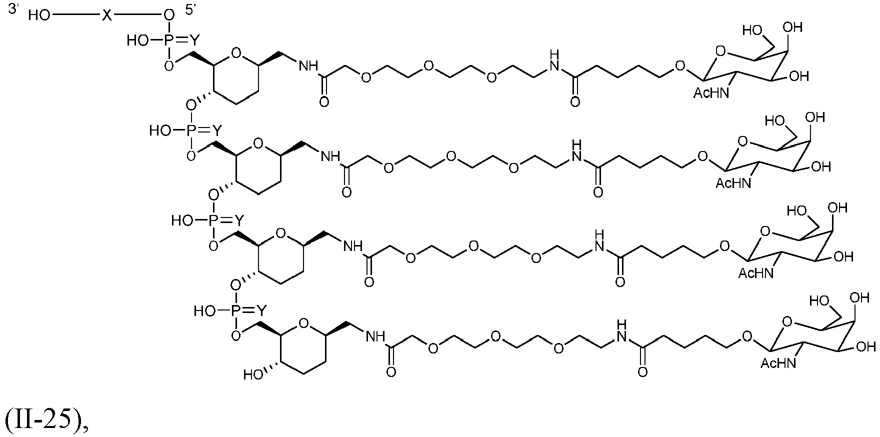 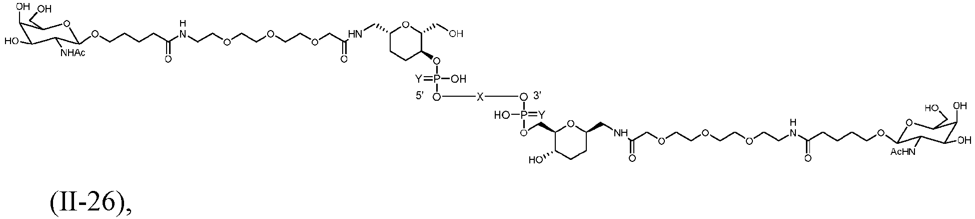 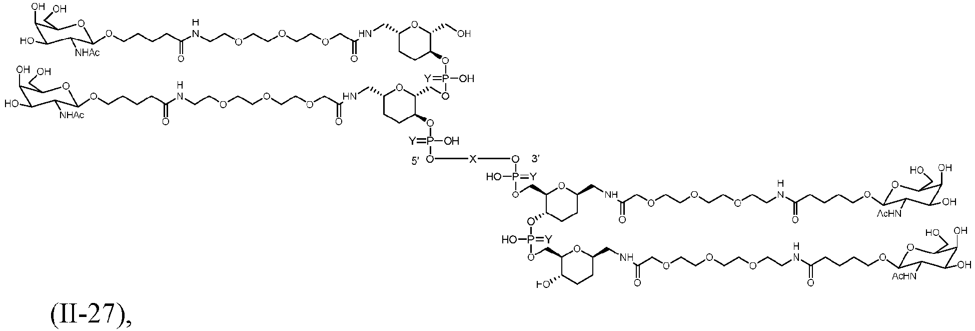 or 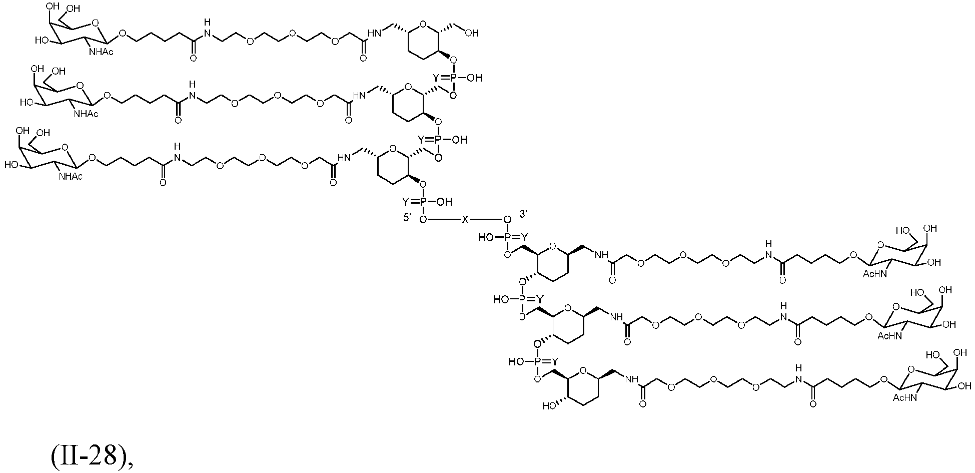 wherein Y is O or S.

23. The LPA RNA interference agent of any of claims 13 to 22, which comprises one of the sequences shown in Table 3.

24. A use of the LPA RNA interference agent of any of claims 1 to 23 in the preparation of a drug, wherein the drug is used to reduce LPA mRNA or protein expression in mammals, or to prevent and/or treat diseases or conditions related to LPA expression, or to reduce the risk of diseases or conditions.

25. The use of claim 24, wherein the LP(a) protein has an expression level of 34.100 nmol/L or more.

26. The use of claim 24, wherein the diseases or conditions comprise diseases of liver origin, inflammatory, cardiovascular and cerebrovascular diseases, myocardial infarction, or metabolic diseases, preferably, the cardiovascular and cerebrovascular diseases comprise hyperlipidemia, stroke, atherosclerosis, thrombosis, coronary heart disease, cardiac apoplexy, cerebral apoplexy or aortic stenosis.

27. The use of claim 24, wherein the drug comprises a pharmaceutically acceptable excipient, and preferably, the pharmaceutically acceptable excipient is a PBS buffer or saline solution.

28. The use of claim 24, wherein the drug is a composition which further comprises at least one selected from the group consisting of LDL-C, cholesterol, and triglyceride lowering agent.

29. The use of claim 24, wherein the drug is a composition which further comprises a PCSK9 RNAi inhibitor, a PCSK9 antibody inhibitor, and a PCSK9 small molecule inhibitor.
